(19) Europäisches Patentamt
European Patent Office
Office européen des brevets

(11) Publication number: **0 127 847**
**A1**

# (12) EUROPEAN PATENT APPLICATION

(21) Application number: **84105904.1**

(22) Date of filing: **24.05.84**

(51) Int. Cl.³: **C 07 D 499/00**
C 07 D 519/00, A 61 K 31/43
//C07D257/04, (C07D519/00, 499/00, 471/00), (C07D519/00, 499/00, 487/00)

(30) Priority: **27.05.83 GB 8314803**

(43) Date of publication of application:
**12.12.84 Bulletin 84/50**

(84) Designated Contracting States:
**AT BE CH DE FR GB IT LI LU NL SE**

(71) Applicant: **HOECHST U.K. LIMITED**
**Hoechst House Salisbury Road**
**Hounslow Middlesex TW4 6JH(GB)**

(72) Inventor: **Blumbach, Jürgen, Dr.**
**13b Manderscheider Strasse**
**D-6000 Frankfurt am Main 71(DE)**

(72) Inventor: **Ross, Barry Clive, Dr.**
**41 Blandford Avenue**
**Luton Bedfordshire(GB)**

(72) Inventor: **Johnson, Graham, Dr.**
**1130 Bandera Drive**
**Ann Arbor Michigan 48103(GB)**

(74) Representative: **Becker, Heinrich Karl Engelbert, Dr. et al,**
**HOECHST AKTIENGESELLSCHAFT Central Patent Department P.O. Box 80 03 20**
**D-6230 Frankfurt am Main 80(DE)**

## (54) 7-Oxo-4-thia-1-azabicyclo(3.2.0)hept-2-ene derivatives.

(57) A process for the preparation of a compound of the general formula I

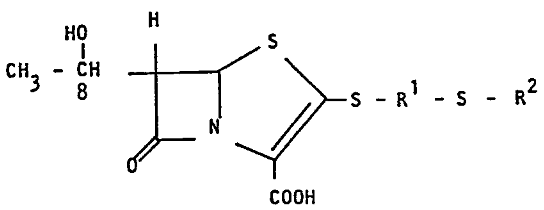

(I)

is described according to which a compound of the general formula IIb

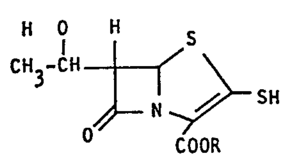

(IIb)

is reacted with a compound of formula III

$$L - R^1 - S - R^2 \qquad (III)$$

in which

L represents a leaving group, generally carrying out the reaction in the presence of a base.

$R^1$ represents an alkylene, alkenylene or alkynylene group, having from 1 to 4 carbon atoms, and

$R^2$ represents a heterocyclic group having a five-membered or 6-membered ring with 1 to 4 heteroatoms, selected from oxygen sulfur and nitrogen, and being linked to the adjacent sulphur atom via a ring carbon atom.

EP 0 127 847 A1

7-Oxo-4-thia-1-azabicyclo/3.2.0/hept-2-ene derivatives.

This invention relates to certain derivatives of 7-oxo-4-thia-1-azabicyclo/3.2.0/hept-2-ene, to a process for their preparation, and to pharmaceutical preparations comprising them.

7-Oxo-4-thia-1-azabicyclo/3.2.0/hept-2-ene has the following structure:

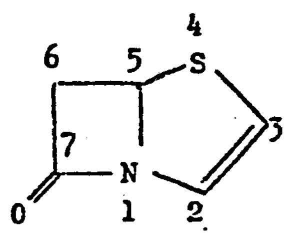

The present invention provides a compound of the general formula I

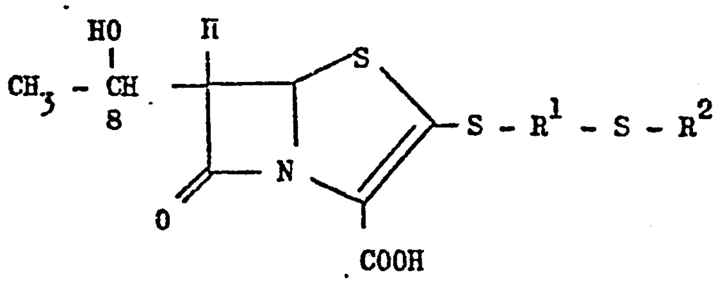

(I)

in which $R^1$ represents a straight or branched chain alkylene, alkenylene or alkynylene group, or a cycloalkylene or cycloalkenylene group, and

$R^2$ represents an unsubstituted or substituted heterocyclic group.

The present invention also provides esters and salts of the compound of formula I, especially the physiologically tolerable esters and salts, and references herein to " a compound of formula I" includes the salts and esters thereof.

Alkylene, alkenylene and alkynylene groups $R^1$ generally have up to 6 carbon atoms, and especially up to 4 carbon atoms, for example, methylene, ethylene and vinylene groups.

Cycloalkylene and cycloalkenylene groups $R^1$ generally have from 3 to 7 carbon atoms. A cycloalkylene or cycloalkenylene group $R^1$ may be linked to the adjacent atoms at any one or two positions about the ring, that is to say, the group may be spiro or otherwise, for example, $R^1$ may be a 1,1-, 1,2-, 1,3-, 1,4-, 1,5-, 1,6-, or 1,7- group, as appropriate.

$R^2$ represents a monocyclic or polycyclic heterocyclic group, and is linked to the adjacent sulphur atom via a ring carbon atom.

A heterocyclic group $R^2$ may be completely or partially hydrogenated, but is preferably non-hydrogenated.

Preferred monocyclic groups $R^2$ are optionally substituted five-membered or six-membered rings which have 1 to 4 hetero-atoms, for example, selected from oxygen, sulphur and nitrogen atoms, in particular nitrogen atoms, optionally together with sulphur and/or oxygen atoms.

A polycyclic ring system $R^2$ may have fused rings, for example, a monocyclic heterocyclic group may be fused to an aromatic 5-membered or 6-membered ring, which may be carbocyclic or heterocyclic, for example, a pyridine, triazole or benzene ring. The monocyclic ring is preferably as defined above.

A polycyclic group $R^2$ may contain up to 8 heteroatoms in the ring system.

The following fundamental ring systems may be mentioned as examples of the radical $R^2$: thienyl, furyl, pyrrolyl, imidazolyl, pyrazolyl, thiazolyl, isothiazolyl, oxazolyl, isoxazolyl, triazolyl, thiadiazolyl, oxadiazolyl,tetrazolyl, thiatriazolyl, oxatriazolyl, pyridyl, pyrimidyl, pyrazinyl, pyridazinyl, thiazinyl, oxazinyl, triazinyl, thiadiazinyl, ozadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl and

purinyl, as well as benzofused derivatives thereof, for example benzoxazolyl, benzthiazolyl, benzimidazolyl and indolyl, and also fused heterocyclic rings, i.e. tetrazolopyridazinyl, triazolotetrazinyl, triazolotriazolyl, triazolothiadiazolyl, triazolopyrimidinyl, pyrazolopyrimidinyl, and triazolopyridinyl.

5-membered ring systems with a sulphur or oxygen atom and 1 to 3 nitrogen atoms, for example, thiazolyl, in particular thiazol-2-yl and thiazol-2-yl N-oxide; thiadiazolyl, in particular 1,3,4-thiadiazol-5-yl and 1,2,4-thiadiazol-5-yl; oxazolyl, preferably oxazol-2-yl; and oxadiazolyl, for example, 1,3,4-oxadiazol-5-yl, are preferred. Furthermore, 5-membered ring systems with 2 to 4 nitrogen atoms, for example, imidazolyl, preferably imidazol-2-yl; triazolyl, preferably 1,3,4-triazol-5-yl and 1,2,3-and 1,2,4-triazol-5-yl; and tetrazolyl, preferably 1 H-tetrazol-5-yl and 2H-tetrazolyl, are preferred. Benzofused derivatives, in particular benzoxazol-2-yl, benzthiazol-2-yl and benzimidazol-2-yl, are also preferred.

Further preferred ring systems are 6-membered ring systems with 1 to 3, preferably 1 or 2, nitrogen atoms, for example, pyridyl, for example, pyrid-2-yl, pyrid-3-yl and pyrid-4-yl; pyrimidyl, preferably pyrimid-2-yl and pyrimidyl-4-yl; triazinyl, preferably 1,3,4-triazin-2-yl and 1,3,5-triazin-4-yl; and pyridazinyl, in particular pyridazin-3-yl, and pyrazinyl. Pyridyl, pyrimid-2-yl, pyrimid-4-yl and pyridazinyl radicals.

Further preferred ring systems are 5 and 6 membered fused heterocycles, for example, tetrazolo[1,5-b]pyridazin-6-yl; 1,2,4-triazolo

[4,3-b]tetrazin-7-yl; 1,2,4-triazolo[3,4-b]1,3,4-thiadiazol-6-yl; 1,2,4-triazolo[1,5-c]1,2,4-triazol-3-yl; 1,3,4-thiadiazolo[2,3-b] tetrazol-5-yl; pyrazolo[3,4-d]pyrimidin-4-yl; 1,2,4-triazolo[4,3-a] pyridin-3-yl; 1,2,4-triazolo[4,3-b]pyridazin-6-yl; tetrazolo[1,5-c] pyrimidin-5-yl; 1,2,3-triazolo[4,5-d]pyrimidin-4-yl; 1,2,3-triazolo-[4,5-d]pyridazin-4-yl; 1,2,4-triazolo[1,5-a]pyrimidin-2-yl.

$R^2$ can be monosubstituted or polysubstituted, examples of substituents being the following:

i) straight-chain and branched alkyl groups with, for example, 1 to 15 carbon atoms, for example, methyl, ethyl, n-propyl, i-propyl, n-butyl, tert.-butyl, n-hexyl, undecyl and pentadecyl groups, preferably those with 1 - 4 carbon atoms, for example, methyl and ethyl groups;

ii) alkyl groups with 1 to 4 carbon atoms, for example, methyl groups, which are substituted by one or more substituents for example selected from aromatic groups, for example, phenyl or thienyl groups; aryloxy groups for example phenoxy groups; lower alkoxy groups, for example, methoxy and ethoxy groups; lower alkoxycarbonyl groups, for example, methoxy- and ethoxycarbonyl groups; halogen atoms, for example, chlorine and bromine atoms; hydroxy groups; aliphatic acylamido groups, preferably with 1 to 4 carbon atoms, for example, acetamido groups; aromatic acylamido groups, for example, benzamido groups; amino groups; alkylamino groups with 1 to 4 carbon atoms, for example, methylamino and ethylamino groups; dialkylamino groups with 1 - 4 carbon atoms in each alkyl moiety, for example,

dimethyl- or diethyl-amino, it being possible for the alkyl radicals of a dialkylamino group to be closed to form a 5-membered to 7-membered ring which is optionally interrupted by one or more further hetero-atom(s), for example, oxygen and/or nitrogen atoms, for example, to give a morpholino or piperazino group; trifluoromethyl groups; cyano groups; carbamoyl groups; carboxyl groups; carbalkoxyalkoxy groups with 1 - 4 alkyl carbon atoms, for example, carboxymethoxy groups; cyanoalkoxy groups with 1 to 4 alkyl carbon atoms, for example, cyanomethoxy groups; carbamoylalkoxy groups with 1 - 4 alkyl carbon atoms, for example, carbamoylmethoxy groups; alkoxycarbonyloxy groups with 1 - 4 alkyl carbon atoms, for example, methoxycarbonyloxy groups; sulpho groups; alkylsulpho groups, preferably with 1 - 4 carbon atoms, for example, methylsulphonyl groups; sulphamoyl groups; phosphonyl groups; alkylcarbamoyl groups with 1 to 4 alkyl carbon atoms, for example, methylcarbamoyl groups; dialkylcarbamoyl groups with 1 - 4 alkyl carbon atoms in each alkyl group, for example dimethyl-carbamoyl groups; alkyl- and dialkylsulphamoyl groups with 1 - 4 carbon atoms in the or each alkyl moiety, for example, methyl- and dimethylsulphamoyl groups; carboxyalkylcarboxamido groups, preferably with 1 - 4 alkyl carbon atoms, for example, as derived from succinamic acid; cyanoalkylcarboxamido groups, preferably with 1 - 4 alkyl carbon atoms, for example, as derived from malonic acid mononitrileamide; and alkoxycarbonylalkylcarboxamido groups, preferably with 1 - 4 carbon atoms in each alkyl group, it being possible for a carboxamido nitrogen atom to be itself substituted, for example,

as derived from methyl-succinamate and methyl N-methyl-succinamate;

iii) cycloalkyl groups with 3 to 8 carbon atoms, for example, cyclopentyl and cyclohexyl groups;

iv) alkoxy groups with 1 - 4 carbon atoms, for example, methoxy and ethoxy groups;

v) alkenyl groups with 2 - 4 carbon atoms, for example, allyl groups;

vi) alkenyloxy groups with 3 - 5 carbon atoms, for example, allyloxy groups;

vii) alkylthio and alkenylthio groups with up to 4 carbon atoms, for example, methylthio and allylthio groups;

viii) alkylsulphinyl and alkylsulphonyl groups with up to 4 carbon atoms, for example, methylsulphinyl, methylsulphonyl, ethylsulphinyl and ethylsulphonyl groups;

ix) alkoxycarbonyl groups with 1 - 4 alkyl carbon atoms, for example, methoxycarbonyl groups;

x) alkylcarbonyl groups with 1 - 4 alkyl carbon atoms, for example, acetyl groups;

xi) arylcarbonyl groups, for example, benzoyl groups;

xii) carboxyalkoxycarbonyl groups with 1 - 4 alkyl carbon atoms, for example, carboxymethoxycarbonyl groups;

xiii) cyanoalkoxycarbonyl groups with 1 - 4 alkyl carbon atoms, for example, cyanomethoxycarbonyl groups;

xiv) carbamoylalkoxycarbonyl groups with 1 to 4 alkyl carbon atoms, for example, carbamoyloxycarbonyl groups;

xv) alkoxycarbonylamino groups with 1 - 4 alkyl carbon atoms, for example, ethoxycarbonylamino groups;

xvi) carboxyalkylthio groups with 1 - 4 alkyl carbon atoms, for example, carboxymethylthio groups;

xvii) amino groups;

xviii) arylamino groups, for example, phenylamino groups;

xix) heteroarylamino groups, for example, pyrid-2-yl-amino and pyrid-4-yl-amino groups;

xx) monoalkyl- and dialkyl-amino groups with 1 - 4 carbon atoms in the or each alkyl moiety, for example, methylamino, dimethylamino, ethylamino and diethylamino groups, it also being possible for two alkyl substituents to be closed to form a 5-membered to 7-membered ring which is optionally interrupted by one or more hetero-atoms, for example, oxygen and/or nitrogen atoms, for example, morpholino, piperidino, pyrrolidino and piperazino groups;

xxi) carboxyalkylamino groups with 1 - 4 alkyl carbon atoms, for example, carboxymethylamino groups;

xxii) cyanoalkylamino groups with 1 - 4 alkyl carbon atoms, for example, cyanomethylamino groups;

xxiii) alkoxycarbonylalkylamino groups with 1 - 4 alkoxy and 1 - 4 alkyl carbon atoms, for example, methoxycarbonylmethylamino groups;

xxiv) sulphoalkylamino groups with 1 - 4 carbon atoms, for example, sulphomethyl groups;

xxv) sulphamoylalkylamino groups with 1 to 4 carbon atoms, for example, sulphamoylmethylamino groups;

xxvi) alkylsulphamoylalkylamino groups with 1 - 4 carbon atoms in each alkyl moiety, for example, methylsulphamoylmethylamino groups;

0127847

xxvii) dialkylsulphamoylalkylamino groups with 1 - 4 carbon atoms in each alkyl moiety, for example, dimethylsulphamoylmethylamino groups;

xxviii) alkoxysulphonylalkylamino groups with 1 - 4 alkoxy carbon and 1 - 4 alkyl carbon atoms, for example, methoxysulphonylmethylamino groups;

xxix) oxido groups and oxo groups;

xxx) hydroxy groups;

xxxi) hydroxyalkyl groups with 1 - 4 alkyl carbon atoms, for example, hydroxymethyl and hydroxyethyl groups;

xxxii) carboxyalkylcarbonyloxy groups with 1 - 4 alkyl carbon atoms, for example, carboxymethylcarbonyloxy groups;

xxxiii) cyanoalkylcarbonyloxy groups with 1 to 4 alkyl carbon atoms, for example, cyanomethylcarbonyloxy groups;

xxxiv) alkoxycarbonylalkylcarbonyloxy groups with 1 - 4 carbon atoms in each alkyl moiety, for example, methoxycarbonylmethylcarbonyloxy groups;

xxxv) carboxyalkoxy groups with 1 - 4 alkyl carbon atoms, for example, carboxymethoxy groups;

xxxvi) cyanoalkoxy groups with 1 - 4 alkyl carbon atoms, for example, cyanomethoxy groups;

xxxvii) alkoxycarbonylalkoxy groups with 1 - 4 carbon atoms in each alkoxy moiety, for example, methoxycarbonylmethoxy groups;

xxxviii) carbamoylalkoxy groups with 1 - 4 alkyl carbon atoms, for example, carbamoylmethoxy groups;

xxxix) carbamoylalkylcarbonyloxy groups with 1 - 4 alkyl carbon atoms, for example, carbamoylmethylcarbonyloxy groups;

0127847

xl) sulphoalkoxy groups with 1 - 4 carbon atoms, for example sulphomethyl groups;

xli) sulphamoylalkoxy groups with 1 - 4 carbon atoms, for example, sulphamoylmethoxy groups;

xlii) nitro groups;

xliii) cyano groups;

xliv) halogen atoms, preferably chlorine atoms;

xlv) trifluoromethyl groups;

xlvi) mercapto groups;

xlvii) carboxyl groups;

xlviii) carbamoyl groups;

xlix) carboxyalkylaminocarbonyl groups with 1 - 4 alkyl carbon atoms, for example, carboxymethylaminocarbonyl groups;

l) carbamoylalkylaminocarbonyl groups with 1 - 4 alkyl carbon atoms, for example, carbamoylmethylaminocarbonyl groups;

li) alkoxycarbonylalkylaminocarbonyl groups with 1 - 4 alkoxy and 1 - 4 alkyl carbon atoms, for example, methoxycarbonylmethylaminocarbonyl groups;

lii) aromatic groups, for example, phenyl groups; and substituted phenyl groups, for example, alkoxyphenyl groups with 1 - 4 alkoxy carbon atoms, for example, methoxyphenyl and ethoxyphenyl groups; alkylthiophenyl groups with 1 - 4 alkylthio carbon atoms, for example, methylthiophenyl groups; halogenophenyl groups, for example, chlorophenyl groups; hydroxyphenyl groups; aminophenyl groups; alkylamino- and dialkylaminophenyl groups with 1 - 4 alkyl carbon atoms in the or each alkyl moiety, for example, methylamino- and dimethylaminophenyl groups; alkylphenyl

groups, in particular alkylphenyl groups with 1 - 4 alkyl carbon atoms, for example, tert.-butylphenyl, tolyl and cetylphenyl groups; hydroxyalkylphenyl groups with 1 - 4 alkyl carbon atoms, for example, hydroxyethylphenyl groups; halogenoalkylphenyl groups with 1 - 4 alkyl carbon atoms, for example, trifluoromethylphenyl and chloromethylphenyl groups; alkoxyalkylphenyl groups with 1 - 4 alkoxy and 1 - 4 alkyl carbon atoms, for example, methoxymethylphenyl groups; alkenylphenyl groups with 2 to 6, preferably 3 - 5, alkenyl carbon atoms, for example, allylphenyl groups; alkenyloxyphenyl groups with 2 - 6, preferably 3 - 5, alkenyloxy carbon atoms, for example, allyloxyphenyl groups; cyanophenyl groups; carbamoylphenyl groups; carboxyphenyl groups; alkoxycarbonylphenyl groups with 1 - 4 alkyl carbon atoms, for example, methoxycarbonylphenyl groups; alkylcarbonyloxyphenyl groups with 1 - 4 alkyl carbon atoms, for example, acetoxyphenyl groups; sulphophenyl groups; alkoxysulphophenyl groups with 1 - 4 alkoxy carbon atoms, for example, methoxysulphophenyl groups; sulphamoylphenyl groups; nitrophenyl groups; biphenyl groups, and optionally correspondingly substituted naphthyl radicals and heterocyclic radicals which are derived from heterocyclic 5-membered or 6-membered rings with 1 to 4 hetero-atoms, in particular nitrogen, sulphur and/or oxygen, for example, pyridyl, furyl, quinolyl, isoquinolyl, thienyl, thiazolyl, N-pyrrolyl, pyrrolyl, imidazolyl, pyrazolyl, isoxazolyl, tetrazolyl and triazolyl groups;

liii) cyanoalkylaminocarbonyl groups with 1 - 4 carbon atoms, for example, cyanomethylaminocarbonyl groups;

liv) carboxyalkylcarboxamido groups with 1 - 4 carbon atoms, for example, as derived from succinamic acid;

lv) alkoxyalkylcarboxamido groups with 1 - 4 carbon atoms, for example, as derived from methylsuccinamate groups;

lvi) cyanoalkylcarboxamido groups with 1 - 4 carbon atoms, for example, as derived from malonic acid nitrile-monoamide;

lvii) alkylcarbonyl groups with 1 - 4 carbon atoms, for example, methylaminocarbonyl groups;

lviii) dialkylcarbamoyl groups with 1 - 4 carbon atoms, for example, dimethylaminocarbonyl groups, it also being possible for the two alkyl radicals to be closed to form a carbocyclic ring with 5 - 7 carbon atoms, which can be interrupted by nitrogen, sulphur and/or oxygen atoms, for example, morpholinocarbonyl groups;

lix) alkoxycarbonylalkoxyalkyl groups with 1 - 4 carbon atoms in each alkyl moiety, for example, methoxycarbonylalkoxyalkyl groups;

lx) alkylcarbamoylalkoxyalkyl groups with 1 - 4 carbon atoms, for example, methylcarbamoylmethoxymethyl groups;

lxi) alkoxyalkylaminocarbonylalkyl groups, for example, a methoxymethylaminocarbonylmethyl group;

lxii) amino groups and amino groups which are monosubstituted by a lower alkyl group, it being possible for the amino group to be acylated by a lower aliphatic or an aromatic carboxylic acid, for example, an acetamido or benzamido group, or an aryl or hetero-aromatic radical which is substituted by trifluoromethyl or alkylcarboxyl with 1 - 4 carbon atoms.

In the passage above relating to substituents for a group $R^2$, the number of alkyl carbon atoms (1 - 4) relates to the or each alkyl group present in the particular substituent described.

In the description below, as far as the end of paragraph (m), there are up to 6 and preferably up to 4 carbon atoms in each alkyl, alkenyl, alkanyl or other aliphatic group, whether that group is a group in itself or is part of a larger group.

Of the 5-membered rings with 2 - 4 hetero-atoms, such as nitrogen, sulphur and/or oxygen atoms, preferably at least one hetero-atom being nitrogen, and 6-membered rings with 1 - 3 hetero-atoms, in particularnitrogen atoms, which are preferred according to tthe invention, for $R^2$, the following radicals of the general formulae 1 to 12 may be mentioned as examples of particularly preferred radicals:

(a) A thiazolyl radical of the general formula I

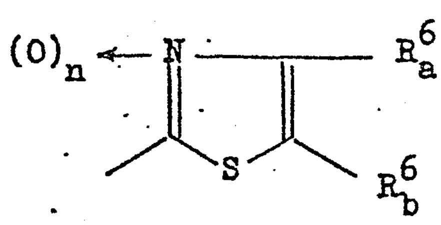

(1)

in which $R_a^6$ and $R_b^6$ can be identical or different and each represents a hydrogen atom; a straight chain or branched alkyl group, which is unsubstituted or substituted by one or more substituents selected from halogen atoms, alkoxy groups, hydroxyl groups, amino groups, alkylamino and dialkylamino groups, trifluoromethyl groups, and phenyl groups; a straight chain or branched alkenyl group; a carbocyclic ring with 3 - 8 carbon atoms; an amino group, an alkylamino or dialkylamino group; aliphatic acylamido group; a carboxyalkyl group; analkoxycarbonylalkyl group; a carbamoylalkyl group; a carboxyl, carbamoyl, cyano, or cyanoalkyl group; an alkoxycarbonyl, carboxyalkylaminocarbonyl, alkoxycarbonylalkylaminocarbonyl, or cyanoalkylaminocarbonyl group; a carboxyalkylcarboxamido, alkoxycarbonylalkylcarboxamido or cyanoalkylcarboxamido group; a carb_oxyalkylthio group; an optionally substituted heteroaryl radical or a phenyl radical, which radicals are optionally substituted by one or two substituents selected from halogen atoms, alkyl and alkoxy groups, hydrpxyl groups, nitro, carboxy, carbamoyl groups, alkylamino and dialkylamino groups, alkylthio groups, cyano and trifluoromethyl groups, it being possible for $R_a^6$ and $R_b^6$ together to form an optionally substituted aromatic or non-aromatic carbocyclic ring with 5 - 7 carbon atoms; and n represents 0 or 1.

Examples which may be mentioned are, in particular: 1,3 - thiazol - 2 - yl,4-methyl - 1,3 - thiazol - 2 - yl,4 - tert. - butyl - 1,3 - thiazol - 2 - yl,4 - n - propyl - 1,3 - thiazol - 2 - yl,4 - ethyl - 1,3 - thiazol - 2 - yl,5 - amino - 1,3 - thiazol - 2 - yl,5 - acetamido - 1,3 - thiazol - 2 - yl,5 - methylamino - 1,3 - thiazol-2 - yl, benzothiazol - 2 - yl,5 - chloro - benzothiazol - 2 - yl,4-methyl - 3 - oxy - 1,3 - thiazol - 2 - yl,3 - oxy - 4 - phenyl - 1,3 - thiazol - 2 - yl,4 - (4 - chlorophenyl) - 3 - oxy - 1,3 - thiazol - 2 - yl,3 - oxy - 1,3 - thiazol - 2 - yl,4 - (4 - bromophenyl) - 3 - oxy - 1,3 - thiazol - 2 - yl,3 - oxy - 4 - (p - tolyl) - 1,3 - thiazol - 2 - yl,4 - (p - methoxyphenyl) - 3 - oxy - 1,3 - thiazol - 2 - yl,4 - methyl - 3 - oxy - 5 - phenyl - 1,3 - thiazol - 2 - yl,5 - methyl - 3 - oxy - 4 - phenyl - 1,3 - thiazol - 2 - yl,5 - methyl - 1,3 - thiazol - 2 - yl,4 - trifluoromethyl - 1,3 - thiazol - 2 - yl,4 - phenyl - 1,3 - thiazol - 2 - yl,4,5 - dimethyl - 1,3 - thiazol - 2 - yl,4 - (3 - pyridyl) - 1,3 - thiazol - 2 - yl,4 - carboxymethyl - 1,3 - thiazol - 2 - yl,3 - carboxy - 4 - methyl - 1,3 - thiazol - 2 - yl,4 - carboxy - 1,3 - thiazol - 2 - yl,4 - ethoxycarbonyl - 5 - amino - 1,3 - thiazol - 2 - yl,5 - amino - 4 - carboxy - 1,3 - thiazol - 2 - yl,5 - carboxymethylaminocarbonyl - 1,3 - thiazol - 2 - yl,5 -

carboxymethylcarboxamido - 1,3 - thiazol - 2 - yl,5 - carboxymethyl - 4 - phenyl - 1,3 - thiazol - 2 - yl,4 - (5 - nitro - thien - 2 - yl) - 1,3 - thiazol - 2 - yl,4 - (4 - carboxythien - 2 - yl) - 1,3 - thiazol - 2 - yl,4 - (1 - methyl - pyrrol - 2 - yl) - 1,3 - thiazol - 2 - yl,4 - (5 - carbamoyl - fur - 2 - yl) - 1,3 - thiazol - 2 - yl and 5 - carboxy - 4 - methyl - 1,3 - thiazol - 2 - yl groups.

b) A pyridyl radical of the general formula 2

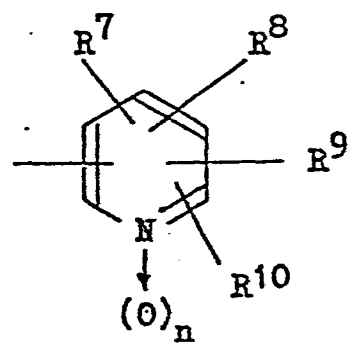

...(2)

in which $R^7$ to $R^{10}$, which may be identical or different, each denotes a hydrogen or halogen atom, a straight chain or branched alkyl or alkenyl group, a trifluoromethyl group, an alkylcarbonyl group, an amino group, an alkylamino or dialkylamino group, a carboxyl, carbamoyl or cyano group, an alkylaminocarbonyl or dialkylaminocarbonyl group, an alkoxycarbonyl group, a hydroxyl group, an alkoxy group, a hydroxyalkyl group, a mercapto group, an alkylthio group, or a nitro group, and n represents 0 or 1.

Examples which may be mentioned are, in particular: 1 - oxo - pyrid - 2 - yl, 3 - methyl - 1 - oxo - pyrid - 2 - yl,4 - methyl -

1 - oxo - pyrid - 2 - yl,1 - oxo - pyrid - 4 - yl,5 - methyl - 1 - oxo - pyrid - 2 - yl,6 - methyl - 1 - oxo - pyrid - 2 - yl,3 - ethoxy - 1 - oxo - pyrid - 2 - yl,5 - bromo - 1 - oxo - pyrid - 2 - yl,pyrid - 2 - yl,pyrid - 3 - yl,pyrid - 4 - yl,3 - hydroxy - pyrid - 2 - yl,3 - nitro - pyrid - 2 - yl,4 - nitro - pyrid - 2 - yl,5 - nitro - pyrid - 2 - yl,2 - amino - 6 - methyl - pyrid - 3 - yl,4 - chloro - 1 - oxo - pyrid - 2 - yl, 2 - carboxy - pyrid - 4 - yl,3 - carboxy - pyrid - 5 - yl,4 - carboxy - pyrid - 5 - yl,6-methylpyrid-2-yl, 5-methylpyrid-2-yl and 4-methylpyrid-2-yl groups.

c) An oxadiazolyl, thiadiazolyl or triazolyl radical of the general formula 3a, 3b or 3c

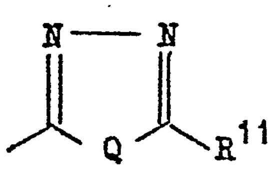

(3a)

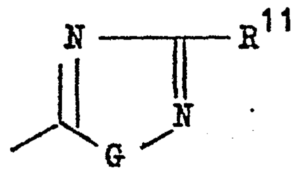

(3b)

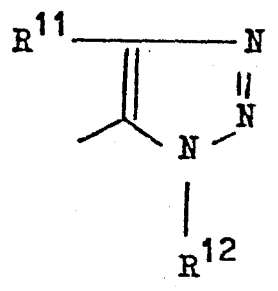

(3c)

in which Q represents an oxygen or sulfur atom or the group

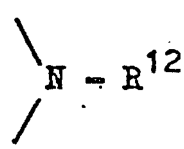

and G represents an oxygen or sulfur atom, and wherein $R^{11}$ denotes a hydrogen atom, a straight chain or branched alkyl, or straight-chain or branched alkenyl group, a carbocyclic ring with 5 - 7 carbon atoms, a hydroxyl group, a hydroxyalkyl group, an alkoxy group, a mercapto, alkylthio, alkylsulphinyl, alkylsulphonyl or alkoxyalkyl group, an amino group, which can optionally be substituted by 1 or 2 alkyl radicals which together with the nitrogen atom can form a ring with 5-7 ring atoms, an aliphatic or aromatic acylamido group, an aminoalkyl group, which may be optionally substituted by one or two branched or straight chain alkyl radicals, which together with the nitrogen can form a ring with 5 - 7 ring atoms, or may be acylated by an aliphatic or aromatic carboxylic acid, a trifluoromethyl group, an alkoxycarbonylalkylamido, carboxyalkylamido or cyanoalkylamido group, an alkoxycarboxyalkoxyalkyl or carboxyalkyl group, an alkoxycarbonylalkyl or cyanoalkyl group, a carboxyl, carbamoyl or cyano group, a carbamoylalkyl group, an alkoxycarbonyl group, an alkylcarbamoyl or dialkylcarbamoyl group, a sulfoalkyl, sulfamoylalkyl, alkylsulfamoyl or dialkylsulfamoylalkyl group, an alkylcarbamoylalkyl or dialkylcarbamoylalkyl group, an alkoxycarbonylalkoxyalkyl or carboxyalkoxyalkyl group, a carbamoylalkoxyalkyl or alkylcarbamoylalkoxyalkyl group, an alkoxyalkylaminocarbonylalkyl group, a carboxyalkylthio group, or an aryl or heterocyclic radical, preferably a phenyl, naphthyl, thienyl, furyl, thiazolyl, pyrrolyl, imidazolyl, pyrazolyl, isoxazolyl, quinolyl, isoquinolyl or pyridyl radical, which is optionally substituted by or two substituents selected from halogen atoms, hydroxy groups, alkoxy

groups, straight chain and branched alkyl, and straight chain and branched alkenyl groups, trifluoromethyl, cyano, amino and carboxyl groups, alkoxycarbonyl groups, sulpho, carbamoyl and sulphamoyl groups, alkylcarboxy, alkylcarbonyl and alkylamino groups, nitro groups and dialkylamino groups; arylamino, heterocyclic-amino and aralkylamino groups, optionally substituted as described above for aryl and heterocyclic groups, and in which $R^{12}$ can be a hydrogen atom, a straight chain or branched alkyl, or straight chain or branched alkenyl group, carboxyalkyl, alkoxycarbonylalkyl, cyanoalkyl, sulphoalkyl, sulphamoylalkyl, alkylsulphamoylalkyl, dialkylsulphamoylalkyl, carbamoylalkyl, alkylcarbamoylalkyl, dialkylcarbamoylalkyl, alkoxycarbonylalkoxyalkyl, carboxyalkoxyalkyl, carbamoylalkoxyalkyl or alkylcarbamoylalkoxyalkyl group, a hydroxyl group, a hydroxyalkyl group, an amino or aminoalkyl group, which can be optionally acylated with an aliphatic carboxylic acid, alkylated with one or two alkyl radicals, or substituted by an aryl group, an alkoxyalkyl group, a carbocyclic ring with 5 to 7 carbon atoms, a pyrrolyl radical, which can be optionally substituted by one or two alkyl groups, or an aryl, aralkyl or heterocyclic radical, preferably a phenyl, benzyl or pyridine radical, which can be optionally substituted by one or more substitutents selected from carboxyl, cyano, trifluoromethyl, carbamoyl, amino, alkylamino, dialkylamino, alkyl, sulpho, sulphamoyl, alkoxycarbonyl, hydroxyl, hydroxyalkyl, alkylcarbonyl, alkoxy and nitro groups, and halogen atoms.

Examples which may be mentioned are, in particular, for

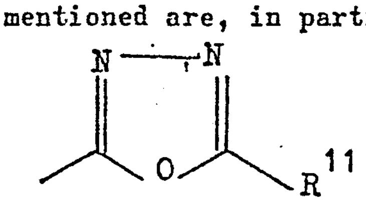

1,3,4 - oxadiazol - 5 - yl,2 - methyl - 1,3,4 - oxadiazol - 5 - yl,2-methoxy-1,3,4-oxadiazol-5-yl, 2-ethoxy-1,3,4-oxadiazol-5-yl, 2-trifluoromethyl-1,3,4-oxadiazol-5-yl, 2-carboxymethyl-1,3,4-oxadiazol-5-yl, 2-methoxycarbonylmethyl-1,3,4-oxadiazol-5-yl, 2-aminocarbonylmethyl-1,3,4-oxadiazol-5-yl, 2-carboxy-1,3,4-oxadiazol-5-yl, 2-aminomethyl-1,3,4-oxadiazol-5-yl, 2-dimethylaminomethyl-1,3,4-oxadiazol-5-yl, 2-aminocarbonylmethoxy-methyl-1,3,4-oxadiazol-.-yl, 2-methoxymethyl-1,3,4-oxadiazol-5-yl, 2-ethoxymethyl-1,3,4-oxadiazol-5-yl, 2-hydroxymethyl-1,3,4-oxadiazol-5-yl, 2-phenyl-1,3,4-oxadiazol-5-yl, 2 - cyclohexyl - 1,3,4 - oxadiazol - 5 - yl,2 - (2 - pyridyl) - 1,3,4 - oxadiazol - 5 - yl,2 - (3 - pyridyl) - 1,3,4 - oxadiazol - 5 - yl,2 - (4 - pyridyl) - 1,3,4 - oxadiazol - 5 - yl,2 - (2 - furyl) - 1,3,4 - oxadiazol - 5 - yl,2 - (3 - furyl) - 1,3,4 - oxadiazol - 5 - yl,2 - (2 - thienyl) - 1,3,4 - oxadiazol - 5 - yl,2 - propyl - 1,3,4 - oxadiazol - 5 - yl,2 - butyl - 1,3,4 - oxadiazol - 5 - yl,2 - ethyl - 1,3,4 - oxadiazol - 5 - yl,2 - (3 - thienyl) - 1,3,4 - oxadiazol - 5 - yl,2-(2 - thiazolyl) - 1,3,4 - oxadiazol - 5 - yl,2 - benzyl - 1,3,4 - oxadiazol - 5 - yl,2 - (1 - naphthyl) - 1,3,4 - oxadiazol - 5 - yl,2 - (2 - pyrrolyl) - 1,3,4 - oxadiazol - 5 - yl,2 - (N - methylcarbamoyl) - 1,3,4 - oxadiazol - 5 - yl,2 - (N - oxadiazol - 5 - yl,2 - (N - ethylcarbamoyl) - 1,3,4 - oxadiazol - 5 - yl,2 - (N,N - dimethylcarbamoyl) - 1,3,4 - oxadiazol - 5 - yl, 2 - (NN - dimethylaminomethyl) - 1,3,4 - oxadiazol - 5 - yl, 2 - (4 - imidazolyl) - 1, 3, 4 - oxadiazol - 5 - yl, 2 - (5 - pyrazolyl) - 1, 3, 4 - oxadiazol - 5 - yl, 2 - (ethoxycarbonylmethoxymethyl - 1, 3, 4 - oxadiazol - 5 - yl, 2 - (carboxymethoxymethyl) - 1, 3, 4 - oxadiazol - 5 - yl and 2 - carbamoyl - 1, 3, 4 - oxadiazol - 5 - yl groups; for

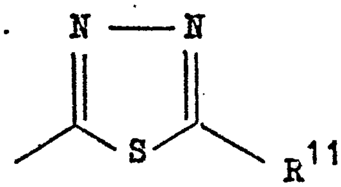

1,3,4 - thiadiazol - 5 - yl,2 - butyl - 1,3,4 - thiadiazol - 5 - yl,2 - propyl - 1,3,4 - thiadiazol - 5 - yl,2 - phenyl - 1,3,4 - thiadiazol - 5 - yl,2 - amino - 1,3,4 - thiadiazol - 5 - yl,2 - ethyl - 1,3,4 - thiadiazol - 5 - yl,2-dimethylamino-1,3,4-thiadiazol-5-yl, 2-dimethylaminomethyl-1,3,4-thiadiazol-5-yl, 2-methylsulphinyl-1,3,4-thiadiazol-5-yl, 2-ethylthio-1,3,4-thiadiazol-5-yl, 2-ethylsulphinyl-1,3,4-thiadiazol-5-yl, 2-methylsulphonyl-1,3,4-thiadiazol-5-yl, 2-ethylsulphonyl-1,3,4-thiadiazolyl-5-yl, 2-carbamoylmethyl-1,3,4-thiadiazol-5-yl, 2-carbamoyl-1,3,4-thiadiazol-5-yl, 2-methoxymethyl-1,3,4-thiadiazol-5-yl, 2-ethoxymethyl-1,3,4-thiadiazol-5-yl,2 - acetamido - 1,3,4 - thiadiazol - 5 - yl,2 - methylamino - 1,3,4 - thiadiazol - 5 - yl,2 - (N - methylacetamido) - 1,3,4 - thiadiazol - 5 - yl,2 - isobutylamino - 1,3,4 - thiadiazol - 5 - yl,2 - piperidino - 1,3,4 - thiadiazol - 5 - yl,2 - pyrrolidino - 1,3,4 - thiadiazol - 5 - yl,2 - aminomethyl - 1,3,4 - thiadiazol - 5 - yl,2 - acetamidomethyl - 1,3,4 - thiadiazol - 5 - yl,2 - benzamido - 1,3,4 - thiadiazol - 5 - yl,2 - ($\beta$ - piperidinoethyl) - 1,3,4 - thiadiazol - 5 - yl,2 - (2 - pyridylamino) - 1,3,4 - thiadiazol - 5 - yl,2 - (3 - pyridylamino) - 1,3,4 - thiadiazol - 5 - yl,2 - (1,3 - thiazol - 2 - yl - amino) - 1,3,4 - thiadiazol - 5 - yl,2 - (1,3,4 - triazolyl - 2 - amino) - 1,3,4 - thiadiazol - 5 - yl,2 - (tetrazolyl - 5 - amino) - 1,3,4 - thiadiazol - 5 - yl,2 - dimethylaminomethyl - 1,3,4 - thiadiazol - 5 - yl,2 - methylamino-

0127847

methyl - 1,3,4 - thiadiazol - 5 - yl,2 - ethyl - 1,3,4 - thiadiazol - 5 - yl,2 - trifluoromethyl - 1,3,4 - thiadiazol - 5 - yl,2 - mercapto - 1,3,4 - thiadiazol - 5 - yl,2 - methylthio - 1,3,4 - thiadiazol - 5 - yl,2 - (2 - pyridyl) - 1,3,4 - thiadiazol - 5 - yl,2 - (3 - pyridyl) - 1,3,4 - thiadiazol - 5 - yl,2 - (4 - pyridyl) - 1,3,4 - thiadiazol - 5 - yl,2 - (2 - thienyl) - 1,3,4 - thiadiazol - 5 - yl,2 - (2 - furyl) - 1,3,4 - thiadiazol - 5 - yl,2 - (3 - furyl) - 1,3,4 - thiadiazol - 5 - yl,2 - methyl - 1,3,4 - thiadiazol - 5 - yl,2 - isopropyl - 1,3,4 - thiadiazol - 5 - yl,2 - (4 - methoxyphenyl) - 1,3,4 - thiadiazol - 5 - yl,2 - (4 - chlorophenyl) - 1,3,4 - thiadiazol - 5 - yl,2 - (1 - naphthyl) - 1,3,4 - thiadiazol - 5 - yl,2 - (2 - quinolyl) - 1,3,4 - thiadiazol - 5 - yl,2 - (1 - isoquinolyl) - 1,3,4 - thiadiazol - 5 - yl,2 - (β - methoxycarbonylpropionylamido) - 1,3,4 - thiadiazol - 5 - yl, 2 - (β - carboxypropionyl - amido) - 1, 3, 4 - thiadiazol - 5 - yl,2 - carboxymethoxymethyl - 1,3,4 - thiadiazol - 5 - yl,2 - ethoxycarbonylmethyl - 1,3,4 - thiadiazol - 5 - yl,2 - carboxymethyl - 1,3,4 - thiadiazol - 5 - yl,2 - (α - carboxyacetamido) - 1,3,4 - thiadiazol - 5 - yl,2 - (α - cyanoacetamido) - 1,3,4 - thiadiazol - 5 - yl,2 - (methoxycarbamoyl) - acetamido - 1,3,4 - thiadiazol - 5 - yl,2 - (N,N - dimethylcarbamoylmethyl) - 1,3,4 - thiadiazol - 5 - yl, 2 - (N,N - diethylcarbamoyl - methyl) - 1,3,4 - thiadiazol - 5 - yl, 2 - (N,N - dipropyl - carbamoylmethyl) - 1,3,4 - thiadiazol - 5 - yl, 2 - (N,N - dibutylcarbamoylmethyl) - 1,3,4 - 5 - yl, 2 - (2 - (2 - acetamidoethyl) - 1,3,4 - thiadiazol - 5 - yl,2 - (2 - aminoethyl) - 1,3,4 - thiadiazol - 5 - yl,2 - hydroxymethyl -

1,3,4 - thiadiazol - 5 - yl,2 - (2 - hydroxyethyl) - 1,3,4 - thiadiazol - 5 - yl,2 - (isobutyryloxymethyl) - 1,3,4 - thiadiazol - 5 - yl,2 - (ethoxycarbonylmethoxymethyl) - 1,3,4 - thiadiazol - 5 - yl,2 - (carbamoylmethoxymethyl) - 1,3,4 - thiadiazol - 5 - yl,2 - (N - methylcarbamoyl) - 1,3,4 - thiadiazol - 5 - yl,2 - isobutyl - 1,3,4 - thiadiazol - 5 - yl,2 - methoxypropylaminocarbonylmethyl - 1,3,4 - thiadiazol - 5 - yl,2 - carboxyethyl - 1,3,4 - thiadiazol - 5 - yl,2 - sulfoethyl - 1,3,4 - thiadiazol - 5 - yl,2 - carboxy - 1,3,4 - thiadiazol - 5 - yl,2 - phenylamino - 1,3,4 - thiadiazol - 5 - yl,2 - o - carboxybenzoylamino - 1,3,4 - thiadiazol - 5 - yl,2 - (1 - carboxyethylthio) - 1,3,4 - thiadiazol - 5 - yl and 2 - (1 - carboxy - 1 - methylethyl) - 1,3,4 - thiadiazol - 5 - yl groups; for

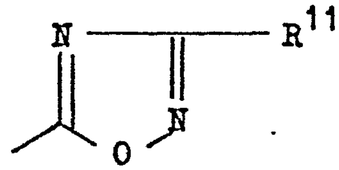

1,2,4 - oxadiazol - 5 - yl,3 - methyl - 1,2,4 - oxadiazol - 5 - yl and 3 - phenyl - 1,2,4 - oxadiazol - 5 - yl groups; for

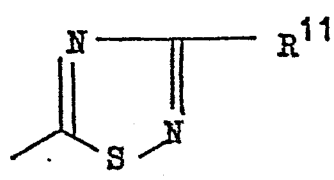

1,2,4 - thiadiazol - 5 - yl,3 - phenyl - 1,2,4 - thiadiazol - 5 - yl,3 - methylthio - 1,2,4 - thiadiazol - 5 - yl, 3 - methyl - 1,2,4 - thiadiazol - 5 - yl,3 - ethyl - 1,2,4 - thiadiazol-

5 - yl and 3-methylsulphenyl - 1,2,4 - thiadiazol - 5 - yl groups; for

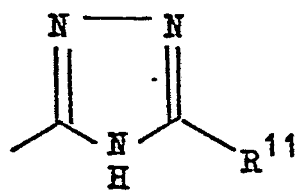

2 - methyl - 1H - 1,3,4 - triazol - 5 - yl,2 - ethyl - 1H - 1,3,4 - triazol - 5 - yl,2 - amino - 1H - 1,3,4 - triazol - 5 - yl,1H - 1,3,4 - triazol - 5 - yl,2 - trifluoromethyl - 1H - 1,3,4 - triazol - 5 - yl,2 - (β - piperidinoethyl) - 1H - 1,3,4 - triazol - 5 - yl,2 - (β - diethylaminoethyl) - 1H - 1,3,4 - triazol - 5 - yl,2 - hydroxy - 1H - 1,3,4 - triazol - 5 - yl,2 - (4 - pyridyl) - 1H - 1,3,4 - triazol - 5 - yl,2 - tert. - butyl - 1H - 1,3,4 - triazol - 5 - yl,2 - (3 - pyridyl) - 1H - 1,3,4 - triazol - 5 - yl,2 - (2 - pyridyl) - 1H - 1,3,4 - triazol - 5 - yl,2 - acetamido - 1H - 1,3,4 - triazol - 5 - yl,2 - propionylamido - 1H - 1,3,4 - triazol - 5 - yl,2 - benzamido - 1H - 1,3,4 - triazol - 5 - yl,2 - (2 - thienyl) - 1H - 1,3,4 - triazol - 5 - yl,2 - (2 - furyl) - 1H - 1,3,4 - triazol - 5 - yl,2 - (3 - furyl) - 1H - 1,3,4 - triazol - 5 - yl,2 - methoxymethyl - 1H - 1,3,4 - triazol - 5 - yl,2 - (4 - sulfamoylphenyl) - 1H - 1,3,4 - triazol - 5 - yl,2 - phenyl - 1H - 1,3,4 - triazol - 5 - yl,2 - (4 - methoxyphenyl) - 1H - 1,3,4 - triazol - 5 - yl,2 - (4 - chlorophenyl) - 1H - 1,3,4 - triazol - 5 - yl,2 - (2 - methyl pyrid - 4 - yl) - 1H - 1,3,4 - triazol - 5 - yl, 2 - ethoxymethyl - 1H - 1,3,4 - triazol - 5 - yl,2 - (2 - ethoxyethyl) -

0127847

1H - 1,3,4 - triazol - 5 - yl,2 - aminoethyl - 1H - 1,3,4 - triazol - 5 - yl,2 - acetamidomethyl - 1H - 1,3,4 - triazol - 5 - yl,5 - ethoxycarbonylmethyl - 1H - 1,3,4 - triazol - 5 - yl,2 - (β - carbomethoxypropionylamido) - 1H - 1,3,4 - triazol - 5 - yl,2 - carboxymethyl - 1H - 1,3,4 - triazol - 5 - yl,2 - carboxymethoxymethyl - 1H - 1,3,4 - triazol - 5 - yl,2 - ethoxycarbonylmethoxymethyl - 1H - 1,3,4 - triazol - 5 - yl,2 - ethoxycarbonyl - 1H - 1,3,4 - triazol - 5 - yl,2 - carbamoyl - 1H - 1,3,4 - triazol - 5 - yl,2 - carbamoylmethoxymethyl - 1H - 1,3,4 - triazol - 5 - yl,2 - (N - ethylcarbamoylmethoxymethyl) - 1H - 1,3,4 - triazol - 5 - yl,2 - hydroxy - 1H-1,3,4 - triazol - 5 - yl, 2-aminomethyl-1H-1,3,4-triazol-5-yl and 2-formamido-1H-1,3,4-triazol-5-yl groups; for

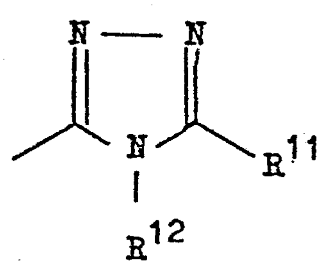

in which $R^{12} \neq$ hydrogen

2 - amino - 1 - methyl - 1,3,4 - triazol - 5 - yl,1 - methyl - 1,3,4 - triazol - 5 - yl,1 - methyl - 2 - trifluoromethyl - 1,3,4 - triazol - 5 - yl,1,2 - dimethyl - 1,3,4 - triazol - 5 - yl,2 - hydroxy - 1 - methyl - 1,3,4 - triazol - 5 - yl,1 - methyl - 2 - (3 - pyridyl) - 1,3,4 - triazol - 5 - yl,1 - methyl - 2 - (4 - pyridyl) - 1,3,4 - triazol - 5 - yl,2 - (2 - furyl) - 1 - methyl - 1,3,4 - triazol - 5 - yl,1 - methyl - 2 - (2 - thienyl) - 1,3,4 - triazol - 5 - yl,1 - methyl - 2 - (2 - pyridyl) -

0127847

1,3,4 - triazol - 5 - yl,2 - (3 - furyl) - 1 - methyl - 1,3,4 - triazol - 5 - yl,1 - methyl - 2 -phenyl - 1,3,4 - triazol - 5 - yl,1 - ethyl - 1,3,4 - triazol - 5 - yl,1 - ethyl - 2 - (3 - pyridyl) - 1,3,4 - triazol - 5 - yl,1 - ethyl - 2 - (4 - pyridyl)- 1,3,4 - triazol - 5 - yl,1 - ethyl - 2 - (2 - pyridyl) - 1,3,4 - triazol - 5 - yl,2 - (3 - furyl) - 1 - methyl - 1,3,4 - triazol - 5 - yl,1 - ethyl - 2 - trifluoromethyl - 1,3,4 - triazol - 5 - yl,1 - ethyl - 2 - (2 - furyl) - 1,3,4 - triazol - 5 - yl,1 - ethyl - 2 - (2 - thienyl) - 1,3,4 - triazol - 5 - yl,1,2 - diethyl - 1,3,4 - triazol - 5 - yl,1 - propyl - 2 - (3 - pyridyl)- 1,3,4 - triazol - 5 - yl,2 - (2 - furyl) - 1 - propyl - 1,3,4 - triazol - 5 - yl,1 - propyl - 1,3,4 - triazol - 5 - yl,1 - isopropyl - 1,3,4 - triazol - 5 - yl,1 - allyl - 1,3,4 - triazol - 5 - yl,1 - butyl - 1 - (2 - furyl) - 1,3,4 - triazol - 5 - yl,1 - cyclohexyl - 1,3,4 - triazol - 5 - yl,1 - benzyl - 1,3,4 - triazol - 5 - yl,1 - hydroxy - 1,3,4 - triazol - 5 - yl,1 - methoxymethyl - 1,3,4 - triazol - 5 - yl,1 - phenyl - 1,3,4 - triazol - 5 - yl,2 - methyl - 1 - phenyl - 1,3,4 - triazol - 5 - yl,1 - (4 - chlorophenyl) - 1,3,4 - triazol - 5 - yl,2 - hydroxy - 1 - phenyl - 1,3,4 - triazol - 5 - yl,2 - amino - 1 - phenyl - 1,3,4 - triazol - 5 - yl,1 - phenyl - 2 - propyl - 1,3,4 - triazol - 5 - yl,2 - (1 - piperidinomethyl) - 1 - phenyl - 1,3,4 - triazol - 5 - yl,2 - (β - diethylaminoethyl) - 1 - phenyl - 1,3,4 - triazol - 5 - yl,1 - (4 - ethoxyphenyl) - 2 - (β - piperidinoethyl) - 1,3,4 - triazol - 5 - yl,1 - (4 - chlorophenyl) - 2 - dimethylaminomethyl - 1,3,4 - triazol - 5 - yl,1 - phenyl - 2 - (4 - pyridyl) - 1,3,4 - triazol - 5 - yl,1 -

(3 - pyridyl) - 1,3,4 - triazol - 5 - yl,2 - hydroxy - 1 - (2 - pyridyl) - 1,3,4 - triazol - 5 - yl,1 - (4 - pyridyl) - 1,3,4 - triazol - 5 - yl,1 - (2 - pyridyl) - 1,3,4 - triazol - 5 - yl,1 - (4 - ethoxyphenyl) - 2 - hydroxy - 1,3,4 - triazol - 5 - yl,1 - (4 - chlorophenyl) - 2 - hydroxy - 1,3,4 - triazol - 5 - yl,1 - amino - 2 - trifluoromethyl - 1,3,4 - triazol - 5 - yl,1 - amino - 2 - (2 - hydroxyphenyl) - 1,3,4 - triazol - 5 - yl,1 - amino - 2 - phenyl - 1,3,4 - triazol - 5 - yl,1 - amino - 2 - (4 - fluorophenyl) - 1,3,4 - triazol - 5 - yl,1 - amino - 2 - (2 - bromophenyl) - 1,3,4 - triazol - 5 - yl,1 - amino - 2 - (2 - methoxyphenyl) - 1,3,4 - triazol - 5 - yl,1 - amino - 2 - (4 - pyridyl) - 1,3,4 - triazol - 5 - yl,1 - amino - 2 - (2 - thienyl) - 1,3,4 - triazol - 5 - yl,1 - amino - 2 - cyclohexyl - 1,3,4 - triazol - 5 - yl,1 - amino - 2 - methyl - 1,3,4 - triazol - 5 - yl, 2 - ethyl - 1 - amino - 1,3,4 - triazol - 5 - yl,2 - phenyl - 1 - phenylamino - 1,3,4 - triazol - 5 - yl,2 - ethyl - 1 - ethylamino - 1,3,4 - triazol - 5 - yl,1 - amino - 2 - methylthio - 1,3,4 - triazol - 5 - yl,1 - amino - 2 - mercapto - 1,3,4 - triazol - 5 - yl,1 - amino - 2 - benzyl - 1,3,4 - triazol - 5 - yl,1 - acetamido - 2 - ethyl - 1,3,4 - triazol - 5 - yl,2 - ethyl - 1 - (2,5 - dimethyl - pyrrol - 1 - yl) - 1,3,4 - triazol - 5 - yl,2 - ethyl - 1 - (pyrrol - 1 - yl) - 1,3,4 - triazol - 5 - yl,1 - methyl - 2 - (4 - sulfamoylphenyl) - 1,3,4 - triazol - 5 - yl,1 - allyl - 2 - (4 - sulfamoylphenyl) - 1,3,4 - triazol - 5 - yl,1 - phenyl - 2 - (4 - sulfamoylphenyl) - 1,3,4 - triazol - 5 - yl,1 - amino - 1,3,4 - triazol - 5 - yl,1 - (4 - ethoxyphenyl)-2 - (4 - pyridyl) - 1,3,4 - triazol - 5 - yl,1 - (4 - ethoxyphenyl) - 2 - (3 - pyridyl) - 1,3,4 - triazol - 5 - yl,1 - (4 - methoxyphenyl) - 2 - (4 - pyridyl) - 1,3,4 - triazol - 5 - yl,1 - (4 - ethoxyphenyl) - 2 - phenyl - 1,3,4 - triazol - 5 - yl,1 - (4 - ethoxyphenyl) - 2 - (4 - aminophenyl) - 1,3,4 - triazol - 5 - yl,1,2 - diphenyl - 1,3,4 - triazol - 5 - yl,1,2 - di - p - tolyl - 1,3,4 - triazol - 5 - yl,1 - allyl - 2 - phenyl - 1,3,4 - triazol - 5 - yl,1 - amino - 2 - carboxymethyl - 1,3,4 - triazol - 5 - yl,2 - carboxymethyl - 1 - methyl - 1,3,4 - triazol - 5 - yl,2 - carboxymethoxymethyl - 1 - methyl - 1,3,4 - triazol - 5 - yl,1 - carboxymethyl - 2 - trifluoromethyl - 1,3,4 - triazol - 5 - yl,1 - sulfoethyl - 2 - trifluoromethyl - 1,3,4 - triazol - 5 - yl,2 - ethoxycarbonylmethoxymethyl - 1 - methyl - 1,3,4 - triazol - 5 - yl,2 - carbamoyl - 1 - methyl - 1,3,4 - triazol - 5 - yl,2 - carbamoylmethoxymethyl - 1 - methyl - 1,3,4 - triazol - 5 - yl,2 - ethoxycarbonyl - 1 - (4 - methoxybenzyl) - 1,3,4 - triazol - 5 - yl,1 - amino - 2 - carboxymethylthio - 1,3,4 - triazol - 5 - yl,2-butyl-1-methyl-1,3,4 - triazol - 5 - yl, 2-amino-1-methyl-1,3,4-triazol-5-yl, 2-acetamido-1-methyl-1,3,4-triazol-5-yl, 2-formimido-1-methyl-1,3,4-triazol-5-yl, 2-methoxymethyl-1-methyl-1,3,4-triazol-5-yl, 2-ethoxymethyl-1-methyl-1,3,4-triazol-5-yl, 2-aminomethyl-1-methyl-1,3,4-triazol-5-yl, 2-acetamido-1-methyl-1,3,4-triazol-5-yl, 2-methoxycarbonyl-1-methyl-1,3,4-triazol-5-yl, 2-carbamoylmethyl-1-methyl-1,3,4-triazol-5-yl, 1-ethyl-2-methyl-1,3,4-triazol-5-yl, 1-ethyl-2-hydroxy-1,3,4-triazol-5-yl, 2-amino-1-ethyl-1,3,4-triazol-5-yl, 2-acetamido-1-ethyl-1,3,4-triazol-5-yl,

2-carbamoyl-1-ethyl-1,3,4-triazol-5-yl, 1-ethyl-2-methoxymethyl-1,3,4-triazol-5-yl, 2-aminomethyl-1-ethyl-1,3,4-triazol-5-yl,1-amino-2-methylsulphinyl-1,3,4-triazol-5-yl, 1-acetamido-2-methyl-1,3,4-triazol-5-yl groups and 1 - carbamoylmethyl - 2 - trifluoromethyl - 1,3,4 - triazol - 5 - yl groups; for

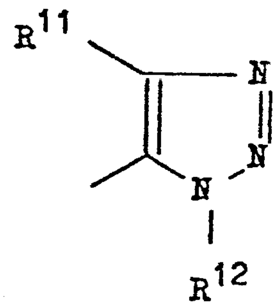

1H - 1,2,3 - triazol - 5 - yl,1 - methyl - 1,2,3 - triazol - 5 - yl,1,4 - dimethyl - 1,2,3 - triazol - 5 - yl,1H - 4 - methyl - 1,2,3 - triazol - 5 - yl,1,4 - diethyl - 1,2,3 - triazol - 5 - yl,4 - carboxy - 1H - 1,2,3 - triazol - 5 - yl,4 - (2 - carboxyethyl) - 1H - 1,2,3 - triazol - 5 - yl,4 - (3 - carboxypropyl) - 1H - 1,2,3 - triazol - 5 - yl,4 - (1 - carboxy - 1 - methylethyl) - 1H - 1,2,3 - triazol - 5 - yl,4 - (2 - carboxy - 2 - methylpropyl) - 1H - 1,2,3 - triazol - 5 - yl,4 - N - methylcarbamoyl - 1H - 1,2,3 - triazol - 5 - yl,4 - N - ethylcarbamoyl - 1H - 1,2,3 - triazol - 5 - yl,4 - N - propylcarbamoyl - 1H - 1,2,3 - triazol - 5 - yl and 4 - N - butylcarbamoyl - 1H - 1,2,3 - triazol - 5 - yl groups.

d) A triazolyl radical of the general formula 4

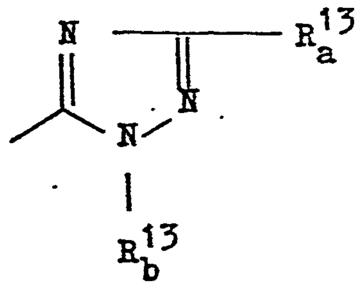

(4)

in which $R^{13}_a$ and $R^{13}_b$, which may be identical or different, each denotes a straight chain or branched alkyl or straight chain or branched alkenyl group, an alkoxyalkyl group, a hydroxyl group, a hydroxyalkyl group, alkoxy or alkylcarbonyl group, or an optionally substituted phenyl radical, and furthermore $R^{13}_b$ can represent a hydrogen atom.

Examples which may be mentioned are, in particular: 1-methyl-1,2,4-triazol-5-yl, 1-butyl-1,2,4-triazol-5-yl, 1-phenyl-1,2,4-triazol-5-yl, 1-methoxymethyl-1,2,4-triazol-5-yl, 1,3-dimethyl-1,2,4-triazol-5-yl, 1-allyl-1,2,4-triazol-5-yl, 3-hydroxy-1-methyl-1,2,4-triazol-5-yl, 3-hydroxy-1-isopropyl-1,2,4-triazol-5-yl, 3-hydroxy-1-phenyl-1,2,4-triazol-5-yl, 3-ethyl-1-methyl-1,2,4-triazol-5-yl, and 3-methyl-1-phenyl-1,2,4-triazol-5-yl groups.

e) A pyrimidinyl or pyridazinyl radical of the general formulae 5a, 5b and 5c

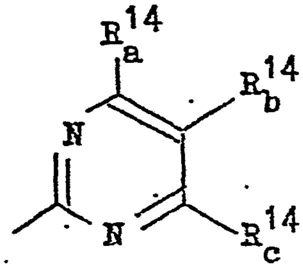

( 5a)

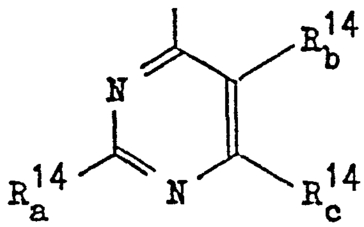

(5b)

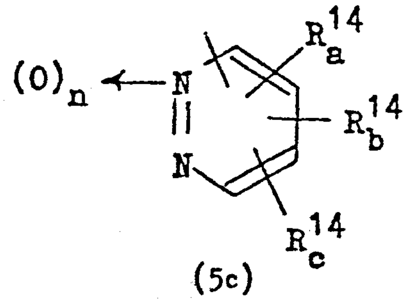

(5c)

in which $R^{14}_{a}$ to $R^{14}_{c}$, which may be identical or different, each denotes a hydrogen or halogen atom, a straight chain or branched alkyl or straight chain or branched alkenyl group, a mercapto group, an alkylthio group, a lower alkylsulphinyl group, a lower alkylsulphonyl group, a hydroxy group, a hydroxyalkyl group, an alkoxy or alkylcarbonyl group, an alkoxyalkyl group, an amino group which can be optionally substituted by one or two alkyl radicals, a carboxyalkyl group, a carboxyl or cyano group, an alkoxycarbonyl group, a carbamoyl group which can be optionally substituted by one or two alkyl groups, which in turn can form a carbocyclic ring with 5 - 7 C atoms which may be optionally interrupted by nitrogen and/or sulfur atoms, an alkoxycarbonylalkylamido or carboxyalkylamido group, a cyanoalkyl group, an optionally substituted phenyl radical or a carboxyalkylthio group; and the heterocyclic rings can also be partially hydrogenated and n represents 0 or 1.

Examples which may be mentioned are, in particular:

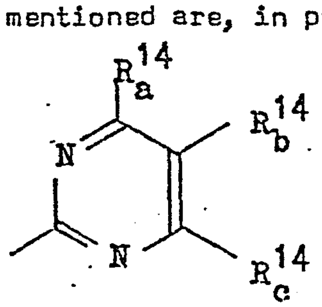

4,6 - diamino - pyrimidin - 2 - yl,4 - amino - 6 - hydroxy - pyrimidin - 2 - y,5,6 - diamino - 4 - hydroxy - pyrimidin - 2 - yl,4,5 - diamino - pyrimidin - 2 - yl,4 - hydroxy-6 - methyl - pyrimidin - 2 - yl,4,6 - dihydroxy - pyrimidin - 2 - yl,4 - hydroxy - pyrimidin - 2 - yl,4 - hydroxy - 6 - propyl - pyrimidin - 2 - yl, pyrimidin - 2 - yl,4 - methyl - pyrimidin - 2 - yl,4,6 - dimethyl - pyrimidin - 2 - yl,4 - mercapto - pyrimidin - 2 - yl,4 - methylthio - pyrimidin - 2 - yl,1,4,5,6 - tetrahydropyrimidin - 2 - yl,4 - hydroxy - 5 - carboxymethyl - 6 - methyl - pyrimidin - 2 - yl,4 - hydroxy - 5-carboxy - pyrimidin - 2 - yl,4 - amino - 5 - carboxy - pyrimidin - 2 - yl,4 - amino - 5 - methoxycarbonyl - pyrimidin - 2 - yl,4 - amino - 5 - ethoxycarbonyl - pyrimidin - 2 -yl,4 - hydroxy - 5 - carboxymethyl - pyrimidin - 2 - yl,4 - hydroxy - 5 - piperidinocarbonyl - pyrimidin - 2 - yl,4 - chloro - 5 - carboxy - pyrimidin - 2 - yl,4 - (β- carboxy - propionylamido) - 6 - hydroxy - pyrimidin - 2 - yl and 5 - cyanoethyl - 4 - hydroxy - 6 - methylpyrimidin - 2 - yl groups; for

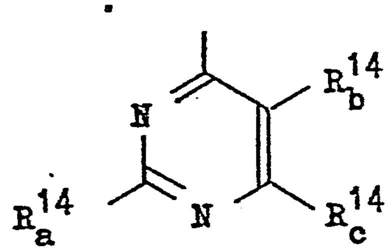

2 - hydroxy - pyrimidin - 4 - yl, pyrimidin - 4 - yl,5 - ethoxycarbonyl - 6 - methyl - 2 - phenyl - pyrimidin - 4 - yl,6 - ethoxy - 5 - ethoxycarbonyl - 2 - phenyl - pyrimidin - 4 - yl,5

ethoxycarbonyl - 6 - amino - 2 - phenyl - pyrimidin - 4 - yl,5 - cyano - 2 - hydroxy - 6 - methyl - pyrimidin - 4 - yl,5 - acetyl - 2,6 - dimethyl - pyrimidin - 4 - yl,5 - ethoxycarbonyl - 2,6 - dimethyl - pyrimidin - 4 - yl,2 - hydroxy - 6 - methyl - pyrimidin - 4 - yl,6 - mercapto - 2 - methyl - pyrimidin - 4 - yl,6 - mercaptopyrimidin - 4 - yl,2 - amino - 6 - mercapto - pyrimidin - 4 - yl,6 - mercapto - 2 - methylthio - pyrimidin - 4 - yl,6 - carboxymethylthio - pyrimidin - 4 - yl,6 - carboxymethylthio - 2 - methyl - pyrimidin - 4 - yl,2 - amino - 4 - carboxymethylthio - pyrimidin - 4 - yl, 6-methylthiopyrimidin-4-yl and 6-methylsulphinylpyrimidin-4-yl groups; and for

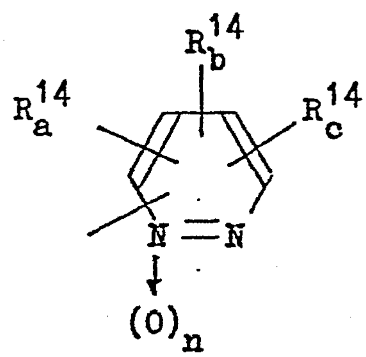

6 - methoxy - 2 - oxo - pyridazin - 3 - yl,6 - butoxy - 2 - oxo - pyridazin - 3 - yl,6-ethoxy - 2 - oxo - pyridazin - 3 - yl,2 - oxopyridazin - 3 - yl,6 - methyl - 1 - oxo - pyridazin - 3 - yl,6 - methyl - 2 - oxo - pyridazin - 3 - yl,pyridazin - 3 - yl,6 - hydroxy - pyridazin - 3 - yl,6 - chloro - 1 - oxopyridazin - 3 - yl,5 - ethoxycarbonyl - 6 - hydroxy - pyridazin - 3 - yl,5 - carboxy - 6 - hydroxy - pyridazin -

3 - yl,4 - ethoxycarbonyl - 6 - hydroxy - pyridazin - 3 - yl, 4 - methyl - 6 - hydroxy - pyridazin - 3 - yl,4 - ethyl - 6 - hydroxy - pyridazin - 3 - yl,5 - ethoxycarbonyl - 6 - hydroxy - 4 - methyl - pyridazin - 3 - yl,5 - ethoxycarbonyl - 4 - ethyl - 6 - hydroxy - pyridazin - 3 - yl,4 - ethoxycarbonyl - 5 - ethyl - 6 - hydroxy - pyridazin - 3 - yl,4 - ethoxycarbonyl - 6 - hydroxy - 5 - methylpyridazin - 3 - yl, 6 - mercapto - pyridazin - 3 - yl and 6 - chloro - 2 - oxy - pyridazin - 3 - yl groups.

f) A tetrazolyl radical of the general formula 6a or 6b

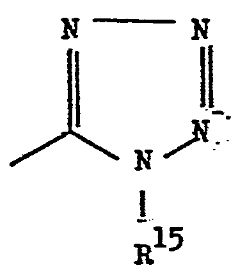

(6a)

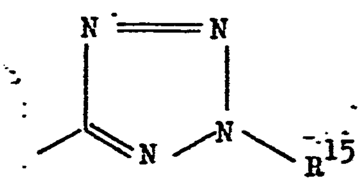

(6b)

in which $R^{15}$ represents a hydrogen atom, a straight chain or branched alkyl or branched or straight chain alkenyl group, an alkoxyalkyl group, an optionally substituted aryl or heteroaryl radical, a carbocyclic ring with 5 - 7 C atoms, arylalkyl, carboxyalkyl, cyanoalkyl, or an alkoxycarbonylalkyl, sulfoalkyl, sulfamoylalkyl, alkylsulfoalkyl, alkylsulfamoylalkyl, dialkylsulfamoylalkyl, carbamoylalkyl, alkylcarbamoylalkyl, dialkylcarbamoylalkyl, aminoalkyl, hydroxyalkyl, alkylaminoalkyl alkanoyloxyalkyl, sulphamoyl, dialkylaminoalkyl, acylamidoalkyl or alkoxyalkylcarbonyloxy group.

Examples which may be mentioned are, in particular: tetrazol - 5 - yl, 1 - ethyltetrazol - 5 - yl, 1 - allyl - tetrazol - 5 - yl, 1 - phenyl - tetrazol - 5 - yl, 1 - butyl - tetrazol - 5 - yl,1 - benzyl - tetrazol - 5 - yl,1 -(4 - fluorophenyl) - tetrazol - 5 - yl,1 - isopropyl - tetrazol - 5 - yl,1 - (2 - pyridyl) - tetrazol - 5 - yl,1 - cyclohexyltetrazol - 5 - yl,1 - (2,4 - dichlorophenyl) - tetrazol - 5 - yl,1 - (2 - tolyl) - tetrazol - 5 - yl,1 - (4 - nitrophenyl) - tetrazol - 5 - yl,1 - (4 - dimethylaminophenyl) - tetrazol - 5 - yl,1 - methoxymethyl - tetrazol - 5 - yl,1 - methyl - tetrazol - 5 - yl,1 - propyltetrazol - 5 - yl,1 - cyclopentyl - tetrazol - 5 - yl,1 - (4 - chlorophenyl) - tetrazol - 5 - yl,1 - carboxymethyl - tetrazol - 5 - yl,1 - carboxyethyl - tetrazol - 5 - yl,1 - cyanomethyl - tetrazol - 5 - yl,1 - sulfomethyl - tetrazol - 5 - yl,1 - sulfoethyl - tetrazol - 5 - yl,1 - sulfopropyl - tetrazol - 5 - yl,1 - sulfamoyl - tetrazol - 5 - yl,1 - sulfamoylethyl-tetrazol - 5 - yl,1 - (2 - N,N - dimethyl - sulfamoylethyl) - tetrazol - 5 - yl,1 - (3 - sulfamoylpropyl) - tetrazol - 5 - yl,1 - (2 - sulfo - 1 - methylethyl) - tetrazol - 5 - yl,1 - (4 - sulfobutyl) - tetrazol - 5 - yl,1 - (2 - carbamoylethyl) - tetrazol - 5 - yl,1 - (N-methylcarbamoylmethyl) - tetrazol - 5 - yl,1 - (N,N - dimethylcarbamoylmethyl)-tetrazol - 5 - yl,1 - (2 - carbamoylpropyl) - tetrazol - 5 - yl,1 - (3 - carboxypropyl)-tetrazol - 5 - yl,1 - (2 - carboxy - 1 - methylethyl) - tetrazol - 5 - yl,1 - (4 - dimethyl-aminophenyl) - tetrazol - 5 - yl,1 - acetamidoethyl - tetrazol - 5 - yl,1 - (2 - hydroxyethyl) - tetrazol - 5 - yl,1 - ethoxycarbonylmethyl -

tetrazol - 5 - yl,1 - (2 - aminoethyl) - tetrazol - 5 - yl,1 - (3 - methoxypropyl) - tetrazol - 5 - yl,1-carboxymethyltetrazol-5-yl, 1-carbamoylmethyltetrazol-5-yl, 1-dimethylaminoethyl-tetrazol-5-yl, 1-acetoxyethyltetrazol-5-yl,1-hexanoyloxyethyl-tetrazol-5-yl, 1-methoxyacetoxyethyltetrazol-5-yl, and 1-sulphamoylmethyltetrazol-5-yl groups.

g) A tetrazolopyridazinyl radical of the general formula 7.

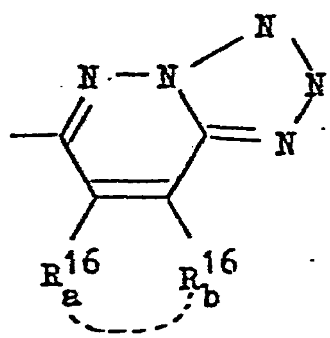

(7)

in which $R_a^{16}$ and $R_b^{16}$, which may be the same or different, each represents a hydrogen or halogen atom, a straight chain or branched alkyl or branched or straight chain alkenyl group, an alkoxyalkyl group, an optionally substituted aryl or heteroaryl radical, a carbocyclic ring with 5 - 7 C atoms, arylalkyl, carboxyalkyl, cyanoalkyl, or an alkoxycarbonyl-alkyl, sulfoalkyl, sulfamoylalkyl, alkylsulfoalkyl, alkyl-sulfamoylalkyl, dialkylsulfamoylalkyl, carbamoylalkyl, alkylcarbamoylalkyl, dialkylcarbamoylalkyl, aminoalkyl,alkylaminoalkyl, hydroxyalkyl, alkylamidoalkyl, alkoxyalkylcarbonyl-amino, hydroxyalkylaminoalkyl, hydroxyalkylthioalkyl, alkylthioalkyl, alkylsuphinylalkyl or alkylsulphonylalkyl group, or a group

$-NH_2$, $-NHR^4$, $-NR^4R^4$, $-NHCOR^4$, $-NHCO_2R^4$, $-NHOR$,

$-NHSO_2R^4$, $-NHSO_3H$, $-CO_2H$, $-CO_2R^4$, $-CONH_2$, $-COR^4$,

$-CONHR^4$, $-CONR^4R^4$, $-CN$, $-CONHNH_2$, $-CONHOH$,

$NO_2$, $-NHCH_2CO_2H$, $NHCH_2CO_2R^4$, $NHCH_2CONH_2$, $NHCH_2CONHR^4$

in which the or each group $R^4$ is, independently, as defined above i.e. represents an alkyl group preferably having from 1 to 4 carbon atoms or an aryl group, or $R^{19}$ and $R^{20}$ together with the carbon atoms to which they are attached, form an aromatic or non-aromatic, carbocyclic or heterocylic ring.

An alkyl group $R^4$ may be substituted, for example, by one or more substituents selected from halogen atoms, amino groups, hydroxy groups and carbamoyl groups.

A ring formed between $R^{16}_a$, $R^{16}_b$ and the carbon atoms to which they are attached may have from 5 to 9 members, generally 5, 6 or 7 members. The ring may contain one or more heteroatoms, for example, selected from nitrogen, oxygen and sulphur atoms.

The ring may be a non-aromatic carbocylic ring, for example, a cyclohexenyl ring, or an aromatic carbocylic ring, for example, a benzo ring. A heterocyclic ring may also be non-aromatic, for example, a piperidine ring, or aromatic, for example, a pyridyl ring. A ring may be substituted if desired, for example, one or more groups as defined above for $R^{16}_a$ and $R^{16}_b$.

Examples of tetrazolopyridazinyl radicals having a further fused ring at positions 7 and 8 are:-

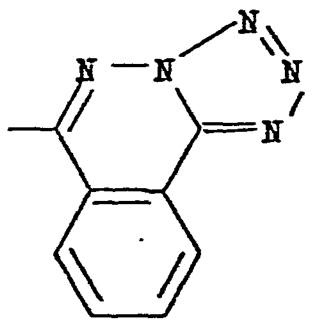

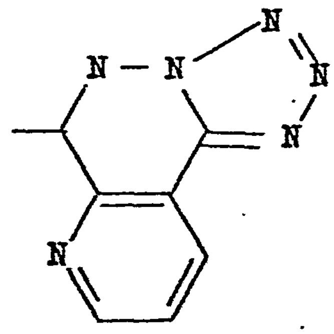

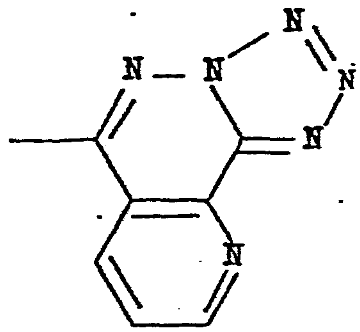

Examples of substituted radicals of formula 7 are 8-methyl-, 8-ethyl-, 8-propyl-, 8-chloro-, 8-fluoro-, 8-bromo-, 8-amino-, 8-methylamino-, 8-ethylamino-, 8-dimethylamino-, 8-acetylamino-, 8-methoxyacetylamino-, 8-methoxycarbonylamino-, 8-methoxycarbonylmethylamino-, 8-hydroxycarbonylmethylamino-, 8-methanesulphonylamino-, 8-hydroxysulphonylamino-, 8-cyano-, 8-hydroxycarbonyl-, 8-methoxycarbonyl-, 8-aminocarbonyl-, 8-methylaminocarbonyl-, 8-(2,2,2-trifluoroethylaminocarbonyl)-, 8-(2-hydroxyethylaminocarbonyl)-, 8-(2-aminoethylaminocarbonyl)-, 8-hydroxyaminocarbonyl-, 8-aminocarbonylmethylamino-, 8-methylaminocarbonylmethylamino-, 8-nitro-, 8-methylcarbonyl-, 8-cyanomethyl-, 8-hydroxycarbonylmethyl-, 8-methoxycarbonylmethyl-, 8-aminocarbonylmethyl-, 8-methylaminocarbonylmethyl-, 8-methylthiomethyl-, 8-methylsulphonylmethyl-, 8-methylsulphinylmethyl-, 8-aminosulphonylmethyl-, 8-hydroxysulphonylmethyl-,

8-aminomethyl-, 8-acetylaminomethyl-, 8-methoxymethyl-, 8-methylaminomethyl-, 8-hydroxyethylaminomethyl-, and 8-hydroxyethylthiomethyl-tetrazolo/1,5-b/ pyridazin-6-yl radicals, and the corresponding 7-substituted radicals.

h) A 1,2,4-triazolo/4,3-b/tetrazinyl radical of the general formula 8

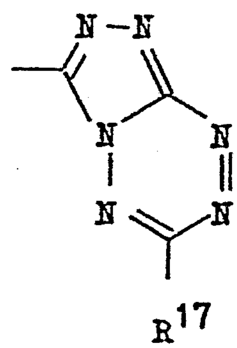

(8)

in which $R^{17}$ represents a hydrogen atom, an alkyl or alkenyl group, an alkoxyalkyl or halo group, a cyano or cyanoalkyl group, a hydroxycarbonyl or an alkoxycarbonyl group, a carbamoyl or carboxamido group, an amino or alkanoylamino group, a hydroxycarbonylalkyl group, a carboxamidoalkyl group, a hydroxyalkyl, alkanoyloxycarbonyl or trifluoromethyl group.

Examples which may be mentioned are, in particular:

1,2,4-triazolo/4,3-b/tetrazin-3-yl, 8-methyl-1,2,4-triazolo /4,3,b/-tetrazin-3-yl, 8-trifluoromethyl-1,2,4-triazolo/4,3,b/ tetrazin-3-yl, 8-ethyl-1,2,4-triazolo/4,3-b/tetrazin-3-yl, 8-allyl-1,2,4-triazolo-/4,3,b/tetrazin-3-yl, 8-methoxymethyl-1,2,4-triazolo/4,3-b/tetrazin-3-yl, 8-ethoxymethyl-1,2,4-triazolo[4,3-b]tetrazin-3-yl, 8-cyano-1,2,4-triazolo[4,3-b] tetrazin-3-yl, 8-cyanomethyl-1,2,4-triazolo[4,3-b]-tetrazin-3-yl, 8-carboxy-1,2,4-triazolo[4,3-b]tetrazin-3-yl, 8-methoxycarbonyl-1,2,4-triazolo[4,3-b]tetrazin-3-yl, 8-carboxamido-1,2,4-triazolo[4,3-b]tetrazin-3-yl, 8-amino-1,2,4-triazolo [4,3-b]tetrazin-3-yl,8-acetamido-1,2,4-triazolo[4,3-b]tetrazin-3-yl, 8-carboxymethyl-1,2,4-triazolo[4,3-b]tetrazin-3-yl, 8-carboxyethyl-1,2,4-triazolo[4,3-b]tetrazin-3-yl, 8-carboxamidomethyl-1,2,4-triazolo[4,3-b]tetrazin-3-yl, 8-hydroxymethyl-1,2,4-triazolo[4,3-b]tetrazin-3-yl, and 8-acetoxymethyl-1,2,4-triazolo[4,3-b]tetrazin-3-yl groups.

i) A 1,2,4-triazolo[2,3-a]pyrimidinyl radical of the general formula 9

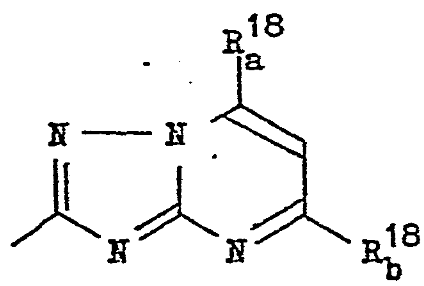

(9)

in which $R^{18}_a$ and $R^{18}_b$, which may be the same or different, each represents a hydrogen atom, a straight or branched chain alkyl group, an amino or alkanoylamino group, or a phenyl group.

Examples which may be mentioned are: 1,2,4-triazolo[2,3-a] pyrimidin-2-yl, 4,6-dimethyl-1,2,4-triazolo[2,3-a]pyrimidin-2-yl, 4,6-diamino-1,2,4-triazolo[2,3-a]pyrimidin-2-yl, and 4,6-diacetamido-1,2,4-triazolo[2,3-a]pyrimidin-2-yl groups.

k) A 1,2,4-triazolo/3,4-b/pyridazinyl radical of the general formula 10

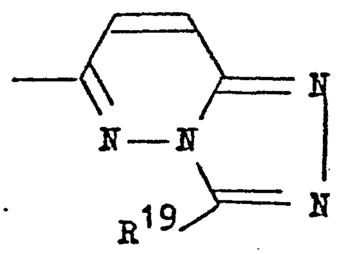

(10)

in which $R^{19}$ represents a hydroxy group, an alkyl, alkenyl, alkoxyalkyl, halogenoalkyl, cyano or cyanoalkyl group, a carboxamido, amino or alkanoylamino group, a hydroxycarbonylalkyl, carboxamidoalkyl, hydroxyalkyl, alkanoylalkyl, alkanoyloxyalkyl or carbamoyl group.
Examples which may be mentioned are: 1,2,4-triazolo/3,4-b/pyridazin-6-yl, 5-methyl-1,2,4-triazolo/3,4-b/pyridazin-6-yl, 5-trifluoromethyl-1,2,4-triazolo/4,3-b/pyridazin-6-yl, 5-ethyl-1,2,4-triazolo/3,4-b/pyridazin-6-yl, 5-methoxymethyl-1,2,4-triazolo/3,4-b/pyridazin-6-yl, 5-cyano-1,2,4-triazolo/3,4-b/pyridazin-6-yl, 5-cyanomethyl-1,2,4-triazolo/3,4-b/pyridazin-6-yl, 5-carboxamido-1,2,4-triazolo/3,4-b/pyridazin-6-yl, 5-amino-1,2,4-triazolo/3,4-b/pyridazin-6-yl, 5-acetamido-1,2,4-triazolo/3,4,b/pyridazin-6-yl, 8-carboxymethyl-1,2,4-triazolo/3,4-b/pyridazin-6-yl, 8-carboxamidomethyl-1,2,4-triazolo/3,4-b/pyridazin-6-yl, 8-hydroxymethyl-1,2,4-triazolo/3,4-b/pyridazin-6-yl, and 8-actoxymethyl-1,2,4-triazolo/3,4-b/pyridazin-6-yl groups.

l) A pyrazolo[3,4-d]pyrimidin-6-yl group of the formula 11

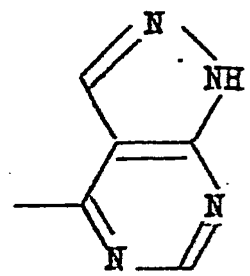

(11)

m) A 1,2,4-triazolo[4,3-a]pyridin-3-yl group of the formula 12

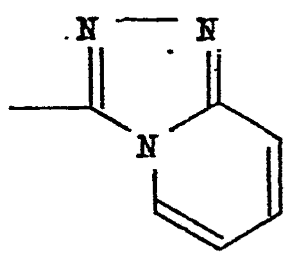

(12)

If in the definition of the radicals $R^5$ to $R^{19}$ substituents or references to particular ring systems occur which are not explained in more detail, they correspond to the preceding statements concerning the general substitution possibilities of the radical $R^2$ in the meaning of "heterocyclic radical". At the same time, they are illustrated further by the particular

accompanying tabular summary of specific radicals.

Further examples of heterocyclic $R^2$ radicals which may be mentioned are:

1,2,3-thiadiazol-5-yl, 1,2,4-thiadiazol-3-yl, 1,2,5-thiadiazol-3-yl, 1,2,3-oxadiazol-5-yl, 4,5-dimethyl-oxazol-2-yl, 4-phenyl-oxazol-2-yl, benzoxazol-2-yl, oxazolin-2-yl, imidazol-2-yl, imidazolin-2-yl, benzimidazolin-2-yl, 1-methyl-imidazolin-2-yl, 2-furyl, 2-thiophenyl, 2-pyrrolyl, 2-thiazolinyl, 3-isoxazolyl, 3-pyrazolyl, thiatriazol-5-yl, purinyl, pyrazinyl, 2-methylmercapto-6-phenyl-1,3,5-triazin-4-yl, 5-methyl-6-hydroxy-1,3,4-triazin-2-yl, 5-phenyl-4H-1,3,4-thiadiazin-2-yl, 5-hydroxy-4H-1,3,4-thiadiazin-2-yl, and 3-hydroxy-/4,5-b/-pyridazin-6-yl, 1-carbamoylmethyltetrazol-5-yl, 1-hydroxy-aminocarbonylmethyl-tetrazol-5-yl, 1-diazanylcarboxymethyl-tetrazol-5-yl, 1-(1-methyl-1-ethylenediazanecarboxymethyl)-tetrazol-5-yl, 1-carbamimidoylmethyl-tetrazol-5-yl, 1-(N-methyl-carbamimidoylmethyl)-tetrazol-5-yl, 1-(N,N,N-trimethyl-carbamimidoylmethyl)-tetrazol-5-yl, 1-/(1-iminoethyl)aminoethyl/-tetrazol-5-yl, 1-/(N-methyl-iminomethyl)aminoethyl/-tetrazol-5-yl and 1-(N,N,N-ethylenecarbamimidoylmethyl)-tetrazol-5-yl groups.

In the present specification, the term halogen includes chlorine, bromine, fluorine and iodine; and the term 'lower' denotes a group or molecule having up to 6 and especially up to 4 carbon atoms.

In a compound of formula I, the carboxyl group may be esterified with a carboxyl esterifying group R.

An esterified carboxyl group -COOR is, for example, an ester formed with an unsubstituted or substituted aliphatic, cycloaliphatic, cycloaliphatic-aliphatic, aryl, araliphatic, heterocyclic or heterocyclic-aliphatic alcohol having up to 20 carbon atoms, or is, for example, a silyl or stannyl ester.

R may represent, for example, a straight or branched chain substituted or unsubstituted alkyl, alkenyl or alkynyl group having up to 18 carbon atoms, preferably up to 8 carbon atoms, and especially up to 4 carbon atoms, for example, a methyl, ethyl, n-propyl, iso-propyl, n-butyl, sec-butyl, iso-butyl, tert-butyl, n-pentyl, n-hexyl, allyl, or vinyl group.

An aliphatic group R, especially a methyl group, may be substituted by a cycloalkyl, aryl or heterocyclic group, or R may itself represent a cycloalkyl, aryl or heterocyclic group.

A cycloaliphatic group R may have up to 18 carbon atoms and is, for example, a cyclopentyl, cyclohexyl or adamantyl group. An aryl group may have up to 12 carbon atoms and may have two or more fused rings. An aryl group R is, for example, an unsubstituted or substituted phenyl group, and an unsubstituted or substituted aralkyl group is, for example, a benzyl, p-nitrobenzyl, benzhydryl or p-methoxybenzyl group.

A heterocyclic group may have one or more heteroatoms, selected from oxygen, nitrogen and sulphur, and up to 14 atoms in total. A heterocyclic group is, for example, an oxygen-containing heterocyclic group, for example, a tetrahydropyranyl or phthalidyl group.

A stannyl group R may have up to 24 carbon atoms, for example, R may represent a stannyl group having three substituents, which may be the same or different, selected from alkyl, alkenyl, cycloalkyl, aryl, aralkyl, alkoxy and aralkoxy groups, for example, alkyl groups having up to 4 carbon atoms, for example, n-butyl groups, phenyl and benzyl groups, especially three n-butyl groups.

A silyl group R may also have up to 24 carbon atoms and three substituents, which may be the same or different, selected from alkyl, alkenyl, cycloalkyl, aryl and aralkyl groups, for example, alkyl groups having up to 4 carbon atoms, for example, methyl and t-butyl groups.

Any group R that is capable of substitution may be substituted, for example, with a halogen atom, especially a chlorine or bromine atom, or a nitro group.

The group R may be removable by hydrolysis, by photolysis, by reduction or by enzyme action to give the free acid, or two or more methods may be used, for example, reduction followed by hydrolysis. A group R that may be removed readily without substantial degradation of the rest of the molecule is particularly useful as a carboxyl protecting group. Examples of esters that are readily split by reduction are arylmethyl esters, for example, benzyl, p-nitrobenzyl, benzhydryl and trityl esters.

A stannyl ester, for example, a tri-n-butyl stannyl ester, may be split readily by hydrolysis, for example, by solvolysis, for example, using water, an alcohol, a phenol or a carboxylic acid, for example, acetic acid.

Certain ester groups may be split off by base hydrolysis, for example, acetylmethyl and acetoxymethyl ester groups.

There may be used an esterifying group that is removable under physiological conditions, that is to say, the esterifying group is split off *in vivo* to give the free acid or the carboxylate, for example, an acyloxymethyl ester, e.g. an acetoxymethyl or pivaloyloxymethyl ester, an aminoalkanyloxymethyl ester, for example, an L-glycyloxymethyl, L-valyloxymethyl or L-leucyloxymethyl ester, or a phthalidyl ester, or an optionally substituted 2-aminoethyl ester, for example, a 2-diethylamino-ethyl or 2-(1-morpholino)-ethyl ester.

Preferred esters are the p-nitrobenzyl, phthalidyl, pivaloyloxymethyl, acetylmethyl and acetoxy-methyl esters.

The present invention also provides the salts of those compounds of formula I that have salt-forming groups, especially the salts of free acids of formula I and the acid addition salts of compounds of formula I having a basic group. The salts are especially physiologically tolerable salts, for example, alkali metal and alkaline earth metal salts, for example, sodium, potassium, lithium, calcium and magnesium salts, ammonium salts and salts with an organic amine; also physiologically tolerable acid addition salts. These may be formed, with suitable inorganic and organic acids, for example, hydrochloric acid, sulphuric acid, organic carboxylic and organic sulphonic acids, for example, trifluoroacetic acid and p-toluenesulphonic acid. Some compounds of formula I which contain a basic centre may exist as Zwitterions; such salts are also part of this invention.

A salt of a free acid of formula I may be produced by reacting the free acid with the appropriate base in a solvent, preferably under conditions under which the salt precipitates. A preferred base is potassium ethylhexanoate.

A salt may be produced directly from an ester by splitting off the ester group under suitable reaction conditions, for example, catalytic reduction of an ester, for example, a p-nitrobenzyl ester, in an aqueous/organic solvent, for example, comprising water and ethyl acetate,

dioxane, or tetrahydrofuran, in the presence of a metal salt, especially a bicarbonate, for example, in an equivalent amount or in a slight excess, yields a salt directly.

The present invention also provides a process for the preparation of a compound of the general formula I or an ester or salt thereof, which comprises

reacting a compound of the general formula IIa a compound of the general formula IIb or a mixture thereof

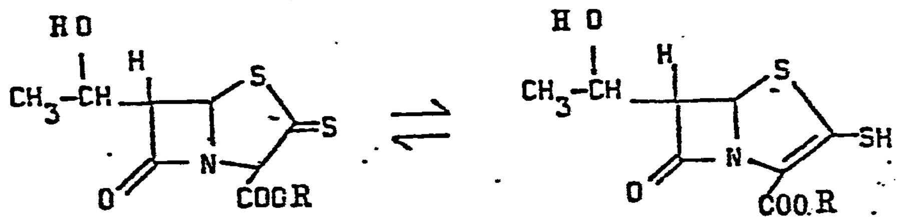

(IIa) (IIb)

in which R is as defined above, with a compound of formula III

$$L - R^1 - S - R^2 \qquad (III)$$

in which $R^1$, $R^2$, X, Y, m and n are as defined above, and L represents a leaving group that is capable of being replaced by a nucleophilic group, generally carrying out the reaction in the presence of a base;

and, if desired, carrying out any one or more of the following steps in any desired order on a resulting compound of formula I :

(i) converting an ester of formula I into the corresponding free acid,

(ii) converting a free acid of formula I into an ester thereof,

(iii) transesterifying an ester of a compound of formula I,

(iv) converting a free acid or an ester of formula I into a salt, or a salt into a free acid, an ester or another salt,

(v) removing any protective groups present other than an esterifying group ,

(vi) converting a substitutent of a group $R^2$ into another substitutent thereof.

In formula III , L represents a leaving group that is capable of being replaced by a nucleophilic group and is, for example, a halogen atom, for example, a chlorine, bromine or iodine atom, or a substituted, activated hydroxy group, for example, a sulphonyloxy group, for example, a radical of the formula $-OSO_2R^{20}$.

in which $R^{20}$ represents an aliphatic, cycloaliphatic, aryl or araliphatic group having up to 18 carbon atoms, which may be substituted or unsubst ted, for example, as described above for $R^2$. An aliphatic group $R^{20}$ is, for example, an alkyl group having up to 8 carbon atoms which may be substituted by one or more halogen atoms, for example, chlorine, fluorine and bromine atoms. An aryl group $R^{20}$ has, for example, up to 15 carbon atoms, and may be substituted by one or more substituents, which may be the same or different, selected from alkyl and alkoxy groups, for example, methyl and methoxy groups, nitro groups, and halogen atoms, especially bromine atoms, $R^{20}$ preferably represents an unsubstituted or substituted aryl group having up to 18 carbon atoms, for example, a phenyl, p-tolyl, p-bromophenyl or p-nitrophenyl group, or an unsubstituted or substituted alkyl group, especially with 1 to 4 carbon atoms, and preferably a methyl or trifluoromethyl group.

L preferably represents a bromine or iodine atom or a methylsulphonate, trifluoromethylsulphonate, tolylsulphonate or benzenesulphonate group.

The reaction of a compound of formula IIa and/or a compound of formula IIb with a compound of formula III is generally carried out in a solvent or diluent that is inert under the reaction conditions, for example, an oxygenated hydrocarbon, for example, tetrahydrofuran, dioxane or ethyl acetate; a chlorinated hydrocarbon, for example, methylene chloride or chloroform; or a polar organic solvent, for example, N,N-dimethylacetamide, N,N-dimethylformamide; or water. A mixture of two or more solvents may be used, for example, a mixture of water and another solvent (whether miscible with water or not).

The reaction between a compound of formula II and a compound of formula III is generally carried out in the presence of a base, preferably a non-nucleophilic base, for example, an organic or inorganic base, for example, an alkali metal carbonate or bicarbonate, for example, sodium or potassium carbonate or bicarbonate; an alkali metal hydride, for example, sodium hydride; an alkali metal alkoxide, for example, sodium methoxide; or a tertiary amine, for example, triethylamine, ethyldiisopropylamine, DABCO (diazabicyclo[2,2,2]octane), pyridine or an alkyl-, dialkyl- or amino-substituted pyridine, for example, N,N-dimethylamniopyridine or collidine.

The reaction is generally carried out at a temperature in the range of form -80 to +30°C, preferably from -40 to +30°C.

A compound of the general formula II is described and claimed claimed in our UK Specification No. 2 087 880A, as is a process for its production.

In a compound of the general formula I, the stereochemistry can be R or S independetly at positions 5,6 and 8 (R and S being as defined by the Cahn-Ingold)Prelog system of

nomenclature). The preferred stereochemistry at position 5 is R, at position 6 is S, and at position 8 is R. 5R-Stereochemistry is that found in naturally occurring penicillin and is, in general, preferable to 5S-stereochemistry, more 5R compounds being antibiotically active than are 5S compounds. In order to produce predominantly the desired 5R compound of formula I, it is particularly advantageous to use the 5R isomer of the compound of formula II as the starting material for the process of the invention.

The 5R-isomer of a compound of the general formula II and a process for its production are described and claimed in our UK Application No. 83.08276, as follows:

A compound of the general formula IIa' or its tautomer of formula IIb', or a mixture thereof

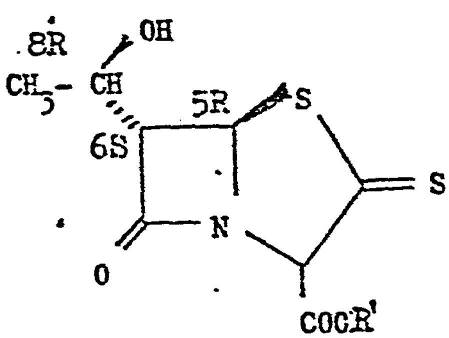

IIa'

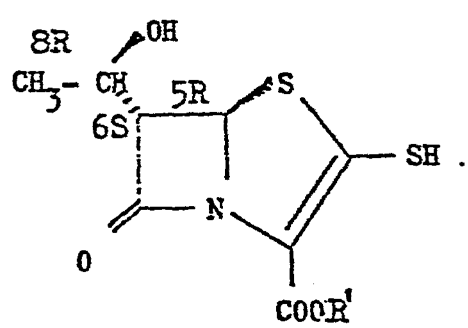

IIb'

in which formulae R' represents a carboxyl esterifying group, for example, a carboxyl esterifying group that may be removed by hydrolysis, photolysis, reduction or enzyme action to give the free acid.

The term "a compound of the general formula $II'$" and "a compound of formula $II'$" are both used herein to denote a compound of the general formula $IIa'$, a compound of the general formula $IIb'$, or any mixture thereof. "A compound of formula III" is used to denote collectively compounds of formulae IIIa, IIIb and IIIc. The terms "a compound of formula IV" and "a compound of formula V" are used analogously.

The present invention also provides a process for the production of a compound of the general formula II, which comprises treating a compound of the general formula IIIa, IIIb, or IIIc

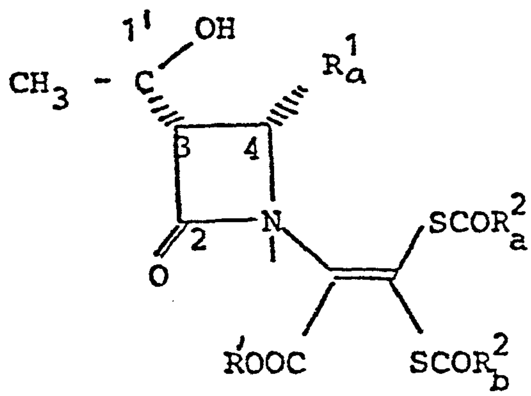

(IIIa)

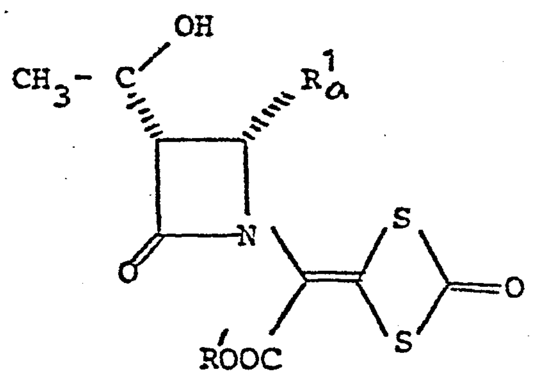

(IIIb)

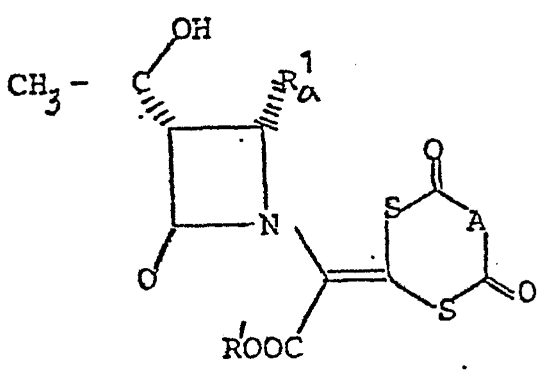

(IIIc)

in which $R'$ is as defined above,
$R_\alpha^1$ represents a chlorine or bromine atom, the radicals $R_a^2$ and $R_b^2$, which may be the same or different, each represents an alkyl group having from 1 to 4 carbon atoms,

especially a methyl or t-butyl group, an aryl group, especially a phenyl group, or an aralkyl group, especially a benzyl group, and A represents a direct bond or the residue of a dicarboxylic acid, with a base.

The base used in the above reaction must be capable of splitting a sulphur-carbonyl bond in the compound of formula III and of bringing about ring closure. The base may be inorganic or organic, for example, ammonia, or an alkali metal (especially a sodium or potassium) carbonate, bicarbonate, or hydroxide; a primary amine, for example, methylamine, ethylamine, aniline or benzylamine; an alkali metal alkoxide, for example, sodium methoxide; or a heterocyclic base, for example, having a $pK_a$ within the range of from 5 to 9, for example, imidazole or pyridine or a substituted pyridine, for example, an alkyl-, amino-, or alkylamino-substituted pyridine, for example, 4-methyl- or 4-dimethylamino-pyridine. Imidazole is particularly preferred.

The reaction is generally carried out in a solvent or diluent, the choice of which is wide, provided that it is inert under the reaction conditions. Examples of solvents and diluents are oxygenated hydrocarbons, for example, alcohols, for example, having up to 4 carbon atoms, for example, methanol and ethanol; ethers, for example, having up to 4 carbon atoms, for example, diethyl ether, also tetrahydrofuran and dioxane; ketones, for example, having up to 4 carbon atoms, for example acetone and methyl ethyl ketone; esters,for example, methyl acetate and ethyl acetate; and amides, for example, dimethylformamide and dimethylacetamide; also chlorinated hydrocarbons, for example, chloroform, methylene chloride and carbon tetrachloride; aromatic hydrocarbons, for example, benzene and toluene; and other solvents, for example, acetonitrile and nitromethane. A mixture of any two or more solvents may be used, and solvents are preferably used

in admixture with water, preferably a water-miscible solvent in admixture with 5 to 20 % (v/v) water, especially a mixture of dioxane and water, preferably 5 to 10 % (v/v) water.

The reaction is generally carried out at a temperature within the range of from 0 to 40°C, preferably from 0 to 20°C .

An esterified carboxyl group -COOR' is, for example, an ester formed with an unsubstituted or substituted aliphatic, cycloaliphatic, cycloaliphatic-aliphatic, aryl, araliphatic, heterocyclic or heterocyclic-aliphatic alcohol having up to 20 carbon atoms, or is, for example, s silyl or stannyl ester.

R' may represent, for example a straight or branched chain substituted or unsubstituted alkyl, alkenyl or alkynyl group having up to 18 carbon atoms, preferably up to 8 carbon atoms, and especially up to 4 carbon atoms, for example, a methyl, ethyl, n-propyl, iso-propyl, n-butyl, sec-butyl, iso-butyl, tert-butyl, n-pentyl, n-hexyl, allyl, or vinyl group.

An aliphatic group R', especially a methyl group, may be substituted by a cycloalkyl, aryl or heterocyclic group, or R' may itself represent a cycloalkyl, aryl or heterocyclic group.

A cycloaliphatic group R' may have up to 18 carbon atoms and is, for example, a cyclopentyl, cyclohexyl or adamantyl group. An aryl group may have up to 12 carbon atoms and may have two or more fused rings. An aryl group R' is, for example, an unsubstituted or substituted phenyl group, and an unsubstituted or substituted aralkyl group is, for example, a benzyl, p-nitrobenzyl or benzhydryl group.

A heterocyclic group may have one or more heteroatoms, selected from oxygen, nitrogen and sulphur, and up to 14 atoms in total. A heterocyclic group is, for example, an oxygen-containing heterocyclic group, for example, a tetrahydropyranyl or phthalidyl group.

A stannyl group $R'$ may have up to 24 carbon atoms, for example, $R'$ may represent a stannyl group having three substituents, which may be the same or different, selected from alkyl, alkenyl, cycloalkyl, aryl, aralkyl, alkoxy and aralkoxy groups, for example, alkyl groups having up to 4 carbon atoms, for example, n-butyl groups, phenyl and benzyl groups, especially three n- butyl groups.

A silyl group $R'$ may also have up to 24 carbon atoms and three substituents, which may be the same or different, selected from alkyl, alkenyl, cycloalkyl, aryl and aralkyl groups, for example alkyl groups having up to 4 carbon atoms, for example, methyl and t-butyl groups.

Any group $R'$ that is capable of substitution may be substituted, for example, with a halogen atom, especially a chlorine or bromine atom, or a nitro group.

The group $R'$ may be removable by hydrolysis, by photolysis, by reduction or by enzyme action to give the free acid, or two or more methods may be used, for example, reduction followed by hydrolysis. A group R that may be removed readily without substantial degradation of the rest of the molecule is particularly useful as a carboxyl protecting group. Examples of esters that are readily split by reduction are arylmethyl esters, for example, benzyl, p-nitrobenzyl, benzhydryl and trityl esters.

A stannyl ester, for example, a tri-n-butyl stannyl ester, may be split readily by hydrolysis, for example, by solvolysis, for example, using water, an alcohol, a phenol or a carboxylic acid, for example, acetic acid.

Certain ester groups may be split off by base hydrolysis, for example, acetylmethyl and acetoxymethyl ester groups.

There may be used an esterifying group that is removable under physiological conditions, that is to say, the esterifying group is split off in vivo to give the free acid or the carboxylate, for example, an acyloxymethyl ester, e.g. an acetoxymethyl or pivaloyloxymethyl

ester, an aminoalkanyloxymethyl ester, for example, an L-glycyloxymethyl, L-valyloxymethyl or L-leucyloxymethyl ester, or a phthalidyl ester, or an optionally substituted 2-aminoethyl ester, for example, a 2-diethylamino-ethyl or 2-(1-morpholino)-ethyl ester.

Preferred esters are the p-nitrobenzyl, phthalidyl, pivaloyloxymethyl, acetylmethyl and acetoxy-methyl esters.

A compound of the general formula IIIa, IIIb, or IIIc is preferably produced by halogenating a compound of the general formula IVa, IVb or IVc, respectively,

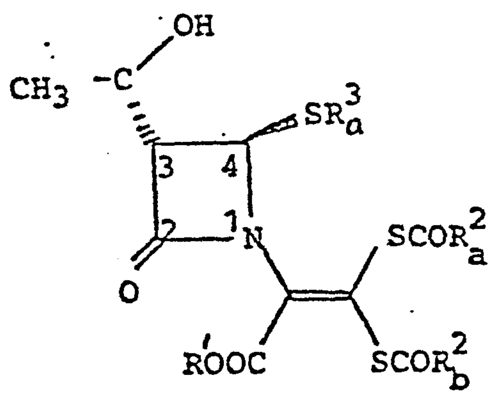

(IVa)

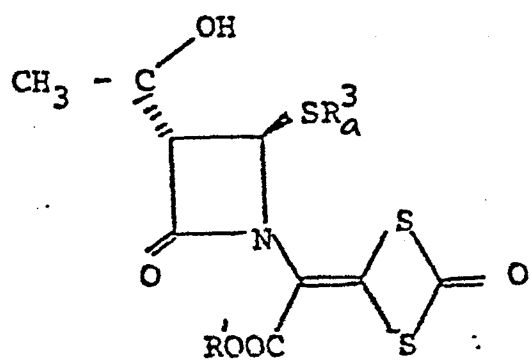

(IVb)

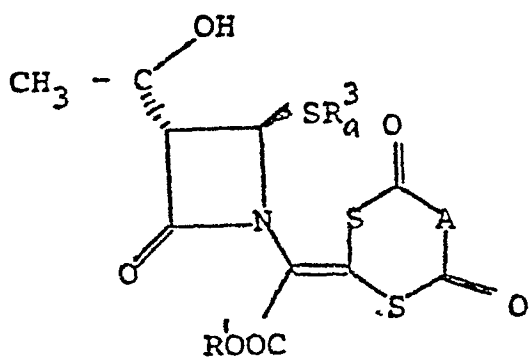

(IVc)

in which $R^1$, $R_a^2$, $R_b^2$ and A are defined as above, and $R_a^3$ represents an alkyl group having from 1 to 8, preferably from 1 to 6, and especially from 1 to 4 carbon atoms, for example, an ethyl group, or an alkenyl group having up to 6 carbon atoms, especially an allyl group.

The halogenation of a compound of formula IVa, IVb or IVc is carried out with an agent capable of splitting a carbon-sulphur bond and introducing a halogen atom. Such agents are well known in the art and include, for example, molecular chlorine, molecular bromine, sulphuryl chloride, sulphuryl bromide, t-butyl hypochlorite and cyanogen chloride.

The halogenating agent is generally used in an amount of from 1 to 2 mole equivalents, calculated on the compound of formula IV. The reaction is generally carried out at a temperature within the range of from -40 to +20°C. The reaction is generally carried out in a solvent or diluent that is aprotic and is inert under the reaction conditions, for example, an ether, a hydrocarbon or a halogenated hydrocarbon, for example, dioxane, benzene, chloroform or methylene chloride. A mixture of two or more solvents may be used. Examples of halogenating systems are: chlorine in chloroform and, especially, chlorine in benzene and t-butyl hypochlorite in benzene. In the latter two cases, the temperature is preferably from 5 to 20°C, and especially from 5 to 10°C.

A compound of formula IVa, IVb, or IVc is preferably produced by removing the protective group from a compound of formula Va, Vb or Vc, respectively,

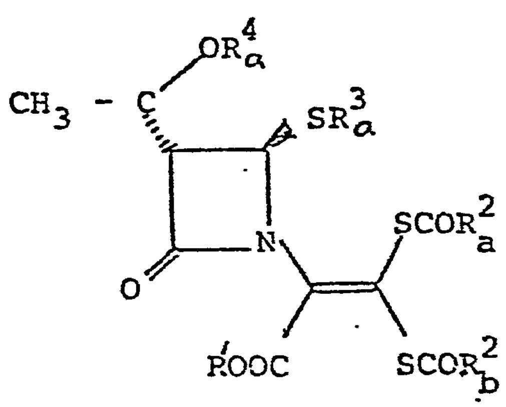

( Va)

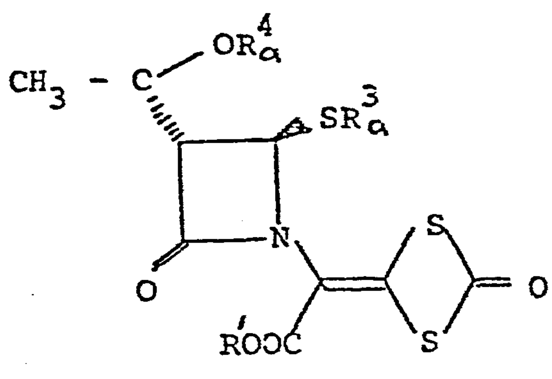

( Vb)

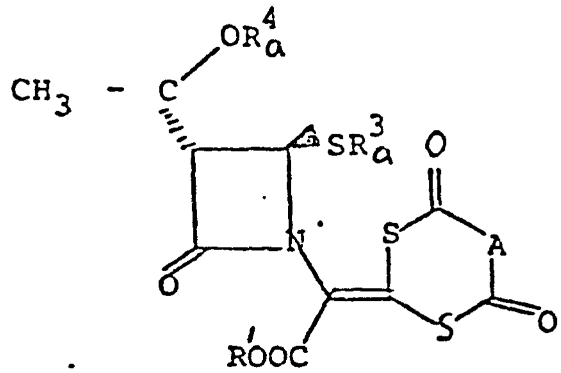

( Vc)

in which R′, $R_a^2$, $R_b^2$, $R_\alpha^3$ and A are defined as above, and $R_\alpha^4$ denotes a hydroxy protecting group.

Preferred groups $R_\alpha^4$ are those which are compatible with the synthesis of the compound of formula V and which may be removed under reaction conditions in which the resulting compound IV is stable. Compound IV has been found to be stable in the presence of a proton source, for example, hydrogen chloride, aqueous hydrochloric acid or aqueous hydrofluoric acid. Accordingly, one type of preferred hydroxy protecting groups $R_\alpha^4$ are those which may be removed under acidic conditions. Such groups are well known in the art and are for example, tetrahydropyranyl and tetrahydrofuranyl groups; acetal and ketal groups, for example, of formula

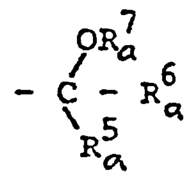

in which $R_\alpha^6$ and $R_\alpha^7$, which may be the same or different, each represents a hydrogen atom or a lower alkyl group, preferably a methyl group, or $R_\alpha^6$ and $R_\alpha^7$ together with the carbon atom to which they are attached represent a ring having from 4 to 7 carbon atoms, for example, a tetrahydropyranyl or tetrahydrofuranyl ring; and $R_\alpha^5$ represents a lower alkyl group, preferably a methyl or ethyl group.

$R_a^4$ may also represent a silyl group, for example, as described above in relation to R, for example, $-SiR_a^8R_a^9R_a^{10}$ groups, in which $R_a^8$, $R_a^9$ and $R_a^{10}$, which may be the same or different, each represents a lower alkyl group or an aryl group, for example, triethylsilyl, t-butyldimethylsilyl and methyldiphenylsilyl groups; and stannyl groups, for example, as described above in relation to R, for example, $SnR_a^{11}R_a^{12}R_a^{13}$ groups, in which $R_a^{11}$, $R_a^{12}$ and $R_a^{13}$, which may be the same or different, each represents a lower alkyl group, for example, a tri-n-butylstannyl group. Preferred $R_a^4$ groups are tetrahydropyranyl, 2-methoxyprop-2yl and t-butyldimethylsilyl groups.

A t-butyldimethylsilyl group may be removed in a known manner by acid hydrolysis, for example, using moderately concentrated hydrochloric acid, for example 6M HCl, e.g., in tetrahydrofuran (cf Belgian Patent Specification No. 881 012),or hydrogen chloride in tetrahydrofuran, dimethylformamide, dioxane, a lower alkanol, or acetonitrile; tetra(n-butyl)ammonium fluoride in an acidic medium, e.g., in acetic acid (cf Belgian Patent Specification No. 882 764); or aqueous hydrogen fluoride e.g., in the presence of acetonitrile (cf J. Chem. Soc. Perkin 1, 1981, 2055). (The term 'known' is used herein to mean in actual use in the art or described in the literature of the art).

A compound of the general formula V may be prepared according to the following reaction scheme:

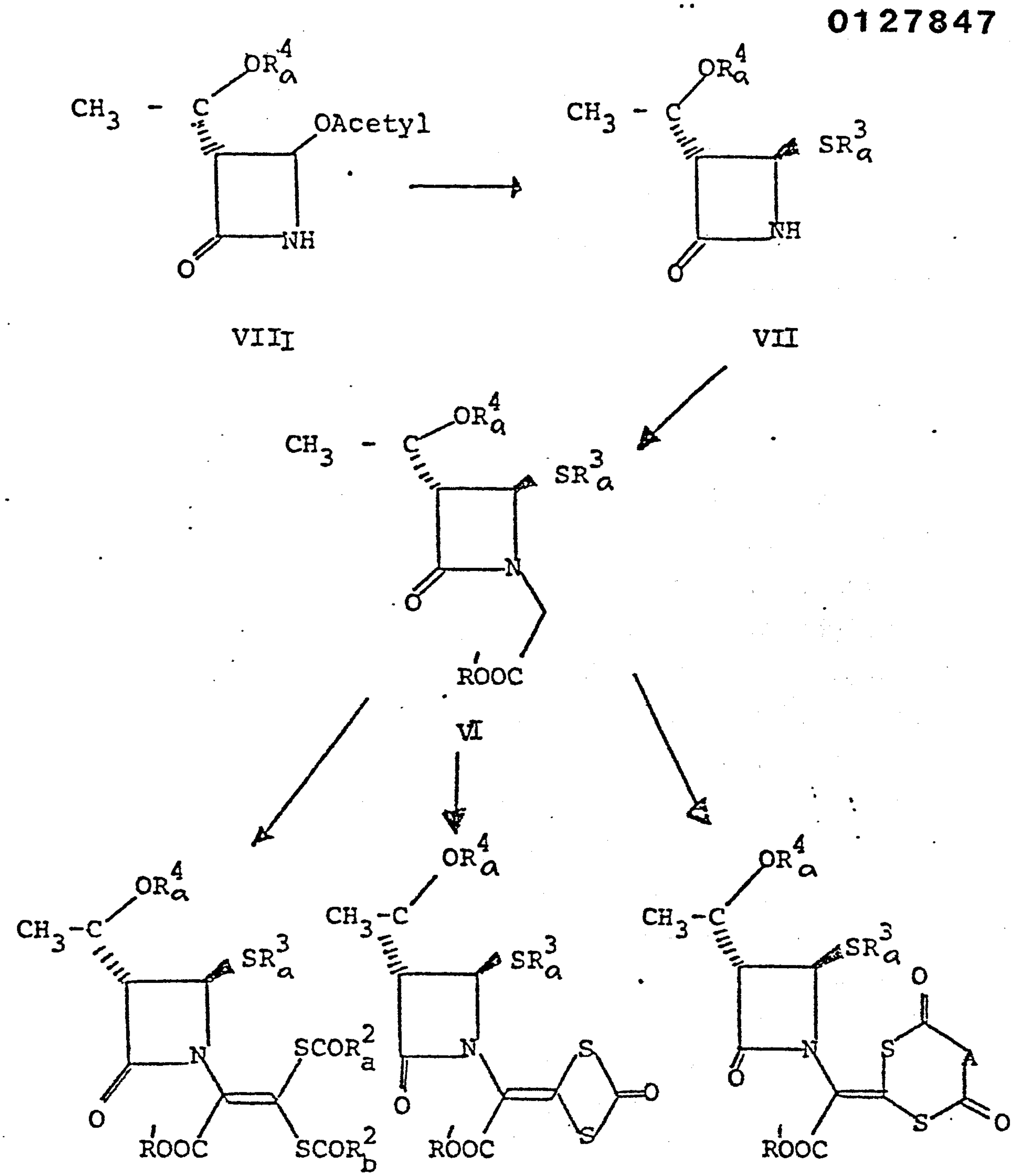

VIII

VII

VI

Va

Vb

Vc

in which $R'$, $R^2_a$, $R^2_b$, $R^3_\alpha$, $R^4_\alpha$ and A are as defined above.

A compound of formula VIII may be prepared as described in Belgian Patent Specification No. 882 764.

A compound of formula VIII may be converted into a compound of formula VII by reaction with a compound of formula IX

$$R^3_\alpha - S - R^{14}_\alpha \qquad (IX)$$

in which $R^3_\alpha$ is as defined above and $R^{14}_\alpha$ represents a hydrogen atom or an alkali metal atom, especially a sodium or potassium atom. $R^3_\alpha$ preferably represents a straight chain lower alkyl group, especially an ethyl group, or a straight chain lower alkenyl group, especially an allyl group.

The reaction is generally carried out in a solvent, preferably a protic solvent, for example, water or an alcohol, or an aprotic, water-miscible solvent which is preferably polar, for example, dimethylformamide, dimethyl sulphoxide, tetrahydrofuran or dioxan. The reaction temperature is, for example, from -20 to +50, preferably from -10 to +20$^o$C.

To obtain a compound of formula VIa compound of formula VII may be reacted, in the presence of a base, with a compound of formula X

$$Y^1CH_2CO_2R' \qquad (X)$$

in which $R'$ is as defined above and

$Y^1$ represents a group that is capable of being replaced by a nucleophilic group and is, for example, a halogen atom, preferably a bromine or iodine atom, or a modified hydroxy group, preferably a sulphonyloxy group of the formula $SO_3R^{16}_\alpha$ in which $R^{16}_\alpha$ represents a lower alkyl or $-CF_3$ group, or a phenyl group which is unsubstituted or is substituted by a p-nitro, p-bromo or p-methyl group.

$Y^1$ preferably represents a bromine or iodine atom or a methylsulphonate, trifluoromethylsulphonate, tolylsulphonate or benzenesulphonate group.

The base may be inorganic, organic or organometallic, for example, an alkali metal or alkaline earth metal hydroxide, oxide, carbonate, bicarbonate or hydride, for example, sodium hydroxide, magnesium oxide, potassium carbonate, potassium bicarbonate or sodium hydride; a tertiary amine, for example, a trialkylamine, for example, triethylamine, DABCO (diazabicyclo[2,2,2]octane), pyridine, or an alkyl-substituted or amino-substituted or dialkylamino-substituted pyridine, for example, N,N-dimethylaminopyridine, or collidine; a guanidine, for example, tetramethylguanidine; DBN (diazabicyclo[4,3,0]non-5-ene) or DBU (diazabicyclo[5,4,0]undec-7-ene), a polymeric base i.e., a base attached to an inert polymeric support e.g., Hunig's base (diisopropylethylamine attached to e.g., polystyrene); a metallated amine, for example, a metallated alkyl- or arylamine, for example, lithium diisopropylamide (LDA), lithium hexamethyldisilazide, lithium piperidide, lithium 2,2,6,6-tetramethylpiperidide, or a Grignard reagent, for example, methylmagnesium bromide. Preferred bases are, for example, potassium carbonate, sodium hydride, lithium diisopropylamide and triethylamine.

The reaction is generally carried out in an aprotic solvent or diluent, for example, a tertiary amide, for example, dimethylformamide, dimethylacetamide or hexamethylphosphoramide; a hydrocarbon, for example, benzene or toluene; or an ether, for example, diethyl ether, tetrahydrofuran or dioxane; or acetonitrile, dimethyl sulphoxide, or sulpholane. Dimethylformamide and dimethylacetamide are preferred. A mixture of two or more solvents and/or diluents may be used.

The reaction may be carried out at a temperature generally within the range of from -80°C to +30°C preferably from -40 to +30°C, and especially from -20 to +20°C.

From 1 to 1.5 moles of compound X are preferably used per mole of compound VII especially from 1 to 1.1 moles of X per mole of VII. The base is used in an amount, for example, from 1 to 4 moles of base per mole of compound VII.

The reaction is preferably carried out by dissolving compound VII in a solvent, advantageously in dimethylformamide with stirring, adding the base, adding the compound of formula X and reacting at the desired temperature. The resulting compound of formula VI may be worked up and isolated in the usual manner, for example, using chromatographic and/or crystallisation techniques, or the subsequent reaction may be carried out directly on the resulting reaction mixture after removal of any solvent that is not compatible with the subsequent reaction.

If R' in formula VI represents a carboxyl esterifying group, this group may be converted into another esterifying group R', for example, to introduce a group R that is more easily removable under desired conditions. This transesterification is generally carried out as follows: the ester for formula VI is hydrolysed in a known manner using, for example, acid or alkaline hydrolysis, preferably using an alkali metal hydroxide, especially sodium or potassium hydroxide. The ester of formula VI, for example, a methyl ester, is preferably hydrolysed using an alkali metal hydroxide, especially one mole thereof per mole of the ester of formula VI in a solvent, for example ethanol, methanol or water, or an aqueous-organic solvent, for example, tetrahydrofuran/water, ethanol/water, or acetonitrile/water.

The reaction mixture may then be acidified to give a solution of pH 1 to 5, preferably 2 to 4, and the free acid may then be isolated and, if desired, the free acid is then esterified with an esterifying agent capable of introducing a different esterifying group R', for example with an alcohol R'OH in the presence of an acid or another

activating agent, for example, dicyclohexylcarbodiimide, or with an alkylating agent $R'Y^1$ in which $Y^1$ is as defined above. Preferably a salt may be isolated and esterified directly.

A compound of formula VI may be converted into a compound of formula V by treatment with a base in the presence of carbon disulphide followed by reaction with an acylating agent, or by treatment with a base, then with carbon disulphide, and finally reaction with an acylating agent. An acylating agent is generally an activated carboxylic acid.

The activated carboxylic acid may be any activated acid derivative comprising the group $R^2_{a/b}$. Such derivatives are well known in the art, and include acid halides, acid anhydrides, and activated esters. An anhydride may be symmetrical or asymmetrical.

For the introduction of a group $-\!\!<\begin{matrix} SCOR^2_a \\ SCOR^2_b \end{matrix}$ to give a compound of formula IVa, the acylating agent preferably has one of the formulae XIa to XIe

$$R^2_aCOZ \qquad \text{(XIa)} \qquad\qquad R^2_bCOZ \qquad \text{(XIb)}$$

$$R^2_a - \overset{O}{\overset{\|}{C}} - O - \overset{O}{\overset{\|}{C}} - R^2_a \qquad\qquad R^2_a - \overset{O}{\overset{\|}{C}} - O - \overset{O}{\overset{\|}{C}} - R^2_b$$

(XIc) (XId)

$$R^2_b - \overset{O}{\overset{\|}{C}} - O - \overset{O}{\overset{\|}{C}} - R^2_b$$

(XIe)

in which $R^2_a$ and $R^2_b$ are as defined above, and Z represents a halogen atom, especially a chlorine or bromine atom or represents an activated ester or amide, or a radical derived from an acid azide. Such coupling reagents are well known in the art of peptide chemistry.

In the case of formula Vb, the group

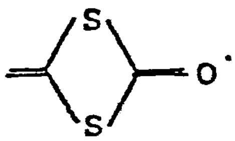

may be introduced by means of an acylating agent of formula XII

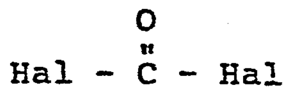

in which Hal represents a halogen atom, especially a chlorine atom.

For the introduction of a group 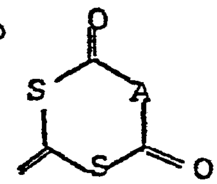 to give a to compound of formula Vc, a dicarboxylic acid derivative of formula XIII is used

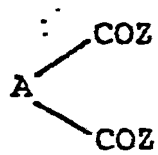

in which A and Z are as defined above, and Z preferably represents a halogen atom, especially a chlorine atom. As mentioned above, A represents the residue of a dicarboxylic acid or represents a direct bond. A is derived, for example, from malonic, dimethylmalonic, succinic, glutaric, adipic, pimelic or phthalic acid.

The compound of formula VI is preferably reacted first with a base, then with carbon disulphide, and then finally with the acylating agent.

The base preferably has a pK$\geq$ 20, and is preferably a metallated amine. Examples of preferred bases are lithium diisopropylamide, lithium 2,2,6,6-tetramethyl-piperidide, lithium cyclohexyl isopropylamide, lithium hexamethyl disilazide, and sodamide.

The reaction is generally carried out in an inert solvent, for example, an oxygenated hydrocarbon, preferably an ether, for example, diethyl ether, tetrahydrofuran,

dioxane, glyme or diglyme. The reaction temperature is, for example, from -120 to +30°C, preferably from -100 to -20°C.

The amount of base used is for example, from 1 to 4 moles, calculated per mole of compound VI, preferably from 2.0 to 3.0 moles of base. Carbon disulphide is preferably used in amount of from 1 to 5 moles, especially from 2 to 3 moles, per mole of compound VI.

The reaction is preferably carried out as follows: to a stirred solution of compound VI under an inert atmosphere is added the base then carbon disulphide, if desired in solution in the same solvent as compound VI or in a different solvent, and finally the acylating agent to complete the reaction.

There may then be admixed a protic source having a pK less than 10, and especially from 5 to 2, for example, acetic, citric, oxalic or formic acid.

The compound of the general formula II' has R stereochemistry at position 5. This is the stereochemistry found in naturally occurring penicillins and is, in general, preferable to 5S stereochemistry, more 5R compounds being antibiotically active than are 5S compounds.

We have found a process that gives predominantly the desired 5R compound of formula II. It has been proposed previously (British Patent Application 2074563A) to halogenate a compound of formula V i.e., a compound having a protected hydroxy group in the side chain attached to the 3-position, but we have found that this process gives only a 4R halogenated compound, which in its turn, gives a compound analogous to that of formula II but having the undesired 5S stereochemistry. We have found that, very surprisingly, if the protective group is removed from compound V prior to halogenation, the resulting halogenated compound of formula III is predominantly 4S. The isomer ratio 4S:4R in compound III resulting from the halogenation varies according to the reaction conditions but is, for

example, in the range of from 3:1 to as high as 9:1. Moreover, the 4R and 4S isomers of formula III can be separated easily, for example, by chromatography.

The 4S halogenated intermediates of formula III give virtually exclusively a compound of formula II with the 5R stereochemistry as shown. The presumed participation of the free hydroxyl group of the side chain of formula III in giving the more sterically hindered compound of formula II is also unexpected and constitutes a valuable advance in the preparation of the penem compounds of formula II.

The esterifying group R' may be removed from a compound of formula II' before reaction with a compound of formula III to give a compound of formula I, but it is preferable to protect the carboxyl group in a compound of formula II. After synthesis of a compound of formula I, an esterifying group may be removed to give the free acid, which may then be converted into a salt, if desired, or the ester may be converted directly into a salt, for example, as described above.

Methods for reomoving and intoducing ester groups, and for producing salts and free acids are well known.

An ester of a compound may be transesterified, or a free acid of formula I may be esterified, to give an ester having particular, desired properties, for example, with regard to ease of removal under physiological conditions.

The invention also provides a modification of the process of the invention, wherein in a compound of formula I, a substitutent of a group $R^2$ is converted into another substitutent of $R^2$.

The following are examples of interconversions of substituents of $R^2$: ($R^4$ being as defined above).

$R^4S-$ to $R^4SO-$

$R^4S-$ or $R^4SO-$ to $R^4SO_2$

$NO_2-$ to $NH_2-$, which may then be alkylated or acylated,

$-CN$ to $-CH_2NH_2$, which may then be alkylated or acylated,

$N_3$ to $NH_2-$, which may then be alkylated or acylated,

HO- may be alkylated or acylated

$R^4CO-O-$ to HO-, which may then be alkylated or acylated

Halogen to -SH, $-SR^4$, $-SO_2H$, $-SO_3H$. -CN, $CO_2H$ or $N_3$ which may be further treated as described above for the appropriate group.

The methods for carrying out such reactions are known in the art, for example, an alkylthio group may be oxidised, preferably with a carboxylic peracid, especially m-chloroperbenzoic acid, to give the corresponding alkylsulphinyl or alkylsulphonyl group; a nitro group may be reduced to an amino group by noble metal catalysed hydrogenation, for example, using platinum, or 10% palladium on carbon, c.f. M. Freifelder, Catalytic Hydrogenation in Organic Synthesis, Willey Interscience, 1978, page 26, and P.N. Rylander, Catalytic Hydrogenation over Platinum Metals, Academic Press, 1967 Chapter 11; an amino group may be alkylated with a conventional alkylating agent, for example, a lower alkyl halide, for example, methyl iodide, or acylated with, for example, an acid chloride or acid anhydride, for example, acetyl chloride or acetic anhydride,

 

a cyano group may be converted into an amino group by reduction, for example, using a metal hydride; an azide group may be converted into an amino group by reduction, for example, using hydrogen sulphide or catalytic reduction; and a hydroxy group may be alkylated or acylated as described above; and a halide, especially an iodide, may be displaced by a nucleophile, for example, CN-, $R^4S-$, $R^4SO_2-$ or $N_3$. In each case, the resulting group may be treated further, if appropriate.

These modifications of the process of the invention are particularly useful for the production of a compound of formula I having a group $R^2$ bearing 1, 2 or 3 substituents, any one or more of which is potentially unstable or incompatible during any one or more of the stages of the reaction sequence described above. The conversion step is, accordingly, carried out after the step in which the substituent is potentially unstable or incompatible.

It will be appreciated that although these modifications are particularly useful for the production of compounds of formula I having substituents on $R^2$ that are potentially unstable in the production process, it is not limited to such groups, and in a further modification of the process of the invention, a substituent of $R^2$ may be produced by conversion of another substituent that does not itself fall within the definition of a substituent of $R^2$, for example, an unsubstituted or substituted, preferably p-nitrosubstituted, benzyloxycarbonylamino group may be converted into a free amino group, for example, by noble metal catalysed hydrogenation, c.f. M. Freifelder, loc. sit., page 111, P.N. Rylander, loc. cit., page 455, and C. Berse et al, J. Org. Chem. 22, 805, 1957.

All of the compounds that are provided by the invention may exist in any isomeric form, as discussed above, either as a pure isomer or as a mixture of any two or more isomers.

A compound of formula I may have the R- or S-stereo-chemistry independently at positions 5, 6 and 8. Further isomeric forms will occur when any substituent contains a chiral carbon atom. Any mixture of two or more isomeric forms may be resolved if desired, or a compound of formula I can be used in the form of the isomeric mixture. R stereochemistry is generally preferred at position 5 in compound I, (as in naturally occurring penicillins and cephalosporins). At position 6, S stereochemistry is generally preferred, and at position 8 R is generally preferred. It appears that the stereochemistry at position 5 is the most important.

The compounds of formula I and salts thereof possess antibacterial properties and have enhanced bioavailability. They may be used in humans and other animals, for example, to treat bacterial infections caused by gram-positive and gram-negative bacteria, for example, Staphylococcus aureus, Streptacoccus py ogenes, Bacillus subtilis, E. coli, Pseudomonas aeruginosa, and Proteus morganii, some strains of which are penicillin-resistant. The compounds of formula I are also β-lactamase inhibitors, and the compounds are generally stable to the action of β-lactamases produced by gram-positive organisms, for example, by Staphylococcus aureus and gram negative organisms, for example, Enterobactercloacae.

The invention accordingly provides a pharmaceutical preparation which comprises a compound of formula I, or a physiologically

tolerable salt thereof, or a mixture of two or more such substances as active ingredient, in admixture or conjunction with a pharmaceutically suitable carrier. The preparation may also comprise one or more other pharmaceutically active substances, for example, another antibacterial substance, especially one which has a β-lactam ring. The preparations may be in a form suitable for enteral or parenteral administration, for example, for oral, intravenous, or intra-muscular administration, for example, as tablets, capsules, syrups, or sterile injectable or infusible solutions. The preparations are advantageously in unit dosage form and preferably comprise from 10 to 2000 mg of the active ingredient. The daily dosage of the active ingredient is generally from 20 to 8000 mg, particularly from 50 - 5000mg, preferably from 50 - 500 mg, in divided doses, generally up to 4 doses, relating to a body weight of 75 kg.

The invention also provides the use of an active ingredient as defined above as a β-lactamase inhibitor and/or as an antibacterial agent.

The invention further provides a pharmaceutical preparation which comprises an active ingredient as defined above, in unit dosage form.

The invention also provides a pharmaceutical preparation which comprises an active ingredient as defined above, or a physiologically tolerable salt thereof or a mixture of two or more such substances, and one or more further pharmaceutically active substances, for example, as described above and, for example, in unit dosage form.

Unit dosages are preferably as described above.

The following Examples 1 to 90 illustrate the invention. Preparations 1 to 2.41 illustrate the preparation of starting materials for the Examples.

Preparation 1: 1-Methyl-5-chloromethyl-thio-1,2,3,4-tetrazole

To a solution of 1.93 g of 1-methyl-5-mercaptotetrazole sodium salt in 5 ml of DMF at $0^{o}$ was added 3.74 g of chloroiodomethane. The reaction mixture was permitted to warm to room temperature, stirred for 16 hours and poured into 50 ml of ethyl acetate and 50 ml of water. The organic layer was separated, washed with water, dried over magnesium sulphate and the solvent evaporated to give 1.36 g of the title compound as a cream crystalline solid.

$\delta(CDCl_3)$ 4.01 (3H,s)
5.28 (2H,s)

Preparation 2: 1-Methyl-5-iodomethyl-thio-tetrazole

To a solution of 2.41 g of 1-methyl-5-chloro-methyl-thio-1,2,3,4-tetrazole in 30 ml of acetone was added 4.38 g of sodium iodide and the mixture heated at reflux for 16 hours. The reaction mixture was poured into 50 ml of ethyl acetate, washed with water, dried over magnesium sulphate and the solvent evaporated. Chromatography over silica gel using ethyl acetate/hexane mixtures as eluant gave 2.11 g of the title compound in purified form.

($CDCl_3$) 3.98 (3H,s)
4.77 (2H,s)

According to the methodology described in Preparations 1 and 2 the following compounds were prepared.

1.1

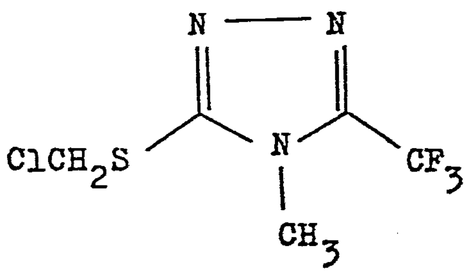

$\delta$ ($CDCl_3$) 2.25 (3H,s)
3.13 (2H,s)

2.1

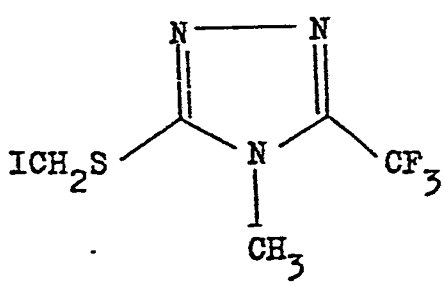

$\delta$ ($CDCl_3$) 2.21 (3H,s)
2.78 (2H,s)

1.2

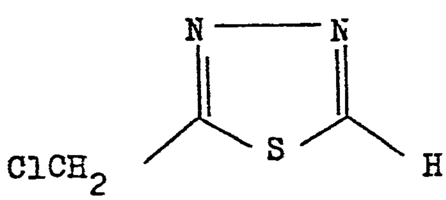

$\delta$ ($CDCl_3$) 5.30 (2H,s)
9.17 (1H,s)

2.2

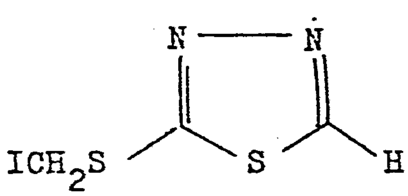

$\delta$ ($CDCl_3$) 4.85 (2H,s)
9.19 (1H,s)

1.3

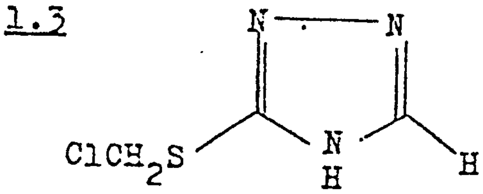

$\delta$ ($CD_3CN$) 5.25 (2H,s)
8.15 (1H,s)
8.40 (1H,brs)

2.3

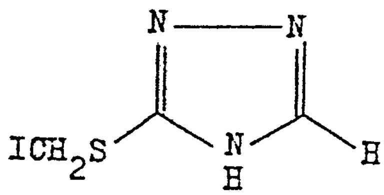

$\delta$ ($CD_3CN$) 4.80 (2H,s)
6.26 (1H,s)
8.24 (1H,brs)

1.4

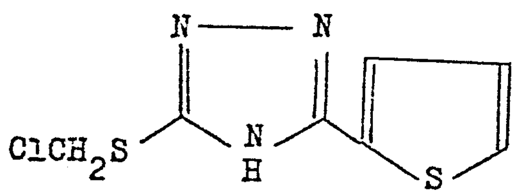

$\delta$ ($CDCl_3$) 5.23 (2H,s)
6.81-7.59 (3H,m)

2.4

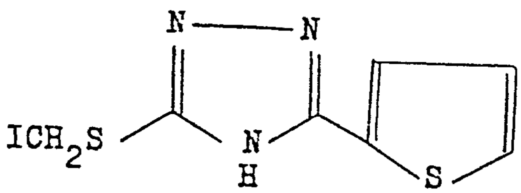

$\delta$ ($CD_3CN$) 4.71 (2H,s)
6.90-7.53 (3H,m)

1.5

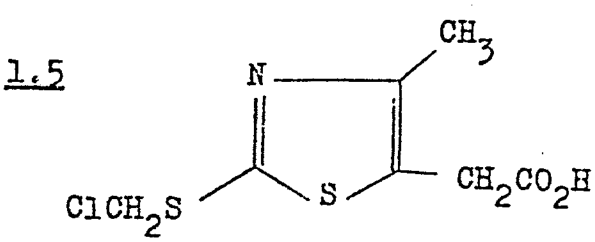

$\delta$ ($CD_3OH$) 2.38 (3H,s)
3.82 (2H,s)
5.29 (2H,s)

2.5

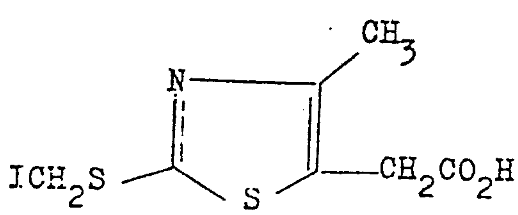

$\delta$ ($CD_3OH$) 2.39 (3H,s)
3.86 (2H,s)
4.85 (2H,s)

1.6

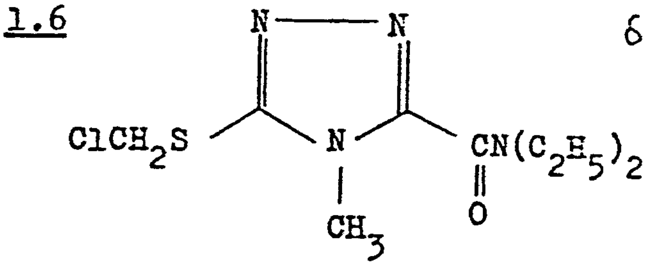

$\delta(CDCl_3)$ 1.22 (3H,t)
1.27 (3H,t)
3.50 (2H,q)
3.51 (3H,s)
3.70 (2H,q)
5.12 (2H,s)

2.6

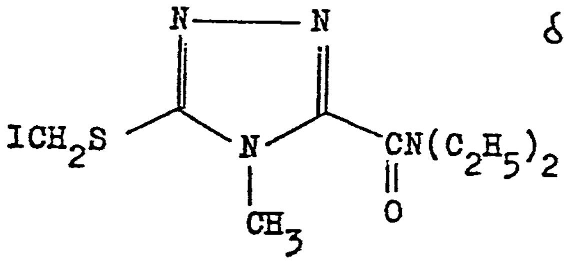

$\delta(CDCl_3)$ 1.22 (3H,t)
1.30 (3H,t)
3.52 (2H,q)
3.75 (3H,s)
3.80 (2H,q)
4.72 (2H,s)

1.7

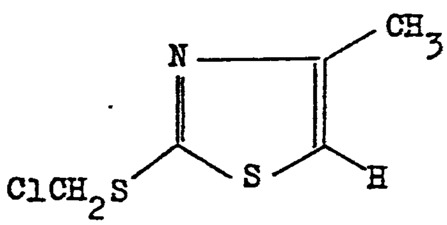

$\delta(CDCl_3)$ 2.43 (3H,s)
5.12 (2H,s)
6.85 (1H,s)

2.7

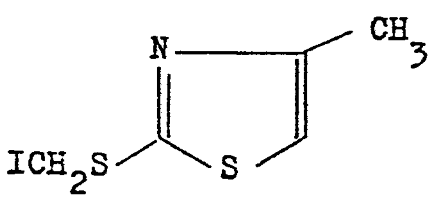

$\delta(CDCl_3)$ 2.44 (3H,s)
4.68 (2H,s)
6.83 (1H,s)

1.8

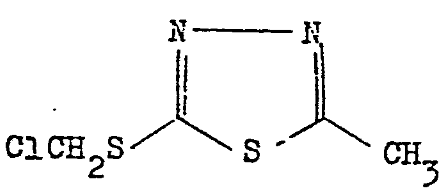

$\delta(CDCl_3)$ 2.80 (3H,s)
5.25 (2H,s)

2.8

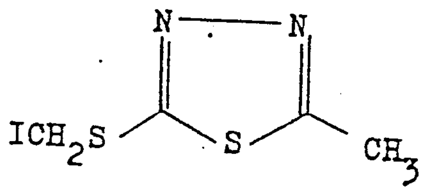

$\delta(CDCl_3)$ 2.80 (3H,s)
4.78 (2H,s)

1.9

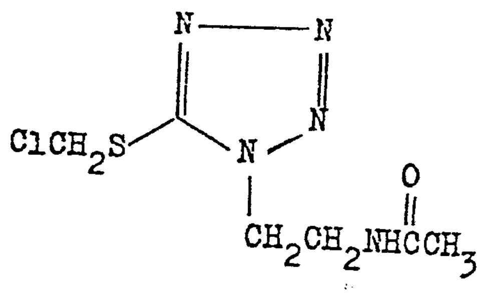

$\delta(CDCl_3)$ 1.99 (3H,s)
3.71 (2H,m)
4.45 (2H,m)
5.26 (2H,s)
7.26 (1H,brs)

2.9

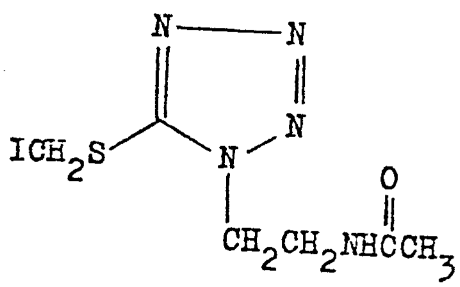

$\delta(CDCl_3)$ 2.02 (3H,s)
3.71 (2H,m)
4.42 (2H,m)
4.75 (2H,s)
6.70 (1H,brs)

1.10

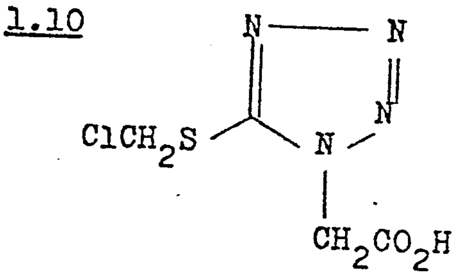

$\delta(CD_3COCD_3)$ 5.38 (2H,s)
5.43 (2H,s)

2.10

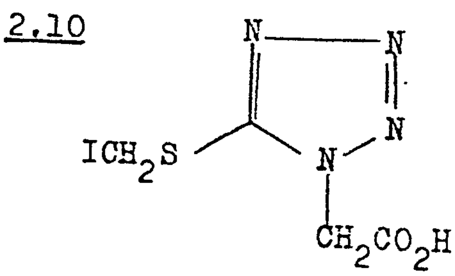

$\delta(CD_3COCD_3)$ 4.93 (2H,s)
5.35 (2H,s)

1.11

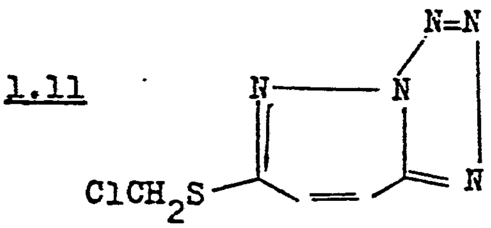

δ($CDCl_3$) 5.38 (2H,s)

7.48-8.40 (2H,AB)

2.11

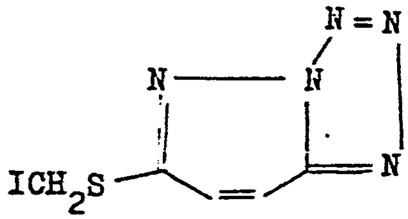

δ($CDCl_3$) 4.75 (2H,s)

7.25-8.15 (2H,AB)

1.12

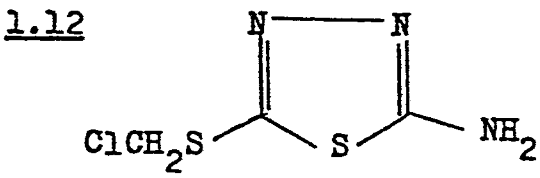

δ($CD_3COCD_3$) 5.28 (2H,s)

6.87 (2H,brs)

2.12

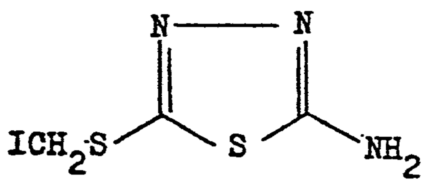

δ($CD_3COCD_3$) 4.86 (2H,s)

6.84 (2H,brs)

1.13

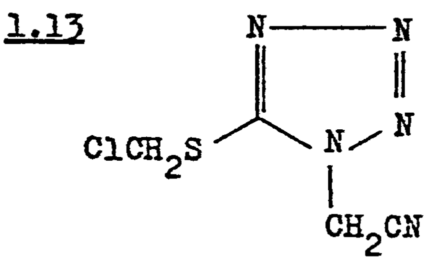

δ($CD_3COCD_3$) 5.55 (2H,s)

5.85 (2H,s)

2.13

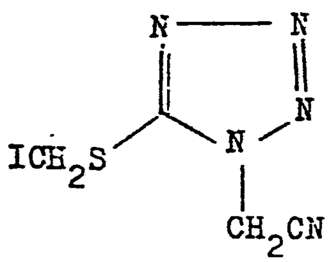

δ($CD_3COCD_3$) 4.99 (2H,s)

5.78 (2H,s)

1.14

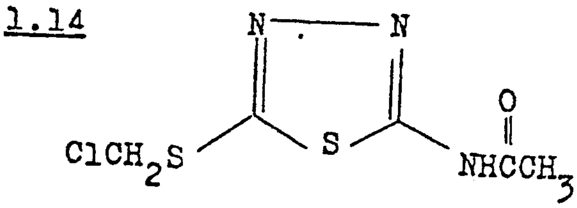

δ($CD_3COCD_3$) 2.31 (3H,s)
5.44 (2H,s)

2.14

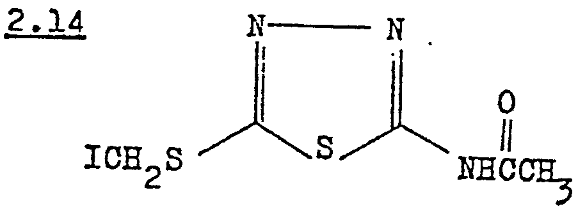

δ($CD_3COCD_3$) 2.33 (3H,s)
4.99 (3H,s)

1.15

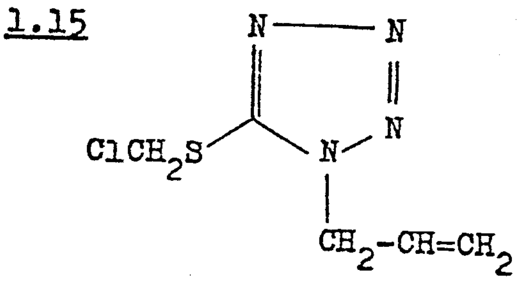

δ($CDCl_3$) 4.85 (2H,d)
5.20 (2H,s)
5.25 (2H,d)
5.73 (1H,m)

2.15

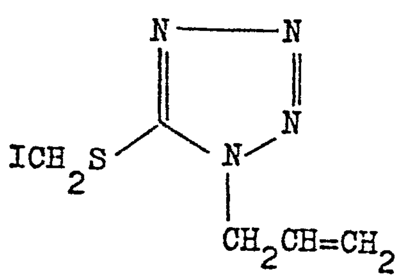

δ($CDCl_3$) 4.80 (2H,s)
4.95 (2H,d)
5.45 (2H,d)
5.95 (1H,m)

1.16

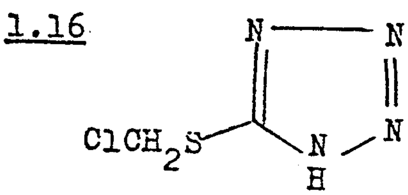

δ($CDCl_3$) 5.21 (2H,s)
8.00 (1H,brs)

2.16

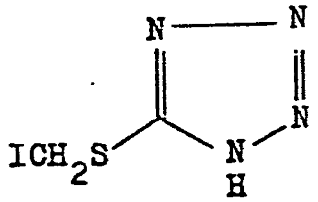

$\delta(CD_3COCD_3)$ 5.00 (2H,s)
5.55 (1H,brs)

1.17

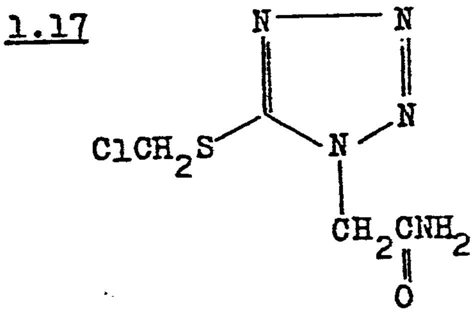

$\delta(CD_3COCD_3)$ 4.93 (2H,s)
5.52 (2H,s)

2.17

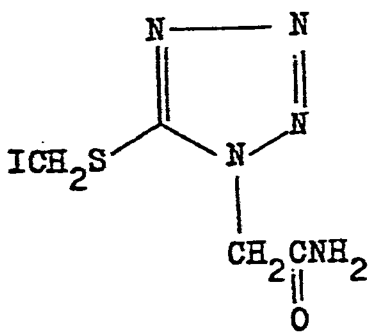

$\delta(CD_3COCD_3)$ 4.93 (2H,s)
5.27 (2H,s)

1.18

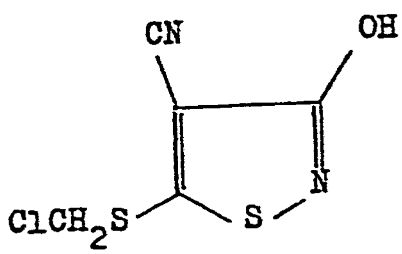

$\delta(CD_3COCD_3)$ 5.47 (2H,s)
11.5 (1H,brs)

2.18

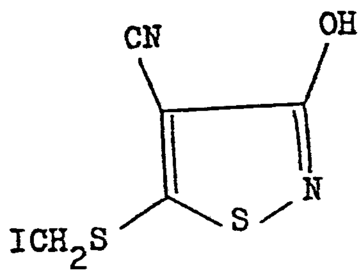

$\delta(CD_3COCD_3)$ 4.96 (2H,s)
11.3 (1H,brs)

1.19

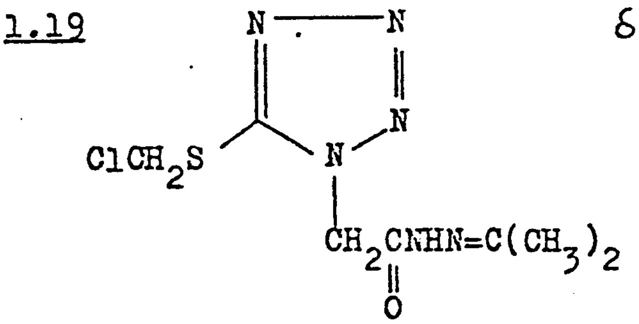

δ($CD_3COCD_3$) 1.80 (3H,s)
2.05 (3H,s)
5.15 (2H,s)
5.51 (2H,s) .

2.19

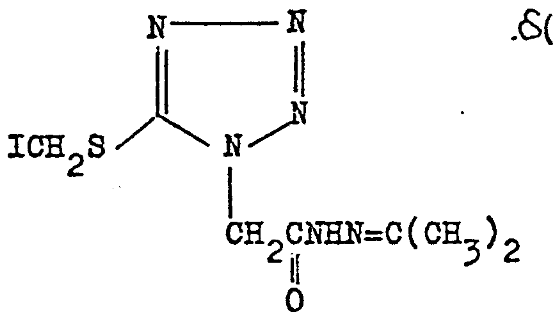

δ($CD_3COCD_3$) 1.95 (3H,s)
2.05 (3H,s)
4.90 (2H,s)
5.58 (2H,s)

1.20

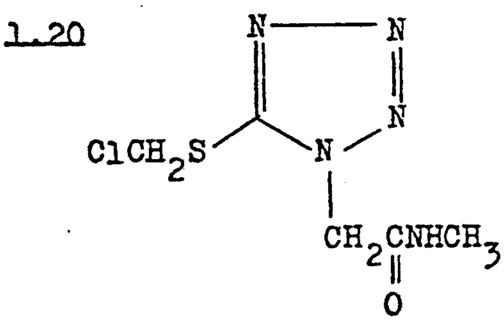

δ($CD_3CN$) 2.85 (3H,d)
5.15 (2H,s)
5.41 (2H,s)

2.20

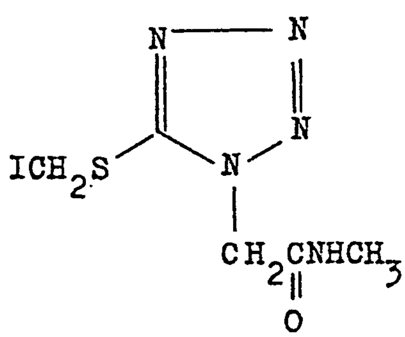

δ($CD_3CN$) 2.83 (3H,d)

1.21

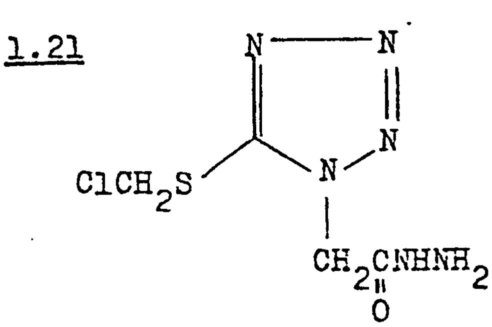

δ($CD_3COCD_3$) 2.90 (3H,brs)
5.41 (2H,s)
5.60 (2H,s)

2.21

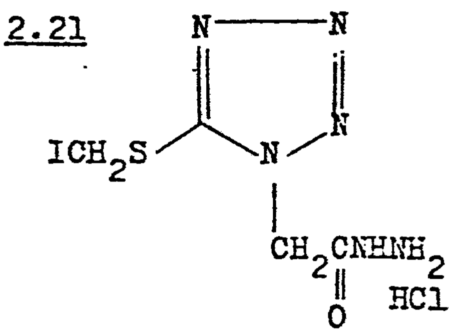

δ($D_2O$) 4.79 (2H,s)

5.41 (2H,s)

1.22

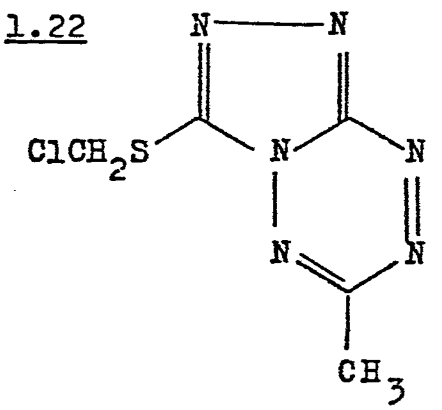

δ($CDCl_3$) 3.08 (3H,s)

5.40 (2H,s)

2.22

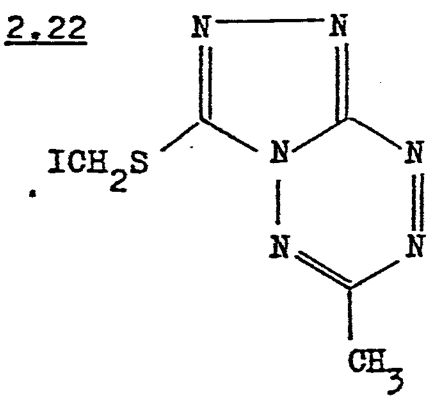

δ($CDCl_3$) 3.10 (3H,s)

4.94 (2H,s)

1.23

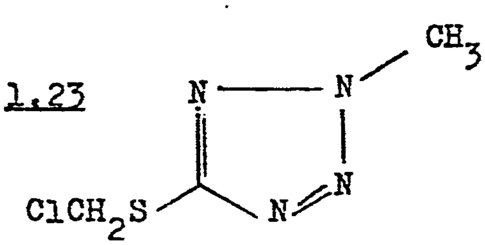

δ($CDCl_3$) 2.63 (3H,s)

6.65 (2H,s)

2.23

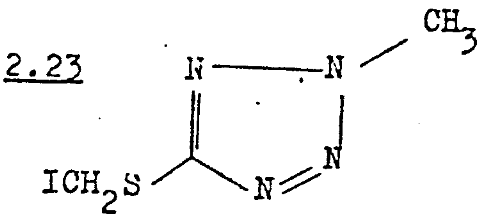

$\delta(CDCl_3)$ 2.65 (3H,s)
6.18 (2H,s)

1.24

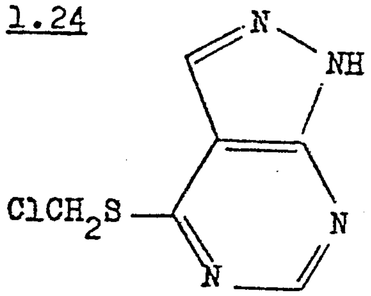

$\delta(CD_3COCD_3)$ 5.65 (2H,s)
8.10 (1H,s)
8.67 (1H,s)

2.24

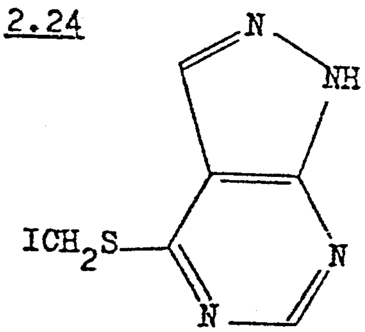

$\delta(CD_3CN)$ 5.15 (2H,s)
8.18 (1H,s)
8.80 (1H,s)

1.25

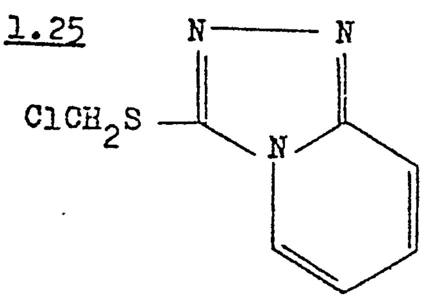

$\delta(CD_3CN)$ 5.05 (2H,s)
6.75-8.45 (4H,m)

2.25

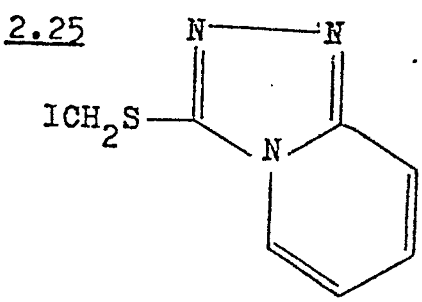

$\delta(CD_3SOCD_3)$ 4.84 (2H,s)
7.00-8.64 (4H,m)

1.26

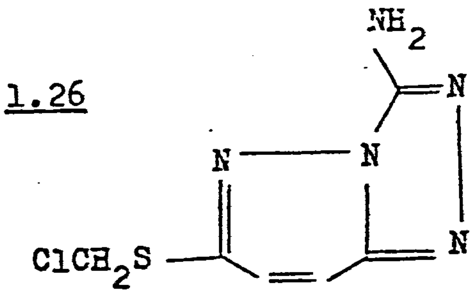

δ($CD_3SOCD_3$) 5.66 (2H,s)
6.64 (2H,brs)
7.04,7.99 (2H,AB)

2.26

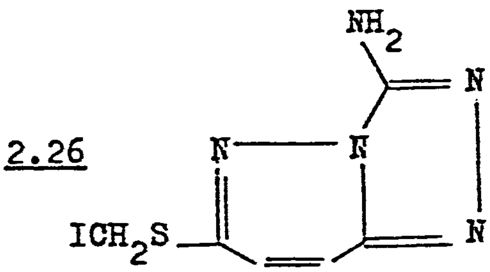

δ($CD_3SOCD_3$) 5.21 (2H,s)
6.61 (2H,brs)
7.01, 7.96 (2H,AB)

1.27

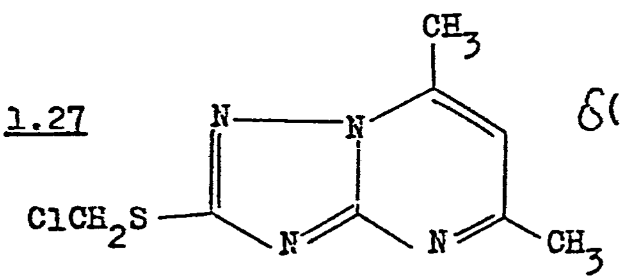

δ($CDCl_3$) 2.60 (3H,s)
2.70 (3H,s)
5.38 (2H,s)
6.71 (1H,s)

2.27

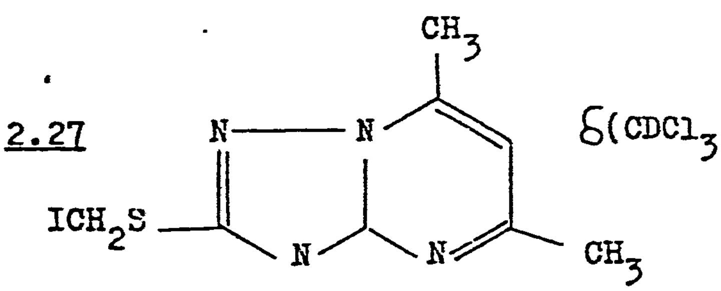

δ($CDCl_3$) 2.63 (3H,s)
2.75 (3H,s)
4.89 (2H,s)
6.70 (1H,s)

1.28

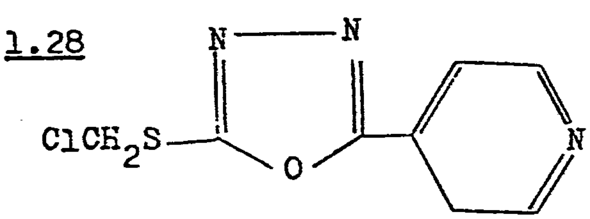

δ($CDCl_3$) 5.25 (2H,s)
7.82, 8.75
(4H, $A_2B_2$)

2.28 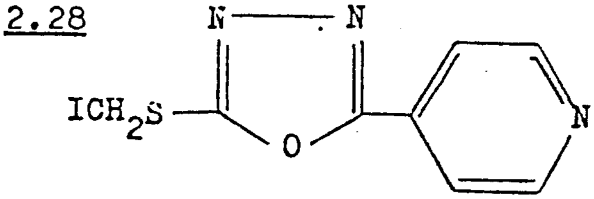 δ($CDCl_3$) 4.65 (2H,s)

7.78, 8.70 (4H, $A_2B_2$)

1.29 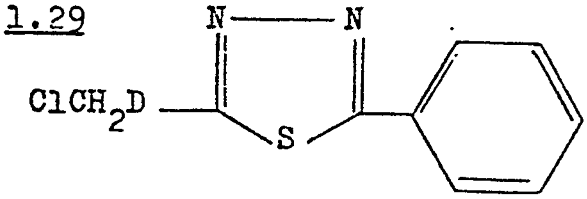 δ($CDCl_3$) 5.31 (2H,s)

7.51-7.95 (5H,m)

2.29 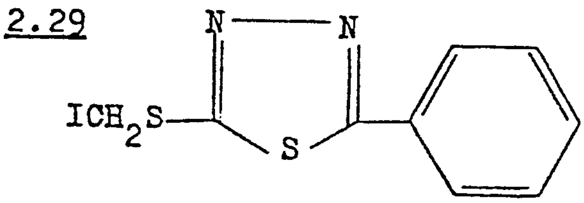 δ($CDCl_3$) 4.70 (2H,s)

7.50-8.10 (5H,m)

1.30 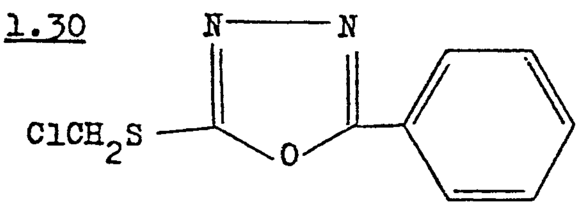 δ($CDCl_3$) 5.26 (2H,s)

7.40-8.10 (5H,m)

2.30 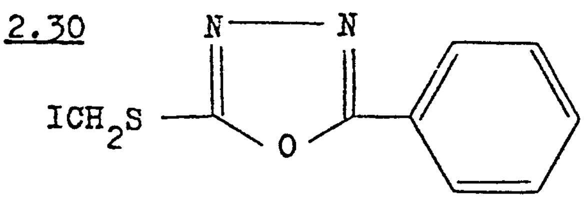 δ($CDCl_3$) 4.65 (2H,s)

7.41-8.10 (5H,m)

1.31 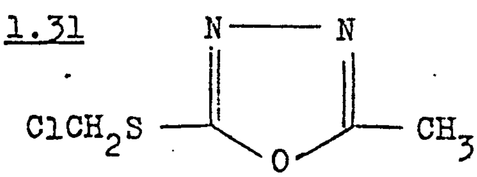 δ($CDCl_3$) 2.56 (3H,s)

5.35 (2H,s)

2.31

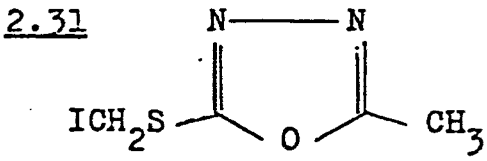

δ($CD_3CN$) 2.68 (3H,s)
4.85 (2H,s)

1.32

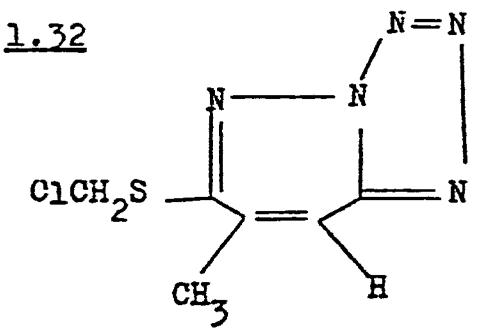

δ($CDCl_3$) 2.50 (3H,s)
5.39 (2H,s)
8.09 (1H,m)

2.32

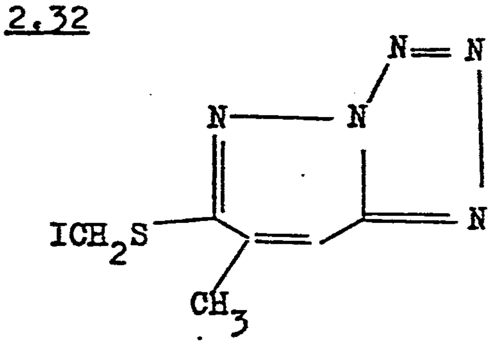

δ($CDCl_3$) 2.44 (3H,s)
4.74 (2H,s)
8.00 (1H,m)

1.33

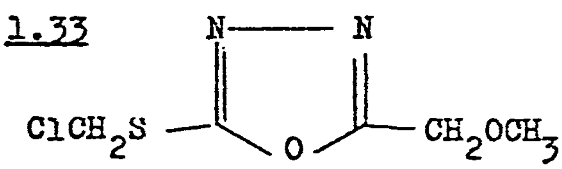

δ($CDCl_3$) 3.53 (3H,s)
4.60 (2H,s)
5.24 (2H,s)

2.33

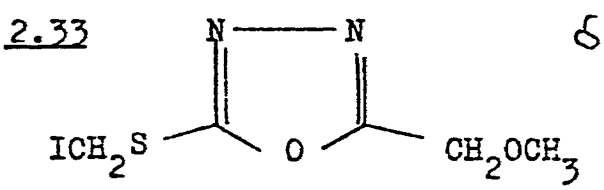

δ($CDCl_3$) 3.45 (3H,s)
4.05 (2H,s)
4.60 (2H,s)

1.34

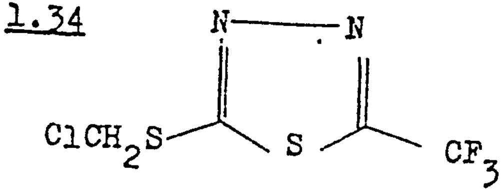

δ($CDCl_3$) 5.33 (2H,s)

2.34

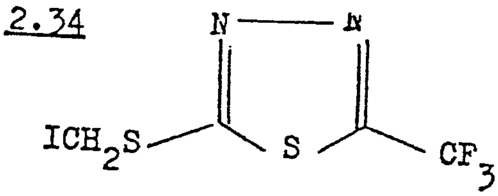

δ($CDCl_3$) 4.90 (2H,s)

1.35

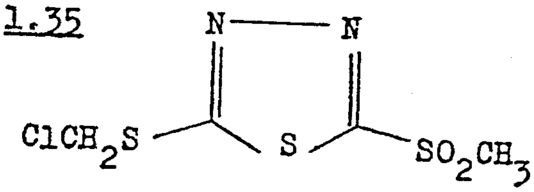

δ($CDCl_3$) 3.12 (3H,s)
5.26 (2H,s)

2.35

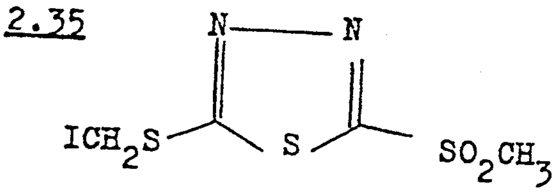

δ($CD_3CN$) 3.05 (3H,s)
4.75 (2H,s)

1.36

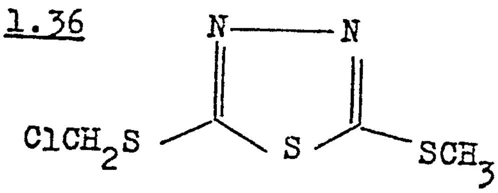

δ($CDCl_3$) 2.80 (3H,s)
5.25 (2H,s)

2.36

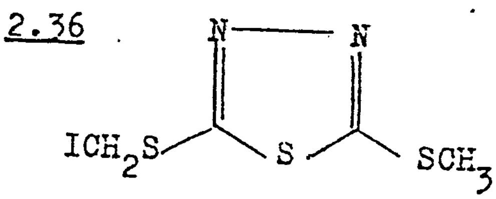

δ($CDCl_3$) 2.85 (3H,s)
4.85 (2H,s)

1.37

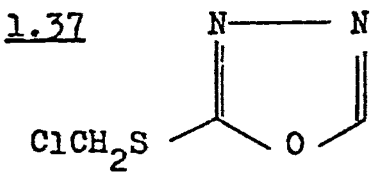

δ($CDCl_3$) 5.05 (2H,s)

8.35 (1H,s)

2.37

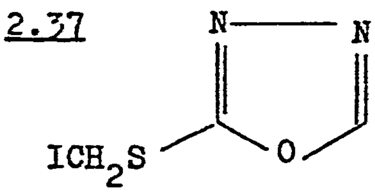

δ($CDCl_3$) 4.75 (2H,s)

8.57 (1H,s)

1.38

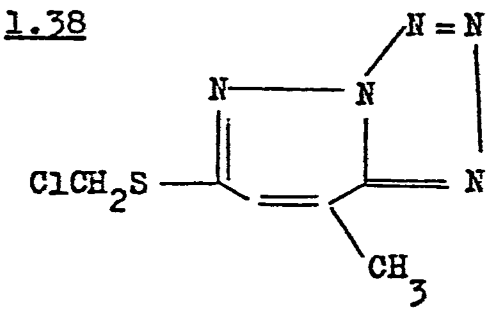

δ($CDCl_3$) 2.86 (3H,s)

5.40 (2H,s)

7.22 (1H,m)

2.38

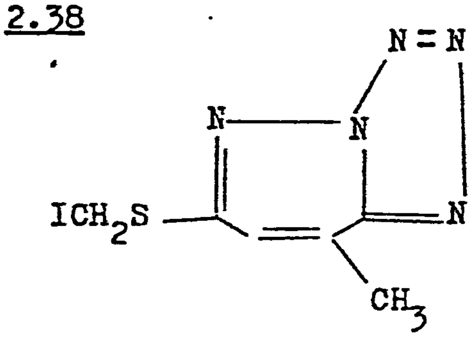

δ($CDCl_3$) 2.74 (3H,s)

4.81 (2H,s)

7.18 (1H,m)

1.39

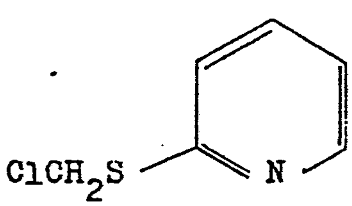

δ($CDCl_3$) 5.03 (2H,s)

6.80-8.50 (4H,m)

2.39

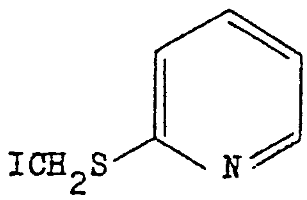

$\delta(CD_3SOCD_3)$ 5.01 (2H,s)
7.10-8.64 (4H,m)

1.40

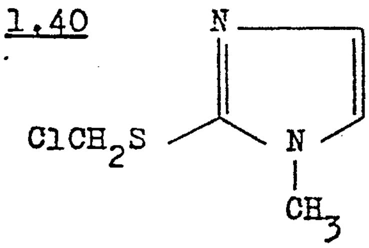

$\delta(CDCl_3)$ 3.70 (3H,s)
4.97 (2H,s)
7.25-7.71 (2H,m)

2.40

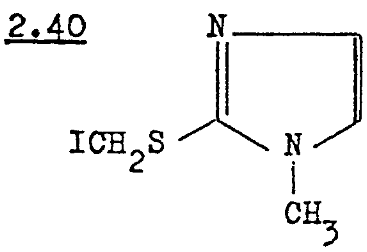

$\delta$ 3.68 (3H,s)
4.59 (2H,s)
7.23-7.69 (2H,m)

2.41

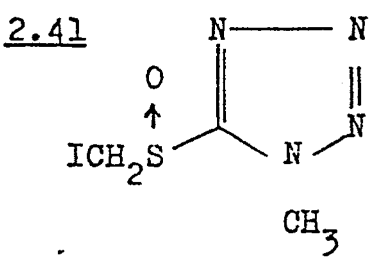

$\delta(CDCl_3)$ 4.35 (3H,s)
4.88 (2H,s)

EXAMPLE 1

4-Nitrobenzyl (5R,6S)-6-(1(R)-hydroxyethyl)-3-(1-methyl-tetrazol-5-yl-thio-methylthio)-7-oxo-4-thia-1-azabicyclo[3.2.0]hept-2-ene-2-carboxylate

To a stirred solution of 154.2mg of 4-nitrobenzyl (5R,6S)-6-(1(R)-hydroxyethyl)-7-oxo-4-thia-3-thioxo-1-azabicyclo[3.2.0]hept-2-ene-2-carboxylate in 10ml dry THF was added 107mg of 5-(iodomethylthio)-1-methyl-tetrazole and 57mg of diisopropylethylamine. The solution was stirred at room temperature for 16 hours and solvents evaporated *in vacuo*. The residue was chromatographed over silica gel, eluting with hexane-ethyl acetate mixtures, to give the title compound as a pale yellow gum (118mg).

$\nu_{max}$ (KBr) 1777cm$^{-1}$

$\delta$ ($d^6$-DMSO)
1.17 (3H, d, J=6.2$H_z$)
3.93 (1H, dd, J=5.8,1.2$H_z$)
3.97 (3H, s)
4.00-4.09 (1H, m)
4.86, 4.94 (2H, ABq, J=13.9$H_z$)
5.29, 5.44 (2H, ABq, J=14.1$H_z$)
5.79 (1H, d, J=1.2$H_z$)
7.62-8.27 (4H,m)

EXAMPLE 2

Potassium (5R,6S)-6-(1(R)-hydroxyethyl)-3-(1-methyl-tetrazol-5-yl-thio-methylthio)-7-oxo-4-thia-1-azabicyclo[3.2.0]hept-2-ene-2-carboxylate

A mixture of 97.1mg of 4-nitrobenzyl (5R,6S)-6-(1(R)-hydroxy ethyl)-3-(1-methyl-tetrazole-5-yl-thio-methylthio)-7-oxo-4-thia-1-azabicyclo[3.2.0]hept-2-ene-2-carboxylate in 10ml of ethyl acetate, 19mg of potassium hydrogen carbonate in water (10ml), and 200mg of 10% palladium/charcoal catalyst was hydrogenated at 50 p.s.i. until T.L.C. analysis indicated complete reaction. The mixture was filtered through a "Hyflo" (Trade Mark" pad and the aqueous layer was separated and lyophilised to give the title compound as an off-white solid (55mg).

$\nu_{max}$ (Nujol) 1785cm$^{-1}$

$\delta$ ($D_2O$) 1.25 (3H, d, J=6.4$H_z$)

3.86 (1H, dd, J=5.9,1.3$H_z$)

4.00 (3H,s)

4.13-4.26 (1H, m)

4.74-4.92 (2H, m)

5.54 (1H, d, J=1.3$H_z$)

EXAMPLE 3

4-Nitrobenzyl (5R,6S)-6-(1(R)-hydroxyethyl)-3-(4-methyl-5-trifluoromethyl-1,2,4-triazol-3-yl-thio-methylthio)-7-oxo-4-thia-1-azabicyclo[3.2.0]hept-2-ene-2-carboxylate

72.4mg of the above compound were obtained by a procedure analogous to that described in Example 1 using 100mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 93mg of 3-iodomethylthio-4-methyl-5-trifluoromethyl-1,2,4-triazole and 55μl of diisopropylethylamine.

$\nu_{max}$ (KBr) 1781cm$^{-1}$

| | |
|---|---|
| ($CDCl_3$) | 1.39 (3H, d, J=6.3$H_z$) |
| | 3.68 (3H, s) |
| | 3.81 (1H, dd, J=6.4,1.5$H_z$) |
| | 4.24-4.35 (1H, br m) |
| | 4.73,4.86 (2H, ABq, J=13.8$H_z$) |
| | 5.21,5.46 (2H, ABq, J=13.7$H_z$) |
| | 5.74 (1H, d, J=1.5$H_z$) |
| | 7.39-8.24 (4H, m) |

EXAMPLE 4

Potassuim (5R,6S)-6-(1(R)-hydroxyethyl)-3-(4-methyl-5-trifluoromethyl-1,2,4-triazol-3-yl-thio-methylthio)-7-oxo-4-thia-1-azabicyclo[3.2.0]-hept-2-ene-2-carboxylate

29mg of the above compound were obtained from 67.5mg of the corresponding 4-nitrobenzyl carboxylate (see Example 3) by a procedure analogous to that described in Example 2, using 11.7mg of potassium hydrogen carbonate.

$\delta$ ($d^6$-DMSO) 1.15 (3H, d, J=6$H_z$)
3.54 (1H, dd, J=6.9,1.3$H_z$)
3.70 (3H, s)
3.88-3.95 (1H, m)
4.51,4.63 (2H, ABq, J=13.6$H_z$)
5.50 (1H, d, J=1.3$H_z$)

EXAMPLE 5

4-Nitrobenzyl (5R,6S)-6-(1(R)-hydroxyethyl)-7-oxo-3-(1,3,4-thiadiazol-2-yl-thio-methylthio)-4-thia-1-azabicyclo[3.2.0]hept-2-ene-2-carboxylate

73.7mg of the above compound were obtained by a procedure analogous to that described in Example 1 using 92.5mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 6o.6mg of 2-iodomethylthio-1,3,4-thiadiazole and 46.3 μl of diisopropylethylamine.

$\nu_{max}$ (film) 1790cm$^{-1}$

δ (CDU3) 1.39 (3H, d, J=6.3Hz)
3.81 (1H, dd, J=6.6,1.6Hz)
4.22-4.36 (1H, m)
4.81,4.91 (2H, ABq, J=13.9Hz)
5.21,5.46 (2H, ABq, J=13.8Hz)
5.74 (1H, d, J=1.6Hz)
7.55-8.26 (4H, m)
9.09 (1H, s)

EXAMPLE 6

Potassium (5R,6S)-6-(1(R)-hydroxyethyl)-7-oxo-3-(1,3,4-thiadiazol-2-yl-thio-methylthio)-4-thia-1-azabicyclo [3.2.0]hept-2-ene-2-carboxylate

55.4mg of the above compound were obtained from 60.4mg of the corresponding 4-nitrobenzyl carboxylate (see Example 5) by a procedure analogous to that described in Example 2, using 13.4mg of potassium hydrogen carbonate.

$\nu_{max}$ (Nujol) $1770 cm^{-1}$

δ ($D_2O$) 1.27 (3H, d, J=6.4Hz)
3.89 (1H, dd, J=5.9,1.2Hz)
4.14-4.29 (1H, m)
4.63,4.85 (2H, ABq, J=14.0Hz)
5.60 (1H, d, J=1.2Hz)
9.42 (1H, s)

EXAMPLE 7

4-Nitrobenzyl (5R,6S)-6-(1(R)-hydroxyethyl)-7-oxo-3-(1H,3,4-triazol-2-yl-thio-methylthio)-4-thia-1-azabicyclo[3.2.0]hept-2-ene-2-carboxylate

77mg of the above compound were obtained by a procedure analogous to that described in Example 1 using 100mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 78.5mg of 2-iodomethylthio-1H,3,4-triazole and 50.2 $\mu l$ of diisopropylethylamine,

$\nu_{max}$ (KBr) 1784cm$^{-1}$

$\delta$ ($(CD_3)_2CO$) 1.36 (3H, d, J=6.2Hz)
3.94 (1H, dd, J=6.2,1.2Hz)
4.18-4.28 (1H, m)
4.74,4.84 (2H, ABq, J=14.2Hz)
5.29,5.51 (2H, ABq, J=14Hz)
5.89 (1H, d, J=1.2Hz)
7.74-8.26 (4H, m)
8.51 (1H, s)
8.53 (1H, br s)

EXAMPLE 8

Potassium (5R,6S)-6-(1(R)-hydroxyethyl)-7-oxo-3-(1H,3,4-triazol-2-yl-thio-methylthio)-4-thia-1-azabicyclo[3.2.0]hept-2-ene-2-carboxylate

43mg of the above compound were obtained from 70mg of the corresponding 4-nitrobenzyl carboxylate (see Example 7) by a procedure analogous to that described in Example 2, using 14.2mg of potassium hydrogen carbonate.

δ ($d^6$-DMSO) 1.26 (3H, d, J=6.4$H_z$)
3.94 (1H, dd, J=6.4,1.4$H_z$)
4.57,4.70 (2H, ABq, J=13.6$H_z$)
5.03-5.14 (1H, m)
5.59 (1H, d, J=1.4$H_z$)
9.36 (1H, s)

EXAMPLE 9

4-Nitrobenzyl (5R,6S)-6-(1(R)-hydroxyethyl)-7-oxo-3-(5-(2-thienyl)-1,2,4-triazol-3-yl-thio-methylthio)-4-thia-1-azabicyclo[3.2.0]hept-2-ene-2-carboxylate

134mg of the above compound were obtained by a procedure analogous to that described in Example 1 using 125mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 158.5mg of 3-iodomethylthio-5-(2-thienyl)-1,2,4-triazole and 68.3 μl of diisopropylethylamine.

$\nu_{max}$ (film) 1790cm$^{-1}$

$\delta$ ($CDCl_3$) 1.38 (3H, d, J=6.3Hz)
3.80 (1H, dd, J=6.3,1.5Hz)
4.24-4.33 (1H, m)
4.60,4.72 (2H, ABq, J=14.2Hz)
5.23,5.48 (2H, ABq, J=14Hz)
5.70 (1H, d, J=1.5Hz)
7.13-8.26 (7H, m)

EXAMPLE 10

Potassium (5R,6S)-6-(1(R)-hydroxyethyl)-7-oxo-3-(5-(2-thienyl)-1,2,4-triazol-3-yl-thio-methylthio)-4-thia-1-azabicyclo[3.2.0]hept-2-ene-2-carboxylate

54mg of the above compound were obtained from 125mg of the corresponding 4-nitrobenzyl ester (see Example 9) by a procedure analogous to that described in Example 2, using 22mg of potassium hydrogen carbonate.

$\nu_{max}$ (KBr) 1774cm$^{-1}$

$\delta$ ($d^6$-DMSO) 1.15 (3H, d, J=6.4Hz)
3.54 (1H, dd, J=6.4, 1.3Hz)
3.85-3.95 (1H, m)
4.39, 4.45 (2H, ABq, J=14.5Hz)
5.10-5.12 (1H, br s)
5.49 (1H, d, J=1.3Hz)
7.04 (1H, dd, J=3.6,4.9Hz)
7.37-7.44 (2H, m)

EXAMPLE 11

4-Nitrobenzyl (5R,6S)-6-(1(R)-hydroxyethyl)-3-(4-metnylthiazol-2-yl-thio-methylthio)-7-oxo-4-thia-1-azabicyclo[3.2.0]hept-2-ene-2-carboxylate.

63mg of the above compound were obtained by a procedure analogous to that described in Example 1 using 111.5mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 87.1mg of 2-iodomethylthio-4-methylthiazole and 58μl of diisopropylethylamine.

$\delta$ ($CDCl_3$)
1.39 (3H, d, J=6.3Hz)
2.44 (3H, d, J=1.0Hz)
3.81 (1H, dd, J=6.6,1.5Hz)
4.24-4.31 (1H, m)
4.62,4.71 (2H, ABq, J=13.6Hz)
5.21,5.46 (2H, ABq, J=13.6Hz)
5.72 (1H, d, J=1.5Hz)
6.85 (1H, q, J=1.0Hz)
7.59-8.25 (4H, m)

EXAMPLE 12

Potassium (5R,6S)-6-(1(R)-hydroxyethyl)-3-(4-methylthiazol-2-ylthio-methylthio)-7-oxo-4-thia-1-azabicyclo[3.2.0]hept-2-ene-2-carboxylate

37.4mg of the above compound were obtained from 60mg of the corresponding 4-nitrobenzyl carboxylate (see Example 11) by a procedure analogous to that described in Example 2, using 11.4mg of potassium hydrogen carbonate.

$\nu_{max}$ (KBr) 1788cm$^{-1}$

$\delta$ (d$^6$-DMSO) 1.15 (3H, d, J=6.0Hz)
2.34 (3H, d, J=0.9Hz)
3.54 (1H, dd, J=6.2,1.3Hz)
3.86-3.95 (1H, m)
4.48,4.63 (2H, ABq, J=13.6Hz)
5.52 (1H, d, J=1.3Hz)
7.28 (1H, q, J=0.9Hz)

EXAMPLE 13

4-Nitrobenzyl (5R,6S)-6-(1(R)-hydroxyethyl)-7-oxo-3-(1H,2,3,4-tetrazol-5-yl-thio-methylthio)-4-thia-1-azabicyclo[3.2.0]hept-2-ene-2-carboxylate

118mg of the above compound were obtained by a procedure analogous to that described in Example 1 using 154mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 107mg of 5-iodomethylthio-1H,2,3,4-tetrazole and 77$\mu$l of diisopropylethylamine.

$\nu_{max}$ (KBr) 1772$cm^{-1}$

$\delta$ ($d^6$-$Me_2CO$) 1.32 (3H, d, J=6.4Hz)
3.93 (1H, dd, J=5.2,1.5Hz)
4.15-4.26 (1H, m)
4.86,4.94 (2H, ABq, J=14.0Hz)
5.28,5.50 (2H, ABq, J=13.6Hz)
5.88 (1H, d, J=1.5Hz)
7.74-8.26 (4H, m)

EXAMPLE 14

Potassium (5R,6S)-6-(1(R)-hydroxyethyl)-7-oxo-3-(1H,2,3,4-tetrazol-5-yl-thio-methylthio)-4-thia-1-azabicyclo[3.2.0]hept-2-ene-2-carboxylate

67mg of the above compound were obtained from 110mg of the corresponding 4-nitrobenzyl carboxylate (see Example 13) by a procedure analogous to that described in Example 2, using 22mg of potassium hydrogen carbonate.

δ ($d^6$-DMSO) 1.15 (3H, d, J=6.3Hz)
3.60 (1H, dd, J=6.7,1.4Hz)
3.86-3.96 (1H, m)
4.36,4.44 (2H, ABq, J=13.1Hz)
5.55 (1H, d, J=1.4Hz)

EXAMPLE 15

4-Nitrobenzyl (5R,6S)-3-(1-carbamoylmethyl-tetrazol-5-yl-thio-methylthio-6-(1(R)-hydroxyethyl)-7-oxo-4-thia-azabicyclo[3.2.0]hept-2-ene-2-carboxylate

97.7mg of the above compound were obtained by a procedure analogous to that described in Example 1 using 150mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 129mg of 1-carbamoylmethyl-5-iodomethylthio-tetrazol and 75 $\mu L$ of diisopropylethylamine.

$\nu_{max}$ (KBr) 1790$cm^{-1}$

$\delta$ ($d^6$-DMSO) 1.16 (3H, d, J=6.2$H_z$)
3.93 (1H, dd, J=5.8,1.4$H_z$)
3.98-4.06 (1H, m)
4.82,4.90 (2H, ABq, J=13.7$H_z$)
5.10 (2H, s)
5.29,5.40 (2H, ABq, J=14.1$H_z$)
5.79 (1H, d, J=1.4$H_z$)
7.63-8.25 (4H, m)

EXAMPLE 16

Potassium (5R,6S)-3-(1-carbamoylmethyl-tetrazol-5-yl-thio-methylthio)-6-(1(R)-hydroxyethyl)-7-oxo-4-thia-1-azabicyclo [3.2.0]hept-2-ene-2-carboxylate

58mg of the above compound were obtained from 90mg of the corresponding 4-nitrobenzyl carboxylate (see Example 15) by a procedure analogous to that described in Example 2, using 16.3mg of potassium hydrogen carbonate.

$\delta$ ($d^6$-DMSO) 1.14 (3H, d, J=5.8$H_z$)
3.56 (1H, dd, J=6.8,1.3$H_z$)
3.86-3.96 (1H, m)
4.52,4.64 (2H, ABq, J=13.5$H_z$)
5.12 (2H, s)
5.50 (1H, d, J=1.3$H_z$)
7.55 (1H, br s)
8.07 (1H, br s)

EXAMPLE 17

4-Nitrobenzyl (5R,6S)-6-(1(R)-hydroxyethyl)-3-(1-(N-methylcarbamoyl methyl)-tetrazol-5-yl-thio-methylthio)-7-oxo-4-thia-1-azabicyclo[3.2.0]hept-2-ene-2-carboxylate

140mg of the above compound were obtained by a procedure analogous to that described in Example 1 using 137mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 123mg of 5-(iodomethylthio)-1-(N-methylcarbamoylmethyl)-tetrazol and 68.5 μL of diisopropylethylamine.

$\nu_{max}$ (film) 1781cm$^{-1}$

$\delta$ ($d^6$-$(CH_3)_2CO$)
1.31 (3H, d, J=6.3Hz)
2.80 (3H, s)
3.93 (1H, dd, J=6.2,1.5Hz)
4.17-4.29 (1H, m)
4.83,4.91 (2H, ABq, J=13.8Hz)
5.18 (2H, s)
5.30,5.51 (2H, ABq, J=14.0Hz)
5.88 (1H, d, J=1.5Hz)
7.57-7.71 (1H, br s)
7.74-8.29 (4H, m)

EXAMPLE 18

Potassium (5R,6S)-6-(1(R)-hydroxyethyl)-3-(1-N-methylcarbamoylmethyl)-tetrazol-5-yl-thio-methylthio)-7-oxo-4-thia-1-azabicyclo[3.2.0]hept-2-ene-2-carboxylate

74mg of the above compound were obtained from 132mg of the corresponding 4-nitrobenzyl carboxylate (see Example 17) by a procedure analogous to that described in Example 2, using 23.2mg of potassium hydrogen carbonate.

$\nu_{max}$ (KBr) 1771cm$^{-1}$

| | |
|---|---|
| ($D_2O$) | 1.30 (3H, d, J=6.4Hz) |
| | 2.80 (3H, s) |
| | 3.92 (1H, dd, J=6.0,1.4Hz) |
| | 4.18-4.32 (1H, m) |
| | 4.63,4.79 (2H, ABq, J=14.2Hz) |
| | 5.29 (2H, s) |
| | 5.63 (1H, d, J=1.4Hz) |

EXAMPLE 19

4-Nitrobenzyl (5R,6S)-3-(1-(diazanylcarboxymethyl)-tetrazol-5-yl-thio-methylthio)-6-(1(R)-hydroxyetnyl)-7-oxo-4-thia-1-azabicyclo[3.2.0]hept-2-ene-2-carboxylate

160mg of the above compound were obtained by a procedure analogous to that described in Example 1 using 162mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 163mg of 1-(diazanylcarboxymethyl)-5-iodomethylthio-tetrazol and 81μl of diisopropylethylamine

| | | |
|---|---|---|
| δ ($CD_3CN$) | 1.24 | (3H, d, J=6.3Hz) |
| | 3.26-3.41 | (1H, br s) |
| | 3.87 | (1H, dd, J=6.4,1.4Hz) |
| | 4.10-4.17 | (1H, m) |
| | 4.70,4.79 | (2H, ABq, J=13.9Hz) |
| | 4.97 | (2H, s) |
| | 5.23,5.43 | (2H, ABq, J=13.8Hz) |
| | 5.75 | (1H, d, J=1.4Hz) |
| | 7.63-8.23 | (2H, m) |
| | 7.90-8.02 | (1H, br s) |

EXAMPLE 20

Potassium (5R,6S)-3-(1-(diazanylcarboxymethyl)-tetrazol-5-yl-thio-methylthio)-6-(1(R)-hydroxyethyl)-7-oxo-4-thia-1-azabicyclo [3.2.0] hept-2-ene-2-carboxylate

82mg of the above compound were obtained from 160mg of the corresponding 4-nitrobenzyl carboxylate (see Example 19) by a procedure analogous to that described in Example 2, using 28.2mg of potassium hydrogen carbonate.

$\delta$ ($d^6$-DMSO) 1.14 (3H, d, J=6.4$H_z$)
3.57 (1H, dd, J=6.8,1.3$H_z$)
3.85-3.95 (1H, m)
4.46-4.73 (2H, m)
5.08 (2H, s)
5.35-5.47 (1H, br m)
5.51 (1H, d, J=1.3$H_z$)
9.60-9.96 (2H, br s)

EXAMPLE 21

4-Nitrobenzyl (5R,6S)-6-(1(R)-hydroxyethyl)-7-oxo-3-(5-phenyl-1,3,4-oxadiazol-2-yl-thio-methylthio)-4-thia-1-azabicyclo[3.2.0]hept-2-ene-2-carboxylate

190mg of the above compound were obtained by a procedure analogous to that described in Example 1 using 202mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 185mg of 2-iodomethylthio-5-phenyl-1,3,4-oxadiazole and 107μl of diisopropylethylamine.

| | |
|---|---|
| δ ($d^6$-DMSO) | 1.16 (3H, d, J=6.2Hz) |
| | 3.94 (1H, dd, J=6.0,1.4Hz) |
| | 3.98-4.05 (1H, m) |
| | 4.95,5.03 (2H, ABq, J=14.1Hz) |
| | 5.28,5.43 (2H, ABq, J=14.1Hz) |
| | 5.80 (1H, d, J=1.4Hz) |
| | 7.57-8.23 (9H, m) |

EXAMPLE 22

Potassium (5R,6S)-6-(1(R)-hydroxyethyl)-7-oxo-3-(5-phenyl-1,3,4-oxadiazol-2-yl-thio-methylthio)-4-thia-1-azabicyclo[3.2.0]hept-2-ene-2-carboxylate

68mg of the above compound were obtained from 184mg of the corresponding 4-nitrobenzyl carboxylate (see Example 21) by a procedure analogous to that described in Example 2, using 32.2mg of potassium hydrogen carbonate.

$\nu_{max}$ (KBr) 1774cm$^{-1}$

$\delta$ ($d^6$-DMSO) 1.13 (3H, d, J=6.2$H_z$)
3.56 (1H, dd, J=6.4,1.3$H_z$)
3.85-3.94 (1H, m)
4.67,4.79 (2H, ABq, J=13.7$H_z$)
5.10-5.36 (1H, br s)
5.53 (1H, d, J=1.3$H_z$)
7.60-8.02 (5H, m)

EXAMPLE 23

4-Nitrobenzyl (5R,6S)-6-(1(R)-hydroxyethyl)-3-(5-methoxymethyl-1,3,4-oxadiazol-2-yl-thio-methylthio)-7-oxo-4-thia-1-azabicyclo [3.2.0] hept-2-ene-2-carboxylate

120mg of the above compound were obtained by a procedure analogous to that described in Example 1 using 179mg of 4-nitrobenzyl (5R,6S)-6-(1(R)-hydroxyethyl)-7-oxo-4-thia-3-thioxo-1-azabicyclo [3.2.0] hept-2-ene-2-carboxylate, 147mg of 2-iodomethylthio-5-methoxymethyl-1,3,4-oxadiazole and 89.5 μl of diisopropylethylamine.

$\nu_{max}$ (KBr) 1780cm$^{-1}$

$\delta$ ($d^6$-acetone) 1.18 (3H, d, J=6.0Hz)
3.32 (3H, s)
3.94 (1H, dd, J=6.0,1.4Hz)
3.98-4.07 (1H, m)
4.66 (2H, s)
4.86,4.96 (2H, ABq, J=14Hz)
5.29,5.44 (2H, ABq, J=14Hz)
5.80 (1H, d, J=1.4Hz)
7.65-8.25 (4H, m)

EXAMPLE 24

Potassium (5R,6S)-6-(1(R)-hydroxyethyl)-3-(5-methoxymethyl) 1,3,4-oxadiazol-1-yl-thio-methylthio)-7-oxo-4-thia-1-azabicyclo [3.2.0]hept-2-ene-2-carboxylate

54.5mg of the above compound were obtained from 111mg of the corresponding 4-nitrobenzyl carboxylate (see Example 23) by a procedure analogous to that described in Example 2, using 20.6mg of potassium hydrogen carbonate.

δ ($d^6$-DMSO)

1.14 (3H, d, J=6.4Hz)

3.40 (3H, s)

3.57 (1H, dd, J=7.6,1.4Hz)

4.65 (2H, s)

4.58,4.73 (2H, ABq, J=14Hz)

5.52 (1H, d, J=1.4Hz)

EXAMPLE 25

4-Nitrobenzyl (5R,6S)-6-(1(R)-hydroxyethyl)-7-oxo-3-(5-phenyl-1,3,4-thiadiazol-2-yl-thio-methylthio)-4-thia-1-azabicyclo[3.2.0]hept-2-ene-2-carboxylate

118mg of the above compound were obtained by a procedure analogous to that described in Example 1 using 187mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 179mg of 2-iodomethylthio-5-phenyl-1,3,4-thiadiazole and 94 $\mu l$ of diispropylethylamine.

$\nu_{max}$ (film) 1800cm$^{-1}$

$\delta$ ($d^6$-DMSO) 1.17 (3H, d, J=6.0$H_z$)
3.94 (1H, dd, J=6.0,1.4$H_z$)
3.98-4.05 (1H, m)
4.97,5.05 (2H, ABq, J=14.0$H_z$)
5.29,5.44 (2H, ABq, J=14.0$H_z$)
5.81 (1H, d, J=1.4$H_z$)
7.55-8.23 (9H, m)

EXAMPLE 26

Potassium (5R,6S)-6-(1(R)-hydroxyethyl)-7-oxo-3-(5-phenyl-1,3,4-thiadiazol-2-yl-thio-methylthio)-4-thia-1-azabicyclo[3.2.0]hept 2-ene-2-carboxylate

49mg of the above compound were obtained from 115mg of the corresponding 4-nitrobenzyl carboxylate (see Example 25) by a procedure analogous to that described in Example 2, using 19.6mg of potassium hydrogen carbonate.

$\nu_{max}$ (KBr) 1774$cm^{-1}$

$\delta$ ($d^6$-DMSO) 1.14 (3H, d, J=6.0$H_z$)
3.54 (1H, dd, J=8.1,1.3$H_z$)
3.85-3.95 (1H, m)
4.67,4.82 (2H, ABq, J=13.8$H_z$)
5.52 (1H, d, J=1.3$H_z$)
7.55-7.98 (5H, m)

EXAMPLE 27

4-Nitrobenzyl (5R,6S)-6-(1(R)-hydroxyethyl)-7-oxo-3-(5-(4-pyridyl)-1,3,4-oxadiazol-2-yl-thio-methylthio)-4-thia-1-azabicyclo[3.2.0]hept-2-ene-2-carboxylate

131.5mg of the above compound were obtained by a procedure analogous to that described in Example 1 using 209mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 192mg of 2-(iodomethylthio-5-(4-pyridyl)-1,3,4-oxadiazole and 105μl of diisopropylethylamine.

$\nu_{max}$ (KBr) 1778$cm^{-1}$

| | |
|---|---|
| δ ($d^6$-DMSO) | 1.17 (3H, d, J=6.5Hz) |
| | 3.94 (1H, dd, J=6.0,1.3Hz) |
| | 3.99-4.03 (1H, m) |
| | 4.98,5.06 (2H, ABq, J=14.1Hz) |
| | 5.27,5.44 (2H, ABq, J=13.7Hz) |
| | 5.81 (1H, d, J=1.3Hz) |
| | 7.64-8.85 (6H, m) |

EXAMPLE 28

Potassium (5R,6S)-6-(1(R)-hydroxyethyl-7-oxo-3-(5-(4-pyridyl)-1,3,4-oxadiazol-2-yl-thio-methylthio)-4-thia-1-azabicyclo[3.2.0]hept-2-ene-2-carboxylate

52.5mg of the above compound were obtained from 127mg of the corresponding 4-nitrobenzyl carboxylate (see Example 27) by a procedure analogous to that described in Example 2, using 22.2mg of potassium hydrogen carbonate.

$\nu_{max}$ (film) 1779cm$^{-1}$

$\delta$ ($D_2O$) 1.22 (3H, d, J=6.5Hz)
3.83 (1H, dd, J=5.9,1.4Hz)
4.12-4.23 (1H, m)
4.72,4.87 (2H, ABq, J=14Hz)
5.57 (1H, d, J=1.4Hz)
7.93-8.73 (4H, m)

EXAMPLE 29

4-Nitrobenzyl (5R,6S)-6-(1(R)-hydroxyethyl)-7-oxo-3-(1,2,4-triazolo[4,3-a]pyridin-3-yl-thio-methylthio)-4-thia-1-azabicyclo[3.2.0]hept-2-ene-2-carboxylate

169mg of the above compound were obtained by a procedure analogous to that described in Example 1 using 150mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 126mg of 3-iodomethylthio-1,2,4-triazolo[4,3-a]pyridine and 75μl of diispropylethylamine.

| | |
|---|---|
| δ ($CDCl_3$) | 1.39 (3H, d, J=6.4Hz) |
| | 2.47-2.61 (1H, br s) |
| | 3.76 (1H, dd, J=6.8,1.4Hz) |
| | 4.22-4.32 (1H, m) |
| | 4.51,4.66 (2H, ABq, J=13.9Hz) |
| | 5.16,5.41 (2H, ABq, J=13.7Hz) |
| | 5.66 (1H, d, J= 1.4Hz) |
| | 6.90-8.26 (8H, m) |

EXAMPLE 30

Potassium (5R,6S)-6-(1(R)-hydroxyethyl)-7-oxo-3-(1,2,4-triazolo [4,3-a]pyridin-3-yl-thio-methylthio)-4-thia-1-azabicyclo[3.2.0] hept-2-ene-2-carboxylate

80mg of the above compound were obtained from 160mg of the corresponding 4-nitrobenzyl carboxylate (see Example 29) by a procedure analogous to that described in Example 2, using 29.4mg of potassium hydrogen carbonate.

| | | |
|---|---|---|
| $\delta$ ($d^6$-DMSO) | 1.14 | (3H, d, J=5.2Hz) |
| | 3.51 | (1H, dd, J=5.9,1.4Hz) |
| | 3.84-3.95 | (1H, m) |
| | 4.38,4.50 | (2H, ABq, J=13.5Hz) |
| | 5.40 | (1H, d, J=1.4Hz) |
| | 7.07-8.50 | (4H, m) |

EXAMPLE 31

4-Nitrobenzyl (5R,6S)-6-(1(R)-hydroxyethyl)-3-(5-methyl-1,3,4-oxadiazol-2-yl-thio-methylthio)-7-oxo-4-thia-1-azabicyclo[3.2.0]hept-2-ene-2-carboxylate

110mg of the above compound were obtained by a procedure analogous to that described in Example 1 using 183mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 134mg of 2-iodomethylthio-5-methyl-1,3,4-oxadiazole and 91.6 μl of diisopropylethylamine.

δ ($d^6$DMSO) 1.17 (3H, d, J=6.0Hz)
2.50 (3H, s)
3.94 (1H, dd, J=1.2,6.0Hz)
3.99-4.05 (1H, m)
4.83,4.91 (2H, AB, J=11.0Hz)
5.29,5.45 (2H, AB, J=15.0Hz)
5.80 (1H, d, J=1.2Hz)
7.62,8.27 (4H,m)

EXAMPLE 32

Potassium (5R,6S)-6-(1(R)-hydroxyethyl)-3-(5-methyl-1,3,4-oxadiazol-2-yl-thio-methylthio)-7-oxo-4-thia-1-azabicyclo[3.2.0]-hept-2-ene-2-carboxylate

58mg of the above compound were obtained from 104mg of the corresponding 4-nitrobenzyl carboxylate (see Example 31) by a procedure analogous to that described in Example 2, using 20.5mg of potassium hydrogen carbonate.

$\delta$ ($d^6$DMSO)

1.17 (3H, d, J=7.0$H_z$)
2.49 (3H, s)
3.50 (1H, dd, J=1.4$H_z$,7.0$H_z$)
3.86-3.96 (1H, m)
4.53,4.67 (2H, AB, J=14.0$H_z$)
5.53 (1H, d, J=1.4$H_z$)

EXAMPLE 33

4-Nitrobenzyl (5R,6S)-6-(1(R)-hydroxyethyl)-3-(1-carboxymethyl-tetrazol-5-yl-thio-methylthio)-7-oxo-4-thia-1-azabicyclo[3.2.0]-hept-2-ene-2-carboxylate

67mg of the above compound were obtained by a procedure analogous to that described in Example 1 using 150mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 171mg of 1-carboxymethyl-5-iodothiomethyl-tetrazol and 82 μl of diisopropylethylamine.

$\nu_{max}$ (KBr) 1789$cm^{-1}$

$\delta$ ($CD_3COCD_3$) 1.32 (3H, d, J=6.0$H_z$)
3.94 (1H, dd, J=6.2, 1.5$H_z$)
4.15-4.27 (1H, m)
4.88,4.96 (2H, AB, J=13.8$H_z$)
5.29,5.52 (2H, AB, J=14.0$H_z$)
5.39 (2H,s)
5.88 (1H, d, J=1.5$H_z$)
7.74-8.27 (4H,m)

EXAMPLE 34

Potassium (5R,6S)-6-(1(R)-hydroxyethyl)-3-(1-carboxymethyl-tetrazol-5-yl-thio-methylthio)-7-oxo-4-thia-1-azabicyclo[3.2.0]-hept-2-ene-2-carboxylate

27mg of the above compound were obtained from 61mg of the corresponding 4-nitrobenzyl carboxylate (see Example 33) by a procedure analogous to that described in Example 2, using 11mg of potassium hydrogen carbonate.

| | |
|---|---|
| $\delta$ ($d^6$DMSO) | 1.15 (3H, d, J=6.0Hz) |
| | 3.46 (1H, dd, J=5.8,1.2Hz) |
| | 3.86-3.96 (1H, m) |
| | 4.53,4.64 (2H, AB, J=16.1Hz) |
| | 4.58 (2H, s) |
| | 5.53 (1H, d, J=1.2Hz) |

EXAMPLE 35

4-Nitrobenzyl (5R, 6S)-6-(1(R)-hydroxyethyl)-3-(2-methyltetrazol-5-yl-thio-methylthio)-7-oxo-4-thia-1-azabicyclo[3.2.0] hept-2-ene-2-carboxylate

125mg of the above compound were obtained by a procedure analogous to that described in Example 1 using 127mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 93.5mg of 5-iodomethylthio-2-methyltetrazole and 63.6µl of diisopropylamine.

($d^6$-DMSO) 1.17 (3H,d, J=6.4$H_z$)
2.85 (3H, s)
3.94 (1H, dd, J=6.3,1.2$H_z$)
3.97-4.06 (1H, m)
4.85,5.00 (2H, ABq, J=14.0$H_z$)
5.29,5.48 (2H, ABq, J=13.8$H_z$)
5.82 (1H, d, J=1.2$H_z$)
7.61,8.33 (4H, m)

EXAMPLE 36

Potassium (5R,6S)-6-(1(R)-hydroxyethyl)-3-(2-methyltetrazol-5-yl-thio-methylthio)-7-oxo-4-thia-1-azabicyclo [3.2.0] hept-2-ene-2-carboxylate

72mg of the above compound were obtained from 120.7mg of the corresponding 4-nitrobenzyl carboxylate (see Example 35) by a procedure analogous to that described in Example 2, using 23.7mg of potassium hydrogen carbonate.

$\nu_{max}$ (KBr) 1774cm$^{-1}$

$\delta$ ($d^6$DMSO) 1.13 (3H, d, J=6.5$H_z$)
2.66 (3H, s)
3.51 (1H, dd, J=1.3,7.3$H_z$)
3.82-3.96 (1H, m)
5.45 (1H, d, J=1.3$H_z$)
5.89,6.20 (2H, ABq J=14.4$H_z$)

EXAMPLE 37

4-Nitrobenzyl (5R,6S)-6-(1(R)-hydroxyethyl)-3-(1-methyltetrazol-5-yl-sulphinyl-methylthio)-7-oxo-4-thia-1-azabicyclo[3.2.0] hept-2-ene-2-carboxylate

214mg of the above compound (isolated as a 1:1 mixture of R and S sulphoxides) were obtained by a procedure analogous to that described in Example 1 using 300mg of 4-nitrobenzyl (5R,6S)-6-(1(R)-hydroxyethyl)-7-oxo-4-thia-3-thioxo-1-azabicyclo[3.2.0]-hept-2-ene-2-carboxylate, 270mg of 5-iodomethylsulphinyl-1-methyltetrazole and 149 μl of diisopropylethylamine.

δ ($CDCl_3$)

1.38 (3H, d, J=6.0Hz)

3.83 (1H, dd, J=6.0,1.2Hz)

4.20-4.32 (1H, m)

4.34 (3H, s)

4.58,5.18 (2H {ABq, J=15.0Hz)

4.77,4.92 (2H {ABq, J=15.0Hz)

5.23,5.50 (2H, ABq, J=10.0Hz)

5.71 (1H {d, J=1.2Hz)

5.74 (1H {d, J=1.2Hz)

7.58,8.26 (4H, m)

EXAMPLE 38

Potassium (5R,6S)-6-(1(R)-hydroxyethyl)-3-(1-methyltetrazol-5-yl-sulphinyl-methylthio)-7-oxo-4-thia-1-azabicyclo[3.2.0]-hept-2-ene-2-carboxylate

122mg of the above compound (isolated as a 1:1 mixture of R and S sulphoxides) were obtained from 200mg of the corresponding 4-nitrobenzyl carboxylate (see Example 37) by a procedure analogous to that described in Example 2, using 37mg of potassium hydrogen carbonate.

$\nu_{max}$ (KBr) 1774cm$^{-1}$

$\delta$ ($d^6$DMSO) 1.14 (3H, d, J=6.2Hz)

3.83-3.94 (1H, m)

4.10 (s) } 3H
4.24 (s) }

4.69,4.97 (2H, ABq, J=13.6Hz)

5.40 (d, J=1.1Hz) } 1H
5.46 (d, J=1.1Hz) }

EXAMPLE 39

4-Nitrobenzyl (5R,6S)-6-(1(R)-hydroxyethyl)-3-(3-amino-1,2,4-triazolo[4,3-b]pyridazin-6-yl-thio-methylthio)-7-oxo-4-thia-1-azabicyclo[3.2.0]hept-2-ene-carboxylate

91mg of the above compound were obtained by a procedure analogous to that described in Example 1 using 87mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 67mg of 3-amino-6-iodomethylthio-1,2,4-triazolo[4,3,b]pyridazine and 40 μl of diisopropylethylamine.

| | |
|---|---|
| δ ($d^6$DMSO) | 1.15 (3H, d, J=6.0Hz) |
| | 3.92 (1H, dd, J=6.0,1.2Hz) |
| | 3.99-4.05 (1H, m) |
| | 4.91,4.97 (2H, ABq, J=14.5Hz) |
| | 5.26,5.41 (2H, ABq, J=14.0Hz) |
| | 5.79 (2H, d, J=1.2Hz) |
| | 6.70 (2H, br s) |
| | 6.95,7.95 (2H, ABq, J=8.0Hz) |
| | 7.61,8.24 (4H, m) |

EXAMPLE 40

Potassium (5R,6S)-6-(1(R)-hydroxyethyl)-3-(3-amino-1,2,4-triazolo [4,3-b]pyridazin-6-yl-thio-methylthio)-7-oxo-4-thia-1-azabicyclo [3.2.0]hept-2-ene-2-carboxylate

50mg of the above compound were obtained from 84.6mg of the corresponding 4-nitrobenzyl carboxylate (see Example 39) by a procedure analogous to that described in Example 2, using 15.1mg of potassium hydrogen carbonate.

$\nu_{max}$ (KBr) 1786cm$^{-1}$

$\delta$ ($d^6$DMSO)

1.14 (3H, d, J=5.3$H_z$)
3.53 (1H, dd, J=6.9,1.3$H_z$)
3.85-3.95 (1H, m)
4.67 (2H, s)
5.50 (1H, d, J=1.3$H_z$)
6.66 (2H, brs)
6.94,7.86 (2H, ABq,J=9.6$H_z$)

EXAMPLE 41

4-Nitrobenzyl (5R,6S)-3-(4-cyano-3-hydroxy-isothiazol-5-yl-thio-methylthio)-6-(1(R)-hydroxyethyl)-7-oxo-4-thia-1-azabicyclo[3.2.0]hept-2-ene-2-carboxylate

213mg of the above compound were obtained by a procedure analogous to that described in Example 1 using 210mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 180mg of 4-cyano-3-hydroxy-5-iodomethylthio-isothiazole and 105 $\mu l$ of diisopropylethylamine.

$\nu_{max}$ (KBr) 2202,1786cm$^{-1}$

$\delta$ ($CD_3COCD_3$) 1.35 (3H, d, J=6.2$H_z$)
3.96 (1H, dd, J=6.2,1.5$H_z$)
4.17-4.29 (1H, m)
4.67,4.75 (2H, ABq, J=14.0$H_z$)
5.31,5.52 (2H, ABq, J=14.1$H_z$)
5.90 (1H, d, J=1.5$H_z$)
7.76-8.29 (4H, m)

EXAMPLE 42

Potassium (5R,6S)-3-(4-cyano-3-hydroxy-isothiazol-5-yl-thiomethylthio)-6-(1(R)-hydroxyethyl)-7-oxo-4-thia-1-azabicyclo [3.2.0]hept-2-ene-2-carboxylate.

90mg of the above compound were obtained from 194mg of the corresponding 4-nitrobenzyl carboxylate (see Example 41) by a procedure analogous to that described in Example 2, using 35.2mg of potassium hydrogen carbonate.

| | |
|---|---|
| $\delta$ ($d^6$-DMSO) | 1.15 (3H, d, J=5.2$H_z$) |
| | 3.60 (1H, dd, J=6.2,1.4$H_z$) |
| | 3.85-3.97 (1H, m) |
| | 4.50,4.60 (2H, ABq, J=13.8$H_z$) |
| | 5.53 (1H, d, J=1.4$H_z$) |
| | 9.70-9.81 (1H, br s) |

EXAMPLE 43

4-Nitrobenzyl (5R,6S)-3-(5,7-dimethyl-1,2,4-triazolo[1,5-a]pyrimidin-2-yl-thio-methylthio)-6-(1(R)-hydroxyethyl)-7-oxo-4-thia-1-azabicyclo[3.2.0]hept-2-ene-2-carboxylate

48mg of the above compound were obtained by a procedure analogous to that described in Example 1 using 84mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 77mg of 5,7-dimethyl-2-iodomethylthio-1,2,4-triazolo[1,5-a]pyrimidine and 41 µl of diisopropylethylamine.

$\nu_{max}$ (KBr) 1776cm$^{-1}$

$\delta$ ($d^6$-DMSO

| | |
|---|---|
| 1.18 | (3H, d, J=6.2Hz) |
| 2.56 | (3H, s) |
| 2.67 | (3H, s) |
| 3.94 | (1H, dd, J=5.7,1.4Hz) |
| 3.98-4.08 | (1H, m) |
| 4.85,4.94 | (2H, ABq, J=13.8Hz) |
| 5.26,5.41 | (2H,ABq, J=13.1Hz) |
| 5.81 | (1H, d, J=1.4Hz) |
| 7.17 | (1H, s) |
| 7.63-8.23 | (4H, m) |

EXAMPLE 44

Potassium (5R,6S)-3-(5,7-dimethyl-1,2,4-triazolo[1,5-a]pyrimidin-2-yl-thio-methylthio)-6-(1(R)-hydroxyethyl)-7-oxo-4-thia-1-azabicyclo[3.2.0]hept-2-ene-2-carboxylate

23mg of the above compound were obtained from 45.6mg of the corresponding 4-nitrobenzyl carboxylate (see Example 43) by a procedure analogous to that described in Example 2, using 8mg of potassium hydrogen carbonate.

| | |
|---|---|
| $\delta$ ($d^6$-DMSO) | 1.13 (3H, d, J=6.1$H_z$) |
| | 2.55 (3H, s) |
| | 2.68 (3H, s) |
| | 3.53 (1H, dd, J=6.7,1.3$H_z$) |
| | 3.80-3.94 (1H, m) |
| | 4.55,4.65 (2H, ABq, J=13.6$H_z$) |
| | 5.51 (1H, d, J=1.3$H_z$) |
| | 7.13 (1H, s) |

EXAMPLE 45

4-Nitrobenzyl (5R,6S)-6-(1(R)-hydroxyethyl)-7-oxo-3-(pyrazolo [3,4-d]pyrimidin-4-yl-thio-methylthio)-4-thia-1-azabicyclo[3.2.0] hept-2-ene-2-carboxylate

109mg of the above compound were obtained by a procedure analogous to that described in Example 1 using 150mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 13mg of 4-iodomethylthio-1H-pyrazolo[3,4-d]pyrimidine and 75μl of diisopropylethylamine.

$\nu_{max}$ (film) 1780cm$^{-1}$

$\delta$ ($d^6$-DMSO) 1.18 (3H, d, J=6.2Hz)

3.94 (1H, dd, J=5.7,1.3Hz)

3.98-4.06 (1H, m)

4.98,5.06 (2H, ABq, J=13.8Hz)

5.26,5.42 (2H, ABq, J=14.0Hz)

5.82 (1H, d, J=1.3Hz)

7.63-8.23 (m, 4H)

8.37 (s, 1H)

8.84 (s, 1H)

EXAMPLE 46

Potassium (5R,6S)-6-(1(R)-hydroxyethyl)-7- oxo-3-(pyrazolo[3,4-d]pyrimidin-4-yl-thio-methylthio)-4-thia-1-azabicyclo[3.2.0]hept-2-ene-2-carboxylate

31mg of the above compound were obtained from 100mg of the corresponding 4-nitrobenzyl carboxylate (see Example 45) by a procedure analogous to that described in Example 2, using 18mg of potassium hydrogen carbonate.

$\nu_{max}$ (KBr) 1778cm$^{-1}$.

$\delta$ ($d^6$-DMSO)
1.15 (3H, d, J=6.2Hz)
3.55 (1H, dd, J=6.8,1.2Hz)
3.87-3.97 (1H, m)
4.68,4.75 (2H, ABq, J=13.6Hz)
5.53 (1H, d, J=1.0Hz)
8.17 (1H, s)
8.59 (1H, s)

EXAMPLE 47

4-Nitrobenzyl (5R,6S)-6-(1(R)-hydroxyethyl)-3-((1-methyl-1-ethylenediazane)-carboxymethyl-tetrazol-5-yl-thiomethylthio)-7-oxo-4-thia-1-azabicyclo[3.2.0]hept-2-ene-2-carboxylate

144mg of the above compound were obtained by a procedure analogous to that described in Example 1 using 120mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 111mg of 5-iodomethylthio-1-((1-methyl-1-ethylene-diazane)-carboxymethyl)-tetrazol and 60μl of diisopropylethylamine

$\nu_{max}$ (film) 1790cm$^{-1}$

$\delta$ ($CD_3COCD_3$) 1.31 (3H, d, J=6.3$H_z$)
2.03 (3H, s)
2.07 (3H, s)
3.92 (1H, dd, J=6.0,1.5$H_z$)
4.13-4.26 (1H, m)
4.45-4.57 (1H, br s)
4.82,4.90 (2H, ABq, J=13.3$H_z$)
5.30,5.51 (2H, ABq, J=14.0$H_z$)
5.56 (2H, s)
5.86 (1H, d, J=1.5$H_z$)
7.74-8.27 (4H, m)
9.76-9.84 (1H, br s)

EXAMPLE 48

Potassium (5R,6S)-6-(1(R)-hydroxyethyl)-3-((1-methyl-1-ethylene-diazane)-carboxymethyl- tetrazol- 5-yl-thio-methylthio)-7-oxo-4-thia-1-azabicyclo[3.2.0]hept-2-ene-2-carboxylate

68mg of the above compound were obtained from 134mg of the corresponding 4-nitrobenzyl carboxylate (see Example 47) by a procedure analogous to that described in Example 2, using 22mg of potassium hydrogen carbonate.

$\nu_{max}$ (KBr) 1774cm$^{-1}$

$\delta$ ($D_2O$) 1.25 (3H, d, J=6.4Hz)

2.01 (3H, s)

2.06 (3H, s)

3.88 (1H, dd, J=5.7,1.3Hz)

4.17-4.25 (1H, m)

4.63,4.80 (2H, ABq, J=13.6Hz)

5.41 (2H, s)

5.60 (1H, d, J=1.4Hz)

EXAMPLE 49

4-Nitrobenzyl (5R,6S)-6-(1(R)-hydroxyethyl)-3-(1-methyl-tetrazol-5-yl-thio-ethylthio)-7-oxo-4-thia-1-azabicyclo[3.2.0]hept-2-ene-2-carboxylate

82mg of the above compound were obtained by a procedure analogous to that described in Example 1 using 102mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 79mg of 5-(2-iodoethyl) thio-1-methyl-tetrazol and 51 μl of diisopropylethylamine.

$\nu_{max}$ (KBr) 1797cm$^{-1}$

δ ($d^6$-DMSO)
1.17 (3H, d, J=6.2Hz)
3.24-3.68 (4H, m)
3.88 (1H, dd, J=5.8,1.3Hz)
3.94 (3H, s)
3.95-4.09 (1H, m)
5.29,5.44 (2H, ABq, J=14.9Hz)
5.76 (1H, d, J=1.3Hz)
7.64-8.29 (4H, m)

EXAMPLE 50

Potassium (5R,6S)-6-(1(R)-hydroxyethyl)-3-(1-methyl-tetrazol-5-yl-thio-ethylthio)-7-oxo-4-thia-1-azabicyclo[3.2.0]hept-2-ene-2-carboxylate

31.3mg of the above compound were obtained from 76mg of the corresponding 4-nitrobenzyl carboxylate (see Example 49) by a procedure analogous to that described in Example 2, using 14.5mg of potassium hydrogen carbonate.

$\nu_{max}$ (KBr) 1770cm$^{-1}$

$\delta$ ($D_2O$) 1.28 (3H, d, J=6.5Hz)
3.05-3.75 (4H, m)
3.86 (1H, dd, J=6.0,1.1Hz)
4.00 (3H, s)
4.12-4.30 (1H, m)
4.79,4.90 (2H, ABq, J=13.9Hz)
5.60 (1H, d, J=1.1Hz)

EXAMPLE 51

4-Nitrobenzyl (5R,6S)-6-(1(R)-hydroxyethyl)-3-(2-methylsulphonyl-1,3,4-thiadiazol-5-yl-thio-methylthio)-7-oxo-4-thia-1-azabicyclo [3.2.0]hept-2-ene- 2-carboxylate

155mg of the above compound were obtained by a procedure analogous to that described in Example 1 using 146mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 122mg of 5-iodomethylthio-2-methylsulphonyl-1,3,4-thiadiazole and 73μl of diisopropylethylamine.

$\nu_{max}$ (film) 1787cm$^{-1}$

$\delta$ ($d^6$DMSO) 1.17 (3H, d, J=6Hz)
3.13 (3H, s)
3.95 (1H, dd, J=6.0,1.2Hz)
3.98-4.07 (1H, m)
4.97,5.07 (2H, ABq, J=15Hz)
5.29,5.45 (2H, ABq, J=14Hz)
5.80 (1H, d, J=1.2Hz)
7.65-8.25 (4H, m)

EXAMPLE 52

Potassium (5R,6S)-6-(1(R)-hydroxyethyl)-3-(2-methylsulphonyl-1,3,4-thiadiazol-5-yl-thio-methylthio)-7-oxo-4-thia-1-azabicyclo[3.2.0]hept-2-ene- 2-carboxylate

180mg of the above compound were obtained from 150mg of the corresponding 4-nitrobenzyl carboxylate (see Example 51) by a procedure analogous to that described in Example 2, using 26mg of potassium hydrogen carbonate.

$\nu_{max}$ (KBr) 1772cm$^{-1}$

$\delta$ ($d^6$DMSO) 1.14 (3H, d, J=6$H_z$)
3.14 (3H, s)
3.56 (1H, dd, J=6.8,1.2$H_z$)
3.86-3.95 (1H, m)
4.62,4.88 (2H, ABq,J=13$H_z$)
5.52 (1H, d, J=1.2$H_z$)

EXAMPLE 53

4-Nitrobenzyl (5R,6S)-6-(1(R)-hydroxyethyl)-3-(5-trifluoromethyl-1,3,4-thiadiazol-2-yl-thio-methylthio)-7-oxo-4-thia-1-azabicyclo [3.2.0] hept-2-ene-2-carboxylate

221mg of the above compound were obtained by a procedure analogous to that described in Example 1, using 173mg of 4-nitrobenzyl (5R,6S)-6-(1(R)-hydroxyethyl)-7-oxo-4-thia-3-thioxo-1-azabicyclo [3.2.0] hept-2-ene-2-carboxylate, 162mg of 2-iodomethylthio-5-trifluoromethyl-1,3,4-thiadiazole and 87μl of diisopropylethylamine.

$\nu_{max}$ (film) 1780cm$^{-1}$

δ ($d^6$DMSO) 1.18 (3H, d, J=6.0Hz)
3.94 (1H, dd, J=6.0,1.3Hz)
3.99-4.05 (1H, m)
5.01,5.10 (2H, ABq, J=14.0Hz)
5.29,5.44 (2H, ABq, J=14.0Hz)
5.81 (1H, d, J=1.3Hz)
7.63-8.27 (4H, m)

EXAMPLE 54

Potassium (5R,6S)-6-(1(R)-hydroxyethyl)-3-(5-trifluoromethyl-1,3,4-thiadiazol-2-yl-thio-methylthio)-7-oxo-4-thia-1-azabicyclo-[3.2.0]hept-2-ene-2-carboxylate

76mg of the above compound were obtained from 209mg of the corresponding 4-nitrobenzyl carboxylate (see Example 53) by a procedure analogous to that described in Example 2, using 36mg of potassium hydrogen carbonate.

$\nu_{max}$ (KBr) 1784cm$^{-1}$

$\delta$ ($d^6$DMSO) 1.14 (3H, d, J=6.0Hz)
3.57 (1H, dd, J=6.0,1.2Hz)
3.86-3.96 (1H, m)
4.73,4.89 (2H, ABq, J=14.2Hz)
5.53 (1H, d, J=1.2Hz)

EXAMPLE 55

4-Nitrobenzyl (5R,6S)-6-(1(R)-hydroxyethyl)-3-(5-methylthio-1,3,4-thiadiazol-2-yl-thio-methylthio)-7-oxo-4-thia-1-azabicyclo[3.2.0]hept-2-ene-2-carboxylate

58mg of the above compound were obtained by a procedure analogous to that described in Example 1 using 175mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 174mg of 2-iodomethylthio-5-methylthio-1,3,4-thiadiazole and 100 $\mu l$ of diisopropylamine.

$\nu_{max}$ (KBr) 1755cm$^{-1}$

$\delta$ ($d^6$DMSO)
1.18 (2H, d, J=6.2Hz)
2.76 (3H, s)
3.93 (1H, dd, J=6.5,1.2Hz)
3.97-4.08 (1H, m)
4.86,4.95 (2H, ABq, J=14.0Hz)
5.29,5.44 (2H, ABq, J=14.2Hz)
5.79 (1H, d, J=1.2Hz)
7.62-8.29 (4H, m)

EXAMPLE 56

Potassium (5R,6S)-6-(1(R)-hydroxyethyl)-3-(5-methylthio-thiadiazol-2-yl-thio-methylthio)-7-oxo-4-thia-1-azabicyclo[3.2.0]-hept-2-ene-2-carboxylate

28mg of the above compound were obtained from 55mg of the corresponding 4-nitrobenzyl carboxylate (see Example 55) by a procedure analogous to that described in Example 2 using 10mg of potassium hydrogen carbonate.

$\delta$ ($d^6$DMSO)

1.14 (3H, d, J=6.2$H_z$)

2.76 (3H, s)

3.57 (1H, dd, J=6.0,1.2$H_z$)

3.86-3.97 (1H, m)

4.57,4.72 (2H, $AB_q$, J=14$H_z$)

5.52 (1H, d, J=1.2$H_z$)

EXAMPLE 57

4-Nitrobenzyl (5R,6S)-6-(1(R)-hydroxyethyl)-3-(1,3,4-oxadiazol-2-yl-thio-methylthio)-7-oxo-4-thia-1-azabicyclo[3.2.0]hept-2-ene-2-carboxylate

286mg of the above compound were obtained by a procedure analogous to that described in Example 1 using 285mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 190mg of 2-iodomethylthio-1,3,4-oxadiazole and 137 $\mu l$ of diisopropylethylamine.

$\nu_{max}$ (film) 1770$cm^{-1}$

$\delta$ ($d^6$DMSO) 1.17 (3H, d, J=6.1Hz)
3.94 (1H, dd, J=5.7,1.4Hz)
3.99-4.07 (1H, m)
4.89,4.96 (2H, ABq, J=14Hz)
5.29,5.44 (2H, ABq, J=14.1Hz)
5.80 (1H, d, J=1.4Hz)
7.65-8.27 (4H, m)
9.40 (1H, s)

EXAMPLE 58

Potassium (5R,6S)-6-(1(R)-hydroxyethyl)-3-(1,3,4-oxadiazol-2-yl-thio-methylthio)-7-oxo-4-thia-1-azabicyclo[3.2.0]hept-2-ene-carboxylate

145.5mg of the above compound were obtained from 262mg of the corresponding 4-nitrobenzyl carboxylate (see Example 57) by a procedure analogous to that described in Example 2, using 57.2mg of potassium hydrogen carbonate.

$\nu_{max}$ (KBr) 1773cm$^{-1}$

| | |
|---|---|
| $\delta$ ($d^6$DMSO) | 1.14 (3H, d, J=5.9Hz) |
| | 3.56 (1H, dd, J=6.8,1.1Hz) |
| | 3.86-3.96 (1H, m) |
| | 4.58,4.73 (2H, ABq, J=13.7Hz) |
| | 5.53 (1H, d, J=1.1Hz) |
| | 9.35 (1H, s) |

EXAMPLE 59

4-Nitrobenzyl (5R,6S)-6-(1(R)-hydroxyethyl)-7-oxo-3-(1-(2-propenyl)-tetrazol-5-yl-thio-methylthio)-4-thia-1-azabicyclo[3.2.0]hept-2-ene-2-carboxylate

164mg of the above compound were obtained by a procedure analogous to that described in Example 1, using 150mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 121.7mg of 1-(2-propenyl)-5-iodomethylthio-tetrazole and 75 $\mu l$ of diisopropylethylamine.

$\nu_{max}$ (KBr) 1790cm$^{-1}$.

$\delta$ ($d^6$-DMSO) 1.18 (3H, d, J=6.5$H_z$)

3.93 (1H, dd, J=5.8,1.3$H_z$)

3.97-4.10 (1H, m)

4.90-5.48 (8H, m)

5.79 (1H, d, J=1.3$H_z$)

5.86-6.07 (1H, m)

7.63-8.33 (4H, m)

0127847

EXAMPLE 60

Potassium (5R,6S)-6-(1(R)-hydroxyethyl)-7-oxo-3-(1-propyl) tetrazol-5-yl-thio-methylthio)-4-thia-1-azabicyclo-[3.2.0]hept-2-ene-2-carboxylate

86.3mg of the above compound were obtained from 156.7mg of the corresponding 4-nitrobenzyl carboxylate (see Example 59) by a procedure analogous to that described in Example 2, using 29.2mg of potassium hydrogen carbonate.

$\nu_{max}$ (KBr) 1772cm$^{-1}$

$\delta$ ($D_2O$)
0.86 (3H, t, J=7.5Hz)
1.28 (3H, d, J=6.5Hz)
1.79-2.00 (2H, m)
3.89 (1H, dd, J=5.9,1.3Hz)
4.17-4.28 (1H, m)
4.36 (2H, t, J=7.1Hz)
4.64,4.79 (2H, ABq, J=13.6Hz)
5.61 (1H, d, J=1.3Hz)

EXAMPLE 61

4-Nitrobenzyl (5R,6S)-3-(1-(2-acetylaminoethyl)-tetrazol-5-yl-thio-methylthio)-6-(1(R)-hydroxyethyl)-7-oxo-4-thia-1-azabicyclo[3.2.0]hept-2-ene-2-carboxylate

118.2mg of the above compound were obtained by a procedure analogous to that described in Example 1 using 100.3mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 94.3mg of 1-(2-acetylaminoethyl)-5-iodomethylthio-tetrazol and 50 μl of diisopropylethylamine.

$\nu_{max}$ (film) 1778 $cm^{-1}$

$\delta$ ($CDCl_3$)
1.39 (3H, d, J=6.3$H_z$)
1.98 (3H, s)
3.70-3.83 (2H, m)
3.84 (1H, dd, J=7.1,1.3$H_z$)
4.20-4.33 (1H, m)
4.40 (2H, t, J=5.7$H_z$)
4.74,4.84 (2H, ABq, J=13.8$H_z$)
5.21,5.45 (2H, ABq, J=13.6$H_z$)
5.76 (1H, d, J=1.3$H_z$)
5.92 (1H, br s)
7.56-8.27 (4H, m)

EXAMPLE 62

Potassium (5R,6S)-3-(1-(2-acetylaminoethyl)-tetrazol-5-yl-thio-methylthio)-6-(1(R)-hydroxyethyl)-7-oxo-4-thia-1-azabicyclo [3.2.0]hept-2-ene-2-carboxylate

55.3mg of the above compound were obtained from 114.6mg of the corresponding 4-nitrobenzyl carboxylate (see Example 61) by a procedure analogous to that described in Example 2, using 19.7mg of potassium hydrogen carbonate.

$\nu_{max}$ (KBr) 1770cm$^{-1}$

$\delta$ ($D_2O$) 1.27 (3H, d, J=6.4Hz)
1.87 (3H, s)
3.55-3.73 (2H, m)
3.91 (1H, dd, J=5.6,1.3Hz)
4.15-4.30 (1H, m)
4.48-4.64 (2H, m)
4.67,4.83 (2H, ABq, J=13.7Hz)
5.64 (1H, d, J=1.3Hz)

EXAMPLE 63

4-Nitrobenzyl (5R,6S)-3-(5-amino-1,3,4-thiadiazol-2-yl-thiomethylthio)-6-(1(R)-hydroxyethyl)-7-oxo-4-thia-1-azabicyclo [3.2.0]hept-2-ene-2-carboxylate

125mg of the above compound were obtained by a procedure analogous to that described in Example 1 using 151mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 119mg of 5-amino-2-iodomethylthio-1,3,4-thiadiazole and 76 μl of diisopropylethylamine.

$\nu_{max}$ (KBr) 1781cm$^{-1}$

$\delta$ ($d^6$-DMSO) 1.16 (3H, d, J=6.2$H_z$)
3.90 (1H, dd, J=5.8,1.4$H_z$)
3.94-4.06 (1H, m)
4.64,4.73 (2H, ABq, J=13.7$H_z$)
5.28,5.43 (2H, ABq, J=14.1$H_z$)
5.76 (1H, d, J=1.4$H_z$)
7.62-8.27 (4H, m)

EXAMPLE 64

Potassium (5R,6S)-3-(5-amino-1,3,4-thiadiazol-2-yl-thio-methylthio)-6-(1(R)-hydroxyethyl)-7-oxo-4-thia-1-azabicyclo[3.2.0]hept-2-ene-2-carboxylate

65mg of the above compound were obtained from 116mg of the corresponding 4-nitrobenzyl carboxylate (see Example 63) by a procedure analogous to that described in Example 2, using 22mg of potassium hydrogen carbonate.

$\nu_{max}$ (KBr) 1769cm$^{-1}$

$\delta$ ($D_2O$) 1.27 (3H, d, J=6.3Hz)
3.85 (1H, dd, J=5.7,1.3Hz)
4.14-4.29 (1H, m)
4.61,4.83 (2H, ABq, J=13.9Hz)
5.55 (1H, d, J=1.3Hz)

EXAMPLE 65

4-Nitrobenzyl (5R,6S)-3-(1-cyanomethyl-tetrazol-5-yl-thio-methylthio)-6-(1 (R)-hydroxyethyl)-7-oxo-4-thia-1-azabicyclo [3.2.0] hept-2-ene-2-carboxylate

241mg of the above compound were obtained by a procedure analogous to that described in Example 1 using 250mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 161mg of 1-cyanomethyl-5-iodomethylthio-tetrazole and 100 μl of diisopropylethylamine.

δ ($d^6$-acetone) 1.31 (3H, d, J=6.4Hz)
3.94 (1H, dd, J=6.0,1.2Hz)
4.16-4.28 (1H, m)
4.94,5.03 (2H, ABq, J=13.8Hz)
5.30,5.52 (2H, ABq, J=14.0Hz)
5.80 (2H, s)
5.89 (1H, d, J=1.3Hz)
7.76-8.36 (4H, m)

EXAMPLE 66

Potassium (5R,6S)-3-(1-cyanomethyl-tetrazol-5-yl-thiomethylthio)-6-(1(R)-hydroxyethyl)-7-oxo-4-thia-1-azabicyclo[3.2.0]hept-2-ene-2-carboxylate

58mg of the above compound were obtained from 200mg of the corresponding 4-nitrobenzyl carboxylate (see Example 65) by a procedure analogous to that described in Example 2, using 37mg of potassium hydrogen carbonate.

$\nu_{max}$ (KBr) 1767cm$^{-1}$.

$\delta$ ($D_2O$)

1.28 (3H, d, J=6.4$H_z$)
3.91 (1H, dd, J=6.9,1.4$H_z$)
4.18-4.32 (1H, m)
4.92,5.04 (2H, ABq, J=13.9$H_z$)
4.52-4.98 (2H, m)
5.64 (1H, d, J=1.4$H_z$)

EXAMPLE 67

4-Nitrobenzyl (5R,6S)-6-(1(R)-hydroxyethyl)-3-(5-methyl-1,3,4-thiadiazol-2-yl-thio-methylthio)-7-oxo-4-thia-1-azabicyclo[3.2.0]hept-2-ene-2-carboxylate

87mg of the above compound were obtained by a procedure analogous to that described in Example 1 using 104mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 85mg of 2-iodomethylthio-5-methyl-1,3,4-thiadiazole and 52 $\mu l$ of diisopropylethylamine.

$\nu_{max}$ (KBr) 1779$cm^{-1}$

$\delta$ ($d^6$-DMSO)
1.18 (3H, d, J=6.2$H_z$)
2.71 (3H, s)
3.93 (1H, dd, J=5.6,1.3$H_z$)
3.98-4.08 (1H, m)
4.87,4.96 (2H, ABq, J=14.0$H_z$)
5.28,5.44 (2H, ABq, J=14.2$H_z$)
5.79 (1H, d, J=1.3$H_z$)
7.62-8.27 (4H, m)

EXAMPLE 68

Potassium (5R,6S)-6-(1(R)-hydroxyethyl)-3-(5-methyl-1,3,4-thiadiazol-2-yl-thio-methylthio)-7-oxo-4-thia-1-azabicyclo[3.2.0]hept-2-ene-2-carboxylate

49.2mg of the above compound were obtained from 84mg of the corresponding 4-nitrobenzyl carboxylate (see Example 67) by a procedure analogous to that described in Example 2, using 15.9mg of potassium hydrogen carbonate.

$\nu_{max}$ (KBr) 1770cm$^{-1}$.

$\delta$ ($D_2O$) 1.27 (3H, d, J=6.4Hz)
2.73 (3H, s)
3.87 (1H, dd, J=5.7,1.0Hz)
4.14-4.31 (1H, m)
4.69-5.03 (2H, m)
5.57 (1H, d, J=1.0Hz)

EXAMPLE 69

4-Nitrobenzyl (5R,6S)-3-(5-acetamido-1,3,4-thiadiazol-2-yl-thiomethylthio)-6-(1(R)-hydroxyethyl)-7-oxo-4-thia-1-azabicyclo[3.2.0]hept-2-ene-2-carboxylate

123mg of the above compound were obtained by a procedure analogous to that described in Example 1 using 157mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 142mg of 5-acetamido-2-iodomethylthio-1,3,4-thiadiazole and 79μl of diisopropylethylamine.

$\nu_{max}$ (KBr) 1810, 1793cm$^{-1}$

$\delta$ ($d^6$-DMSO)
1.15 (3H, d, J=6.4Hz)
2.17 (3H, s)
3.91 (1H, dd, J=5.8,1.0Hz)
3.96-4.06 (1H, m)
4.80,4.89 (2H, ABq, J=13.9Hz)
5.27,5.42 (2H, ABq, J=14.1Hz)
5.76 (1H, d, J=1.0Hz)
7.60-8.25 (4H, m)
12.70 (1H, s)

EXAMPLE 70

Potassium (5R,6S)-3-(5-acetamido-1,3,4-thiadiazol-2-yl-thiomethylthio)-6-(1(R)-hydroxyethyl)-7-oxo-4-thia-1-azabicyclo[3.2.0]hept-2-ene-2-carboxylate

71.4mg of the above compound were obtained from 123mg of the corresponding 4-nitrobenzyl carboxylate (see Example 69) by a procedure analogous to that described in Example 2, using 21.5mg of potassium hydrogen carbonate.

$\nu_{max}$ (KBr) 1769cm$^{-1}$

$\delta$ ($D_2O$)

1.26 (3H, d, J=6.4Hz)
2.24 (3H, s)
3.81 (1H, dd, J=5.9,1.3Hz)
4.13-4.29 (1H, m)
4.46,4.68 (2H, ABq, J=14.2Hz)
5.50 (1H, d, J=1.3Hz)
9.02 (1H, s)

EXAMPLE 71

4-Nitrobenzyl (5R,6S)-6-(1(R)-hydroxyethyl)-3-(5-carboxymethyl-4-methylthiazol-2-yl-thio-methylthio)-7-oxo-4-thia-1-azabicyclo[3.2.0]hept-2-ene-2-carboxylate

117.2mg of the above compound were obtained by a procedure analogous to that described in Example 1 using 100.2mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 95mg of 5-carboxymethyl-2-iodomethylthio-4-methylthiazole and 50.2 μl of diisopropylethylamine.

$\nu_{max}$ (KBr) 1781$cm^{-1}$

| | | |
|---|---|---|
| δ ($d^6$-DMSO) | 1.16 | (3H, d, J=6.5$H_z$) |
| | 2.24 | (3H, s) |
| | 3.79 | (2H, s) |
| | 3.91 | (1H, dd, J=5.9,1.3$H_z$) |
| | 3.95-4.07 | (1H, m) |
| | 4.74,4.83 | (2H, ABq, J=14.0$H_z$) |
| | 5.27,5.42 | (2H, ABq, J=13.7$H_z$) |
| | 5.77 | (1H, d, J=1.3$H_z$) |
| | 7.60-8.26 | (4H, m) |

EXAMPLE 72

(5R,6S)-6-(1(R)-Hydroxyethyl)-3-(5-carboxymethyl-4-methylthiazol-2-yl-thio-methylthio)-7-oxo-4-thia-1-azabicyclo[3.2.0]hept-2-ene-2-carboxylic acid monopotassium salt

77.8mg of the above compound were obtained from 112.4mg of the corresponding 4-nitrobenzyl carboxylate (see Example 71) by a procedure analogous to that described in Example 2, using 20.2mg of potassium hydrogen carbonate.

| | |
|---|---|
| $\delta$ ($D_2O$) | 1.26 (3H, d, J=6.5$H_z$) |
| | 2.28 (3H, s) |
| | 3.63 (2H, s) |
| | 3.81 (1H, dd, J=5.9,1.1$H_z$) |
| | 4.14-4.27 (1H, m) |
| | 4.39,4.63 (2H, ABq, J=14.3$H_z$) |
| | 5.47 (1H, d, J=1.1$H_z$) |

EXAMPLE 73

4-Nitrobenzyl (5R,6S)-6-(1(R)-hydroxyethyl)-3-(8-methyltetrazolo-[1,5-*b*]pyridazin-6-yl-thio-methylthio)-7-oxo-4-thia-1-azabicyclo[3.2.0]hept-2-ene-2-carboxylate

150mg of the above compound were obtained by a procedure analogous to that described in Example 1 using 200mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 177mg of 6-iodomethylthio-8-methyltetrazolo[1,5-*b*]pyridazine and 100$\mu$l of diisopropylethylamine.

$\nu_{max}$ (KBr) 1785cm$^{-1}$

$\delta$ ($d^6$-DMSO) 1.18 (3H, d, J=6.2Hz)
2.66 (3H, d, J=1.1Hz)
3.94 (1H, dd, J=5.7,1.3Hz)
3.97-4.08 (1H, m)
4.88,4.97 (2H, ABq, J=14.4Hz)
5.26,5.42 (2H, ABq, J=14.0Hz)
5.82 (1H, d, J=1.3Hz)
7.60-8.25 (4H, m)
7.70 (1H, q, J=1.2Hz)

EXAMPLE 74

Potassium (5R,6S)-6-(1(R)-hydroxyethyl)-3-(8-methyltetrazolo-[1,5-b]pyridazin-6-yl-thio-methylthio)-7-oxo-4-thia-1-azabicyclo[3.2.0]hept-2-ene-2-carboxylate

40.8mg of the above compound were obtained from 143mg of the corresponding 4-nitrobenzyl carboxylate (see Example 73) by a procedure analogous to that described in Example 2, using 26mg of potassium hydrogen carbonate.

$\nu_{max}$ (KBr) 1781cm$^{-1}$.

$\delta$ ($D_2O$) 1.28 (3H, d, J=6.4Hz)
2.65 (3H, d, J=0.9Hz)
3.91 (1H, dd, J=6.0,1.3Hz)
4.17-4.28 (1H, m)
4.62,4.61 (2H, ABq, J=13.7Hz)
5.70 (1H, d, J=1.3Hz)
7.40 (1H, q, J=0.9Hz)

EXAMPLE 75

4-Nitrobenzyl (5R,6S)-6-(1(R)-hydroxyethyl)-3-(7-methyltetrazolo-[1,5-b]pyridazin-6-yl-thio-methylthio)-7-oxo-4-thia-1-azabicyclo-[3.2.0]hept-2-ene-2-carboxylate

128mg of the above compound were obtained by a procedure analogous to that described in Example 1 using 111mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 98mg of 6-iodomethylthio-7-methyltetrazolo[1,5-b]pyridazine and 56 $\mu$l of diisopropylethylamine.

$\nu_{max}$ (Nujol) 1779cm$^{-1}$

$\delta$ ($d^6$-DMSO)

1.18 (3H, d, J=6.0Hz)

2.40 (3H, d, J=0.7Hz)

3.95 (1H, dd, J=5.6,1.0Hz)

3.99-4.10 (1H, m)

4.92,5.01 (2H, ABq, J=14.2Hz)

5.27,5.42 (2H, ABq, J=14.0Hz)

5.83 (1H, d, J=1.1Hz)

7.61-8.25 (4H, m)

8.57 (1H, q, J=0.7Hz)

EXAMPLE 76

Potassium (5R,6S)-6-(1(R)-hydroxyethyl)-3-(7-methyltetrazolo-[1,5-b]pyridazin-6-yl-thio-methylthio)-7-oxo-4-thia-1-azabicyclo-[3.2.0]hept-2-ene-2-carboxylate

53mg of the above compound were obtained from 114mg of the corresponding 4-nitrobenzyl carboxylate (see Example 75) by a procedure analogous to that described in Example 2, using 20.3mg of potassium hydrogen carbonate.

$\nu_{max}$ (KBr) 1779cm$^{-1}$.

$\delta$ ($D_2O$)
1.27 (3H, d, J=6.4Hz)
2.44 (3H, d, J=1.1Hz)
3.91 (1H, dd, J=6.0,1.3Hz)
4.15-4.30 (1H, m)
4.66-5.00 (2H, m)
5.69 (1H, d, J=1.3Hz)
8.16 (1H, q, J=1.1Hz)

EXAMPLE 77

4-Nitrobenzyl (5R,6S)-6-(1(R)-hydroxyethyl)-7-oxo-3-tetrazolo[1,5-b]-pyridazin-6-yl-thio-methylthio)-4-thia-1-azabicyclo[3.2.0]-hept-2-ene-2-carboxylate

86.6mg of the above compound were obtained by a procedure analogous to that described in Example 1 using 110.2mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 79mg of 6-iodomethylthio-tetrazolo[1,5-b]pyridazine and 55μl of diisopropylethylamine.

$\nu_{max}$ (KBr) $1780cm^{-1}$

$\delta$ ($CDCl_3$)
1.40 (3H, d, J=6.3Hz)
3.83 (1H, dd, J=6.6,1.5Hz)
4.23-4.36 (1H, m)
4.74,4.85 (2H, ABq, J=14.2Hz)
5.20,5.45 (2H, ABq, J=13.7Hz)
5.78 (1H, d, J=1.5Hz)
7.28 (1H, d, J=9.5Hz)
7.55-8.26 (4H, m)
8.23 (1H, d, J=9.5Hz)

EXAMPLE 78

Potassium (5R,6S)-6-(1(R)-hydroxyethyl)-7-oxo-3-tetrazolo[1,5-b]-pyridazin-6-yl-thio-methylthio)-4-thia-1-azabicyclo[3.2.0]-hept-2-ene-2-carboxylate

46.4mg of the above compound were obtained from 81.7mg of the corresponding 4-nitrobenzyl carboxylate (see Example 77) by a procedure analogous to that described in Example 2, using 15.9mg of potassium hydrogen carbonate.

$\nu_{max}$ (KBr) 1770cm$^{-1}$

$\delta$ ($D_2O$) 1.28 (3H, d, J=6.4Hz)
3.93 (1H, dd, J=6.0,1.5Hz)
4.15-4.31 (1H, m)
4.55-4.87 (2H, m)
5.70 (1H, d, J=1.5Hz)
7.63 (1H, d, J=9.5Hz)
8.35 (1H, d, J=9.5Hz)

EXAMPLE 79

4-Nitrobenzyl (5R,6S)-3-(N,N-diethylcarbamoyl)-4-methyl-1,2,4-triazol-3-yl-thio-methylthio)-(1(R)-hydroxyethyl)-7-oxo-4-thia-1-azabicyclo[3.2.0]hept-2-ene-2-carboxylate

110.1mg of the above compound were obtained by a procedure analogous to that described in Example 1 using 100.9mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 102.7mg of 5-(N,N-diethylcarbamoyl)-2-iodomethylthio-1-methyl-1,3,4-triazole and 50.5 μl of diisopropylethylamine.

$\nu_{max}$ (film) 1791cm$^{-1}$

$\delta$ ($CDCl_3$) 1.25 (3H, t, J=7.1Hz)
1.32 (3H, t, J=7.1Hz)
1.38 (3H, d, J=6.3Hz)
3.55 (2H, q, J=7.1Hz)
3.73 (3H, s)
3.76 (2H, q, J=7.1Hz)
3.81 (1H, dd, J=6.6,1.5Hz)
4.20-4.36 (1H, m)
4.71,4.84 (2H, ABq, J=13.7Hz)
5.21,5.46 (2H, ABq, J=13.7Hz)
5.74 (1H, d, J=1.5Hz)
7.58-8.28 (4H, m)

EXAMPLE 80

Potassium (5R,6S)-3-(5-(N,N-diethylcarbamoyl)-4-methyl-1,2,4-triazol-3-yl-thio-methylthio)-6-(1(R)-hydroxyethyl)-7-oxo-4-thia-1-azabicyclo[3.2.0]hept-2-ene-2-carboxylate

58.4mg of the above compound were obtained from 98.3mg of the corresponding 4-nitrobenzyl carboxylate (see Example 79) by a procedure analogous to that described in Example 2, using 16.2mg of potassium hydrogen carbonate.

$\nu_{max}$ (KBr) 1770cm$^{-1}$

$\delta$ ($D_2O$) 1.17 (3H, t, J=7.1Hz)
1.23 (3H, t, J=7.2Hz)
1.26 (3H, d, J=6.3Hz)
3.40 (2H, q, J=7.1Hz)
3.57 (2H, q, J=7.1Hz)
3.69 (3H, s)
3.86 (1H, dd, J=5.9,1.4Hz)
4.12-4.27 (1H, m)
4.45,4.62 (2H, ABq, J=14.5Hz)
5.55 (1H, d, J=1.4Hz)

EXAMPLE 81

4-Nitrobenzyl (5R,6S)-6-(1(R)-hydroxyethyl)-3-(3-methyl-1,2,4-triazolyl[3,4-b]-1,2,4,5-tetrazin-5-yl-thio-methylthio)-7-oxo-4-thia-1-azabicyclo[3.2.0]hept-2-ene-2-carboxylate

206mg of the above compound were obtained by a procedure analogous to that described in Example 1 using 200mg of 4-nitrobenzyl (5R,6S)-6-(1(R)-hydroxyethyl)-7-oxo-4-thia-3-thioxo-1-azabicyclo[3.2.0]hept-2-ene-2-carboxylate, 161mg of 3-methyl-5-iodothiomethyl-1,2,4-triazolyl[3,4-b]-1,2,4,5-tetrazine, and 109 μl of diisopropylethylamine.

$\nu_{max}$ (KBr) 1780$cm^{-1}$

$\delta$ ($CD_3CN$) 1.24 (3H, d, J=6.4$H_z$)
3.00 (3H, s)
3.86 (1H, dd, J=5.2,1.5$H_z$)
4.07-4.20 (1H, m)
4.83,4.93 (2H, ABq, J=14.0$H_z$)
5.20,5.41 (2H, ABq, J=13.9$H_z$)
5.75 (1H, d, J=1.5$H_z$)

EXAMPLE 82

Potassium (5R,6S)-6-(1(R)-hydroxyethyl)-3-(3-methyl-1,2,4-triazolyl[3,4-b]-1,2,4,5-tetrazin-5-yl-thio-methylthio)-7-oxo-4-thia-1-azabicyclo[3.2.0]hept-2-ene-2-carboxylate.

118mg of the above compound were obtained from 191.3mg of the corresponding 4-nitrobenzyl carboxylate (see Example 81) by a procedure analogous to that described in Example 2, using 34mg of potassium hydrogen carbonate.

$\nu_{max}$ (KBr) 1771cm$^{-1}$

$\delta$ ($D_2O$) 1.25 (3H, d, J=6.3Hz)
3.02 (3H, s)
3.92 (1H, dd, J=6.9,1.4Hz)
4.25-4.35 (1H, m)
4.86,5.00 (2H, ABq, J=13.9Hz)
5.61 (1H, d, J=1.4 Hz)

EXAMPLE 83

4-Nitrobenzyl (5R,6S)-6-(1(R)-acetoxyethyl)-3-(1-methyltetrazol-5-yl-thio-methylthio)-7-oxo-4-thia-1-azabicyclo[3.2.0] hept-2-ene-2-carboxylate

To a stirred solution of 87.2mg of 4-nitrobenzyl (5R,6S)-6-(1(R)-hydroxyethyl)-3-(1-methyltetrazol-5-yl-thio-methylthio)-7-oxo-4-thia-1-azabicyclo[3.2.0]hept-2-ene-2-carboxylate (see Example 1) in 2.5ml tetrahydrofuran was added 80.7μl of acetic anhydride and 2.1mg of 4-N,N-dimethylaminopyridine. After 10 minutes the reaction mixture was diluted with ethyl acetate, washed with saturated sodium bicarbonate solution, with water and the organic portion separated and dried over magnesium sulphate. Evaporation of the solvent gave a crude oil which was purified via silica gel chromatography eluting with ethyl acetate/hexane mixtures. The title compound was isolated as a homogeneous oil, 104mg.

| | |
|---|---|
| δ ($CDCl_3$) | 1.43 (3H, d, J=6.4$H_z$) |
| | 2.08 (3H, s) |
| | 3.93 (1H, dd, J=7.6,1.6$H_z$) |
| | 3.96 (3H, s) |
| | 4.76,4.86 (2H, ABq,J=13.8$H_z$) |
| | 5.20-5.30 (1H, m) |
| | 5.25,5.44 (2H, ABq,J=13.6$H_z$) |
| | 5.71 (1H, d, J=1.6$H_z$) |
| | 7.56-8.27 (4H, m) |

EXAMPLE 84

Potassium (5R,6S)-6-((1R)-acetoxyethyl)-3-(1-methyltetrazol-5-yl-thio-methylthio)-7-oxo-4-thia-1-azabicyclo[3.2.0]hept-2-ene-2-carboxylate

45mg of the above compound were obtained from 100mg of the corresponding 4-nitrobenzyl carboxylate (see Example 83) by a procedure analogous to that described in Example 2, using 18.1mg of potassium hydrogen carbonate.

| | |
|---|---|
| δ ($D_2O$) | 1.27 (3H, d, J=6.3Hz) |
| | 2.02 (3H, s) |
| | 3.94-3.99 (4H, m) |
| | 3.96 (1H, dd, J=6.0,1.6Hz) |
| | 3.97 (3H, s) |
| | 4.57,4.70 (2H, ABq,J=13.5Hz) |
| | 5.03-5.15 (1H, m) |
| | 5.59 (1H, d, J=1.6Hz) |

EXAMPLE 85

4-Nitrobenzyl (5R,6S)-6-(1(R)-phenylacetoxyethyl)-3-(1-methyltetrazol-5-yl-thio-methylthio)-7-oxo-4-thia-1-azabicyclo[3.2.0]hept-2-ene-2-carboxylate

92mg of the above compound were obtained by a procedure analogous to that described in Example 83 using 90mg of 4-nitrobenzyl (5R,6S)-6-(1(R)hydroxyethyl)-3-(1-methyltetrazol-5-yl-thio-methylthio)-7-oxo-4-thia-1-azabicyclo[3.2.0]hept-2-ene-2-carboxylate (see Example 1), 224mg of phenylacetic anhydride, and 4.3mg of 4-N,N-dimethylaminopyridine in 2ml of N,N-dimetnylformamide as solvent.

$\nu$ max

| | |
|---|---|
| $\delta$ ($CDCl_3$) | 1.42 (3H, d, J=6.4Hz) |
| | 3.62 (2H, s) |
| | 3.88 (1H, dd, J=7.9,1.5Hz) |
| | 3.96 (3H, s) |
| | 4.75,4.84 (2H, ABq, J=14Hz) |
| | 5.20,5.41 (2H, ABq, J=13.6Hz) |
| | 5.18-5.32 (1H, m) |
| | 5.55 (1H, d, J=1.5Hz) |
| | 7.19-7.32 (5H, m) |
| | 7.57-8.27 (4H, m) |

EXAMPLE 86

Potassium (5R,6S)-6(1(R)-phenylacetoxyethyl)-3-(1-methyltetrazol-5-yl-thio-methylthio)-7-oxo-4-thia-1-azabicyclo[3.2.0]hept-2-ene-2-carboxylate

15mg of the above compound were obtained from 77mg of the corresponding 4-nitrobenzyl carboxylate (see Example 85) by a procedure analogous to that described in Example 2, using 12.3mg of potassium hydrogen carbonate.

| | |
|---|---|
| $\delta$ ($d^6$DMSO) | 1.27 (3H, d, J=6.4$H_z$) |
| | 3.67 (2H, s) |
| | 3.95 (1H, dd, J=6.6,1.5$H_z$) |
| | 3.97 (3H,s) |
| | 4.70,4.57 (2H, ABq, J=13.6$H_z$) |
| | 5.07-5.18 (1H, m) |
| | 5.53 (1H, d, J=1.5$H_z$) |
| | 7.18-7.36 (5H, m) |

EXAMPLE 87

4-Nitrobenzyl (5R,6S)-6-(1(R)-{1,3-dithiolan-2-yl}carbonyloxyethyl)-3-(1-methyltetrazol-5-yl-thio-methylthio)-7-oxo-4-thia-1-azabicyclo-[3.2.0]hept-2-ene-2-carboxylate

142mg of the above compound were obtained by a procedure analogous to that described in Example 83 using 100mg of 4-nitrobenzyl-(5R,6S)-6(1(R )-hydroxyethyl)-3-(1-methyltetrazol-5-yl-thio-methylthio)-7-oxo-4-thia-1-azabicyclo[3.2.0]hept-2-ene-2-carboxylate, (see Example 1), 304mg of bis (2-{1,3-dithiolan-2-yl}-ethanoic) anhydride, 5mg of 4-N,N-dimethylaminopyndine in 3ml of tetrahydrofuran as solvent.

$\delta$ ($CDCl_3$) 1.49 (3H, d, J=6.4$H_z$)
1.88-2.19 (2H, m)
2.50-2.62 (2H, m)
3.23-3.46 (2H, m)
3.96 (3H, s)
3.99 (1H, dd, J=1.5,6.0$H_z$)
4.12 (1H, s)
4.75,4.86 (2H, ABq, J=14$H_z$)
5.22,5.44 (2H, ABq, J=13.6$H_z$)
5.26-5.38 (1H, m)
5.73 (1H, d, J=1.5$H_z$)
7.58-8.28 (4H, m)

EXAMPLE 88

Potassium (5R,6S)-6-(1(R)-{1,3 dithiolan-2-yl}carbonyloxyethyl)-3-(1-methyltetrazol-5-yl-thio-methylthio)-7-oxo-4-thia-1-azabicyclo [3.2.0]hept-2-ene-2-carboxylate

27mg of the above compound were obtained from 124mg of the corresponding 4-nitrobenzyl carboxylate (see Example 87) by a procedure analogous to that described in Example 2, using 18.9mg of potassium hydrogen carbonate.

δ ($D_2O$)

1.42 (3H, d, J=6.4$H_z$)
1.69-2.16 (2H, m)
2.68-2.80 (2H, m)
3.13-3.26 (2H, m)
3.96-4.09 (2H, m)
4.07 (3H, s)
4.18 (1H, dd, J=5.8,1.5$H_z$)
4.47-4.92 (2H, m)
5.65 (1H, d, J=1.4$H_z$)

EXAMPLE 89

4-Nitrobenzyl (5R,6S)-6-(1(R)-acetoxyethyl)-7-oxo-3-(tetrazolo[1,5-b]-pyridazin-6-yl-thio-methylthio)-7-oxo-4-thia-1-azabicyclo[3.2.0]-hept-2-ene-2-carboxylate

98.5mg of the above compound were obtained by a procedure analogous to that described in Example 83, using 89.1mg of 4-nitrobenzyl (5R,6S)-6-(1(R)-hydroxyethyl)-7-oxo-3-(tetrazolo[1,5-b]-pyridazin-6-yl-thio-methylthio)-4-thia-1-azabicyclo[3.2.0]-hept-2-ene-2-carboxylate (see Example 77) 83 μl of acetic anhydride, 1.9mg of 4-N,N-dimethylaminopyridine in 8ml of tetrahydrofuran as solvent.

$\nu_{max}$ (KBr) 1791,1739$cm^{-1}$

$\delta$ ($CDCl_3$)
1.43 (3H, d, J=6.4$H_z$)
2.08 (3H, s)
3.98 (1H, dd, J=7.4,1.5$H_z$)
4.74,4.85 (2H, ABq, J=14.2$H_z$)
5.21-5.36 (1H, m)
5.21,5.43 (2H, ABq, J=13.8$H_z$)
5.77 (1H, d, J=1.5$H_z$)
7.35,8.27 (2H, ABq, J=9.5$H_z$)
7.53-8.26 (4H, m)

EXAMPLE 90

Potassium (5R,6S)-6-(1(R)-acetoxyethyl)-7-oxo-3-(tetrazolo[1,5-b]-pyridazin-6-yl-thio-methylthio)-4-thia-1-azabicyclo[3.2.0]-hept-2-ene-2-carboxylate

33.8mg of the above compound were obtained from 99.4mg of the corresponding 4-nitrobenzyl carboxylate (see Example 89) by a procedure analogous to that described in Example 2, using 16.8 mg of potassium hydrogen carbonate.

$\nu_{max}$ 1775,1734cm$^{-1}$

$\delta$ ($D_2O$)

1.34 (3H, d, J=6.4Hz)
2.09 (3H, s)
4.14 (1H, dd, J=5.0,1.4Hz)
4.63-5.00 (2H, m)
5.15-5.30 (1H, m)
5.76 (1H, d, J=1.4Hz)
7.63,8.35 (2H, ABq, J=9.6Hz)

EXAMPLE 91

4-Nitrobenzyl (5R,6S)-6-(1(R)-hexanoyloxyethyl)-7-oxo-3-(tetrazolo-[1,5-b]pyridazin-6-yl-thio-methylthio)-4-thia-1-azabicyclo-[3.2.0]hept-2-ene-2-carboxylate

105.8mg of the above compound were obtained by a procedure analogous to that described in Example 83, using 92mg of 4-nitrobenzyl (5R,6S)-6-(1(R)-hydroxyethyl)-7-oxo-3-tetrazolo[1,5-b]-pyridazin-6-yl-thio-methylthio)-4-thia-1-azabicyclo 3.2.0 -hept-2-ene-2-carboxylate (see Example 77), 369μl of hexanoic anhydride, 10.2mg of 4-N,N-dimethylaminopyndine in 5ml of tetrahydrofuran as solvent.

| | |
|---|---|
| δ ($CDCl_3$) | 0.87 (3H, t, J=6.8Hz) |
| | 1.15-1.38 (4H, m) |
| | 1.43 (3H, d, J=6.4Hz) |
| | 1.53-1.72 (2H, m) |
| | 2.31 (2H, t, J=7.5Hz) |
| | 3.97 (1H, dd, J=7.3,1.5Hz) |
| | 4.74,4.84 (2H, ABq,J-13.8Hz) |
| | 5.22-5.38 (1H, m) |
| | 5.76 (1H, d, J=1.5Hz) |
| | 7.34,8.26 (2H, ABq,J=9.4Hz) |
| | 7.52-8.27 (4H, m) |

EXAMPLE 92

Potassium (5R,6S)-6-(1(R)-hexanoyloxyethyl)-7-oxo-3-(tetrazolo[1,5-b]-pyridazin-6-yl-thio-methylthio)-4-thia-1-azabicyclo[3.2.0]-hept-2-ene-2-carboxylate

26mg of the above compound were obtained from 105mg of the corresponding 4-nitrobenzyl carboxylate (see Example 91) by a procedure analogous to that described in Example 2, using 16.2mg of potassium hydrogen carbonate.

$\nu_{max}$ (KBr) 1774,1734cm$^{-1}$

$\delta$ ($D_2O$)
0.70-0.90 (3H, m)
1.15-1.33 (4H, m)
1.35 (3H, d, J=6.4Hz)
1.48-1.68 (2H, m)
2.39 (2H, t, J=7.3Hz)
4.14 (1H, dd, J=5.4,1.4Hz)
4.57-5.07 (2H, m)
5.20-5.35 (1H, m)
5.76 (1H, d, J=1.4Hz)
7.62,8.37 (2H, ABq, J=9.5Hz)

EXAMPLE 93

4-Nitrobenzyl (5R,6S)-6-(1(R)-decanoyloxyethyl)-7-oxo-3-(tetrazolo-[1,5-b]pyridazin-6-yl-thio-methylthio)-4-thia-1-azabicyclo-[3.2.0]hept-2-ene-2-carboxylate

110.3mg of the above compound were obtained by a procedure analogous to that described in Example 83, using 89.9mg of 4-nitrobenzyl (5R,6S)-6-(1(R)-hydroxyethyl)-7-oxo-3-tetrazolo[1,5-b]-pyridazin-6-yl-thio-methylthio)-4-thia-1-azabicyclo[3.2.0]-hept-2-ene-2-carboxylate (see Example 77) 586.3mg of decanoic anhydride, 10mg of 4-N,N-dimethylaminopyridine in 8ml of tetrahydrofuran as solvent.

| | |
|---|---|
| δ ($CDCl_3$) | 0.87 (3H, t, J=6.6Hz) |
| | 1.11-1.40 (9H, m) |
| | 1.42 (3H, d, J=6.5Hz) |
| | 1.51-1.72 (5H, m) |
| | 2.30 (2H, t, J=7.5Hz) |
| | 3.94 (1H, dd, J=7.7,1.5Hz) |
| | 4.73,4.84 (2H, ABq, J=14.1Hz) |
| | 5.21,5.43 (2H, ABq, J=13.7Hz) |
| | 5.22-5.37 (1H, m) |
| | 5.74 (1H, d, J=1.5Hz) |
| | 7.28,8.23 (2H, ABq, J=9.5Hz) |
| | 7.52-8.28 (4H, m) |

EXAMPLE 94

Potassium (5R,6S)-6-(1(R)-decanoyloxyethyl)-7-oxo-3-(tetrazolo[1,5-b]-pyridazin-6-yl-thio-methylthio)-4-thia-1-azabicyclo[3.2.0]-hept-2-ene-2-carboxylate

12mg of the above compound were obtained from 108.3mg of the corresponding 4-nitrobenzyl carboxylate (see Example 93) by a procedure analogous to that described in Example 2, using 15.4mg of potassium hydrogen carbonate.

$\delta_{max}$ (KBr) 1775,1738cm$^{-1}$

($D_2O$)
0.80 (3H, t, J=6.8$H_z$)
1.14-1.36 (9H, m)
1.38 (3H, d, J=6.4$H_z$)
1.45-1.70 (5H, m)
2.38 (2H, d, J=7.3$H_z$)
3.99 (1H, dd, J=5.5, 1.3$H_z$)
4.75,4.85 (2H, ABq, J=14.0$H_z$)
5.21-5.32 (1H, m)
5.75 (1H, d, J=1.3$H_z$)
7.47,8.39 (2H, ABq, J=9.4$H_z$)

EXAMPLE 95

4-Nitrobenzyl (5R,6S)-6-(1(R)-ethoxyacetoxyethyl)-7-oxo-3-(tetrazolo [1,5-b]pyridazin-6-yl-thio-methylthio)-4-thia-1-azabicyclo-[3.2.0]hept-2-ene-2-carboxylate

67.1mg of the above compound were obtained by a procedure analogous to that described in Example 83, using 110mg of 4-nitrobenzyl (5R,6S)-6-(1(R)-hydroxyethyl)-7-oxo-3-tetrazolo[1,5-b]-pyridazin-6-yl-thio-methylthio)-4-thia-1-azabicyclo[3.2.0]-hept-2-ene-2-carboxylate (see Example 77), 380mg of bis(ethoxy-ethanoic)anhydride, 10mg of 4-N,N-dimethylaminopyridine in 10ml of tetrahydrofuran as solvent.

| | |
|---|---|
| δ ($CDCl_3$) | 1.24 (3H, t, J=7.0$H_z$) |
| | 1.47 (3H, d, J=6.4$H_z$) |
| | 3.50-3.68 (2H, m) |
| | 3.98 (1H, dd, J=1.5,7.7$H_z$) |
| | 4.08 (2H, s) |
| | 4.73,4.84 (2H, ABq,J=14.1$H_z$) |
| | 5.21,5.43 (2H, ABq,J=13.7$H_z$) |
| | 5.30-5.45 (1H, m) |
| | 5.77 (1H, d, J=1.5$H_z$) |
| | 7.29,8.23 (2H,ABq,J=9.5$H_z$) |
| | 7.52-8.28 (4H, m) |

EXAMPLE 96

Potassium (5R,6S)-6-(1(R)-ethoxyacetoxyethyl)-7-oxo-3-(tetrazolo[1,5-b]-pyridazin-6-yl-thio-methylthio)-4-thia-1-azabicyclo[3.2.0]-hept-2-ene-2-carboxylate

10mg of the above compound were obtained from 65mg of the corresponding 4-nitrobenzyl carboxylate (see Example 95) by a procedure analogous to that described in Example 2, using 7.5mg of potassium hydrogen carbonate.

$\nu_{max}$ (KBr) 1763cm$^{-1}$

$\delta$ ($D_2O$)
1.12-1.26 (3H, m)
1.38 (3H, d, J=6.5Hz)
3.49-3.71 (2H, m)
3.88 (2H, s)
4.22 (1H, dd, J=4.2,1.5Hz)
4.70-4.90 (2H, m)
5.32-5.47 (1H, m)
5.78 (1H, d, J=1.5Hz)
7.63,8.35 (2H, ABq, J= 9.5Hz)

The following Table gives further Examples of compounds of the invention, which are prepared analogously to the compounds described in the Examples above.

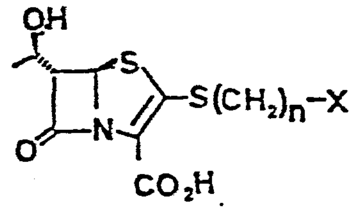

| n | X | n | X |
|---|---|---|---|
| 1 | 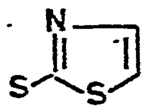 | 1 | 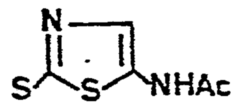 |
| 2 | 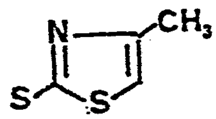 | 2 | 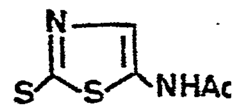 |
| 1 | 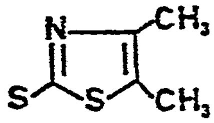 | 1 | 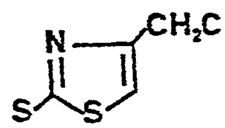 |
| 1 | 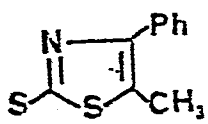 | 1 | 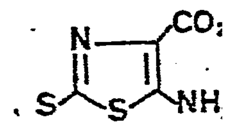 |
| 1 | 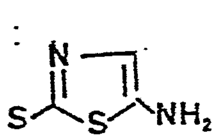 | 1 | 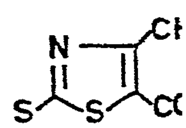 |
| 2 | 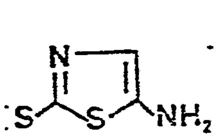 | 1 | 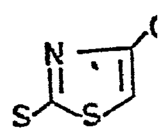 |

| | X | n | X |
|---|---|---|---|
| | 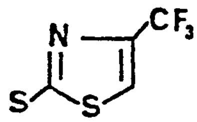 | 1 | 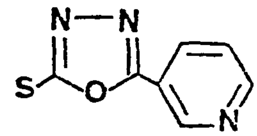 |
| | 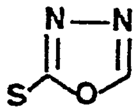 | 2 | 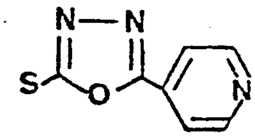 |
| | 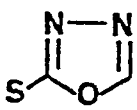 | 1 | 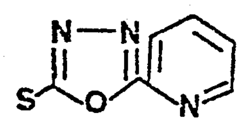 |
| | 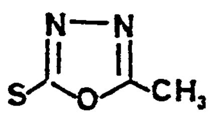 | 1 | 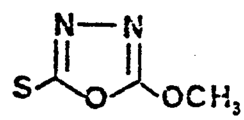 |
| | 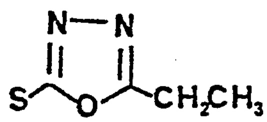 | 2 | 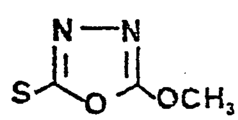 |
| | 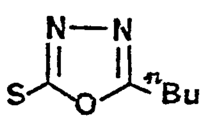 | 1 | 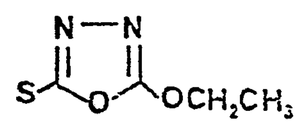 |
| | 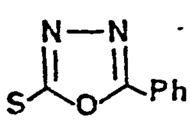 | 2 | 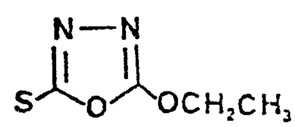 |

| n | X | n | X |
|---|---|---|---|
| 1 | 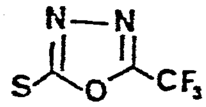 | 1 | 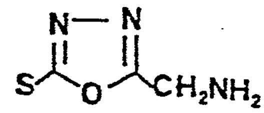 |
| 1 | 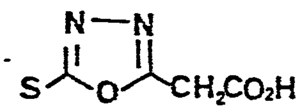 | 1 | 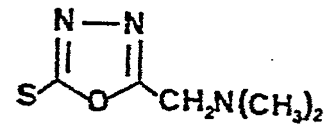 |
| 1 | 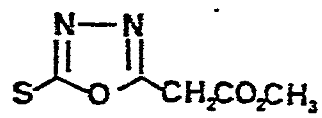 | 1 | 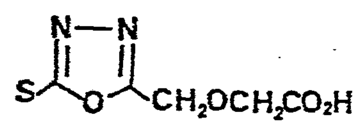 |
| 1 | 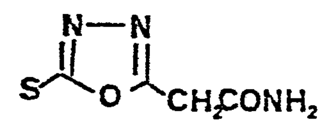 | 2 | 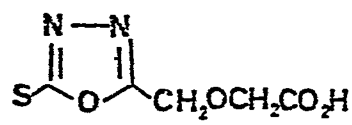 |
| 1 | 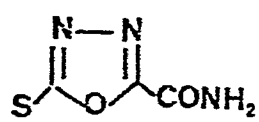 | 1 | 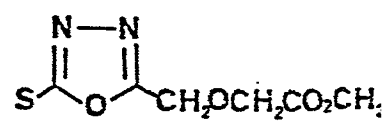 |
| 1 | 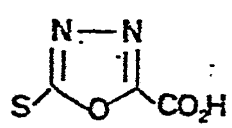 | 1 | 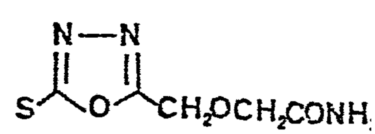 |
| 2 | 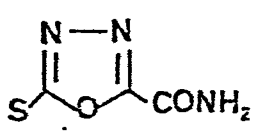 | 2 | 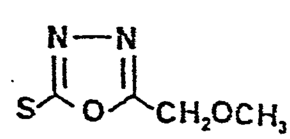 |

| n | X | n | X |
|---|---|---|---|
| 1 | 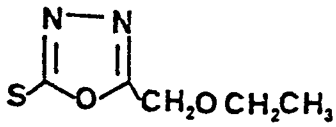 | 1 | 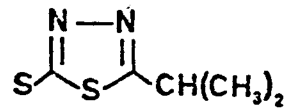 |
| 1 | 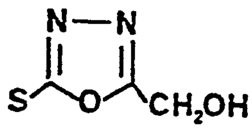 | 2 | 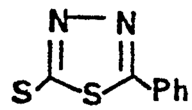 |
| 2 | 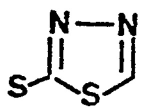 | 1 | 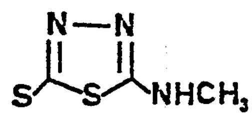 |
| 2 | 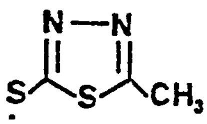 | 2 | 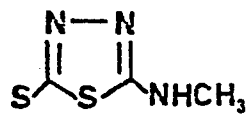 |
| 1 | 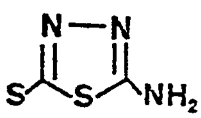 | 1 | 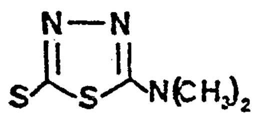 |
| 1 | 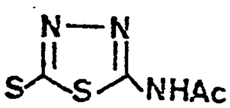 | 1 | 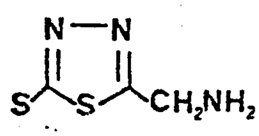 |
| 2 | 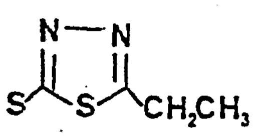 | 1 | 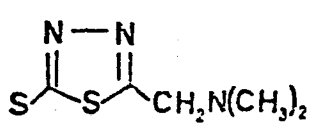 |

| n | X | n | X |
|---|---|---|---|
| 2 | 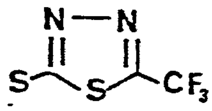 | 1 | 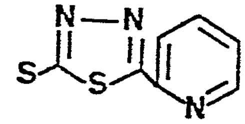 |
| 1 | 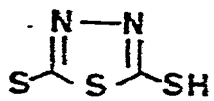 | | 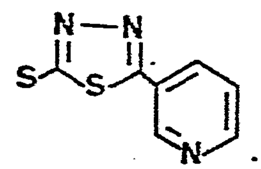 |
| 1 | 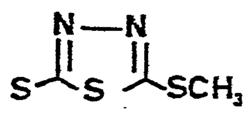 | 1 | 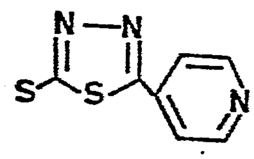 |
| 1 | 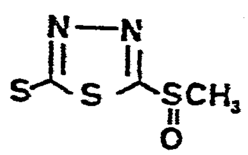 | 1 | 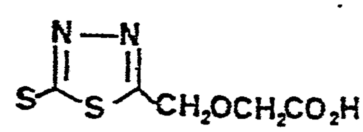 |
| 2 | 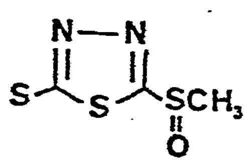 | 1 | 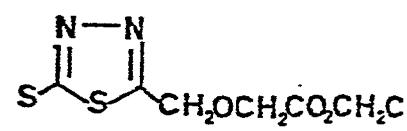 |
| 1 | 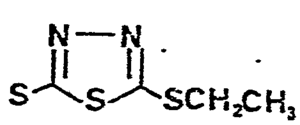 | 1 | 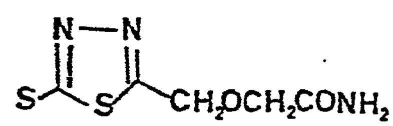 |
| 1 | 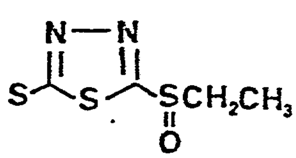 | 1 | 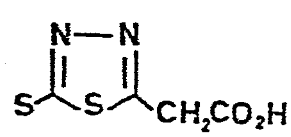 |

| n | X | n | X |
|---|---|---|---|
| 1 | 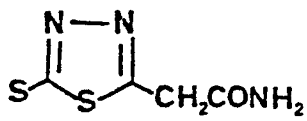 | 1 | 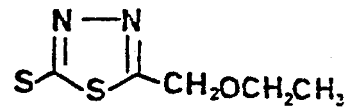 |
| 1 | 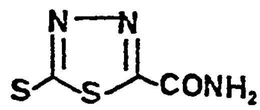 | 1 | 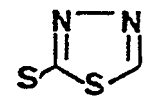 |
| 2 | 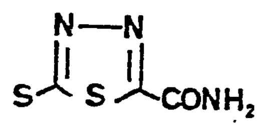 | 1 | 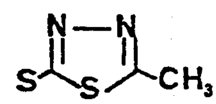 |
| 1 | 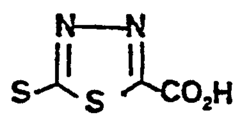 | 1 | 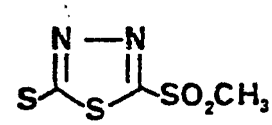 |
| 1 | 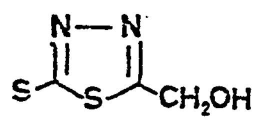 | 1 | 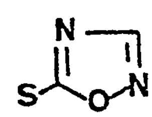 |
| 2 | 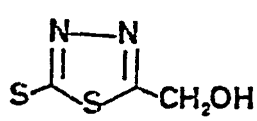 | 1 | 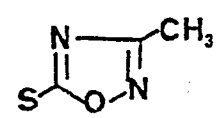 |
| 1 | 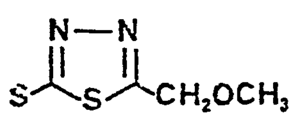 | 1 | 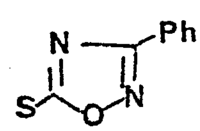 |

| n | X | n | X |
|---|---|---|---|
| 1 | 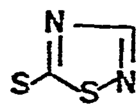 | 1 | 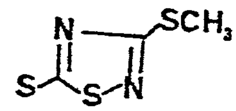 |
| 1 | 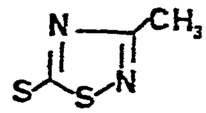 | 1 | 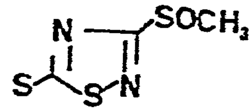 |
| 1 | 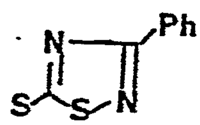 | | |

| n | X | n | X |
|---|---|---|---|
| 2 | 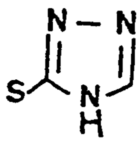 | 1 | 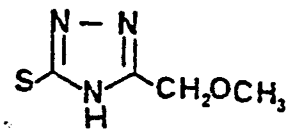 |
| 1 | 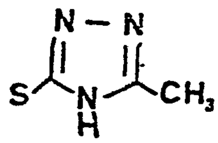 | 1 | 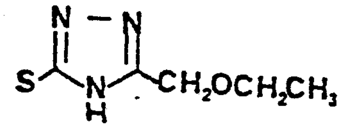 |
| 1 | 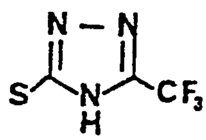 | 1 | 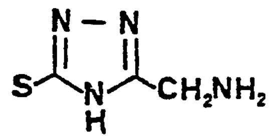 |
| 1 | 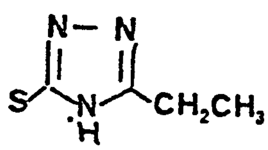 | 1 | 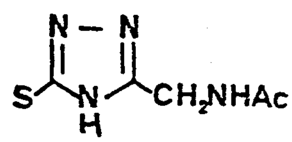 |
| 1 | 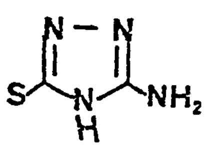 | 1 | 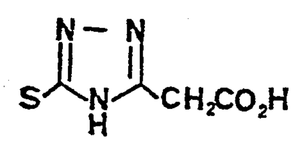 |
| 1 | 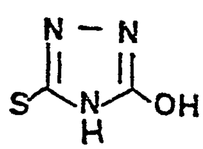 | 1 | 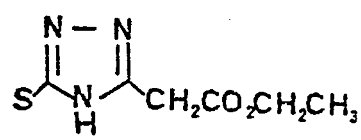 |
| 1 | 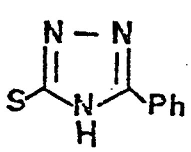 | 1 | 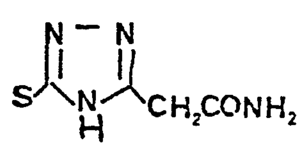 |

| n | X | n | X |
|---|---|---|---|
| 1 | 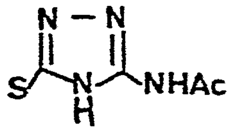 | 1 | 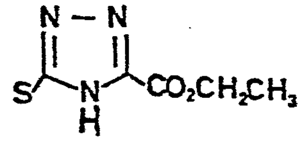 |
| 1 | 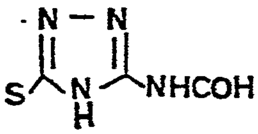 | 1 | 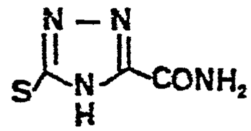 |
| 1 | 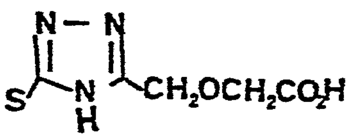 | | |

| n | X |
|---|---|
| 1 | 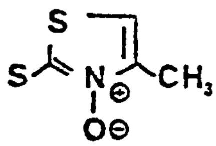 |
| 2 | 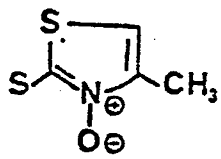 |
| 1 | 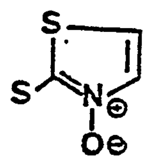 |
| 1 | 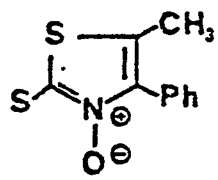 |
| 1 | 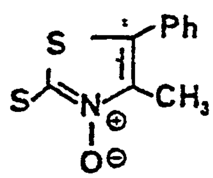 |
| 1 | 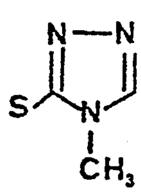 |
| 1 | 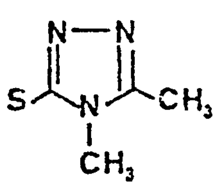 |
| 1 | 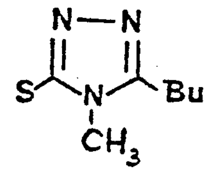 |
| 2 | 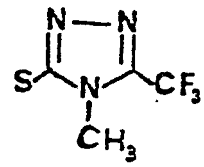 |
| 1 | 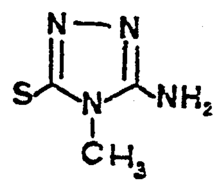 |
| 1 | 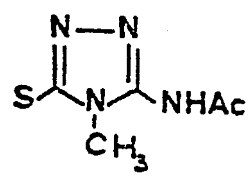 |
| 1 | 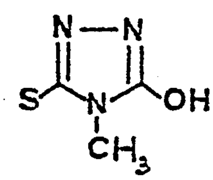 |
| 1 | 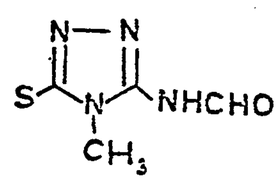 |
| 1 | 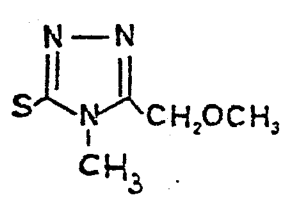 |

| n | X | n | X |
|---|---|---|---|
| 1 | 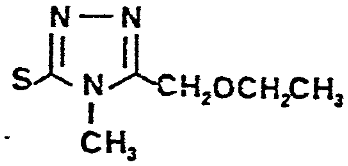 | 1 | 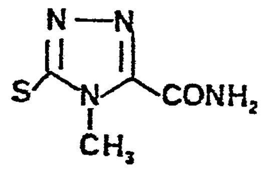 |
| 1 | 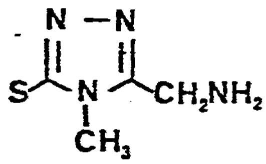 | 1 | 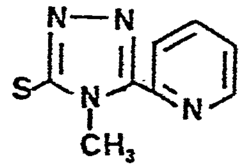 |
| 1 | 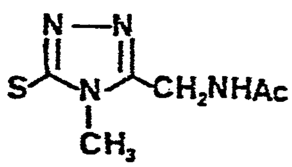 | 1 | 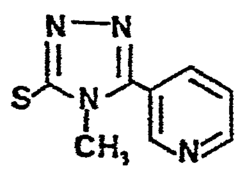 |
| 1 | 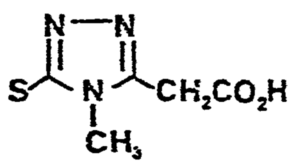 | 1 | 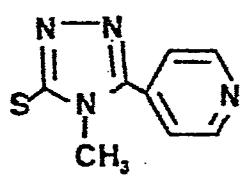 |
| 1 | 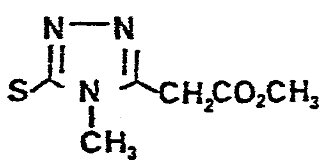 | 1 | 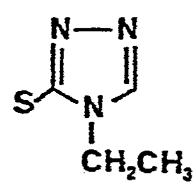 |
| 1 | 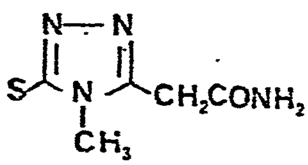 | 1 | 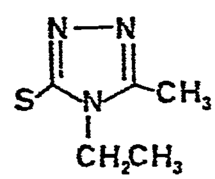 |
| 1 | 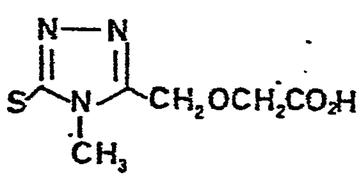 | 1 | 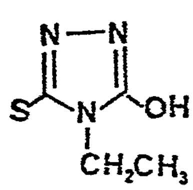 |

| n | X | n | X |
|---|---|---|---|
| 1 | 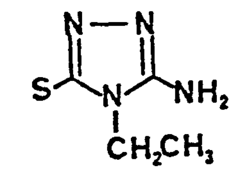 | 1 | 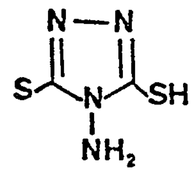 |
| 1 | 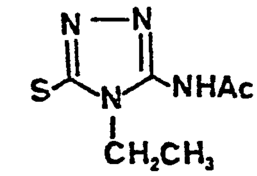 | 1 | 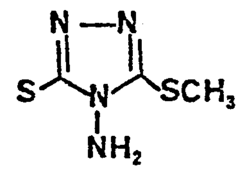 |
| 1 | 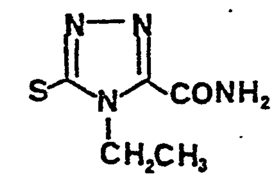 | 1 | 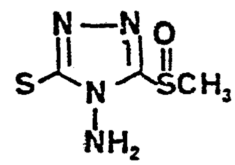 |
| 1 | 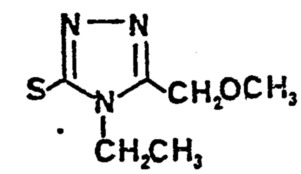 | 1 | 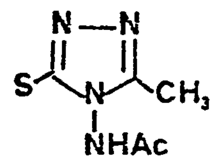 |
| 1 | 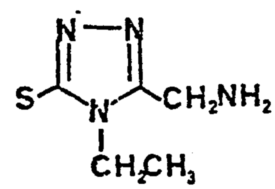 | 1 | 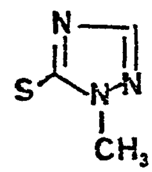 |
| 1 | 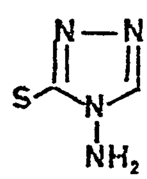 | 1 | 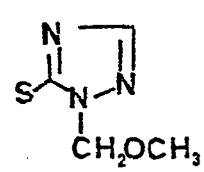 |
| 1 | 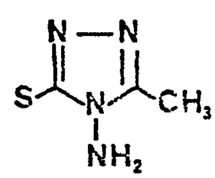 | 1 | 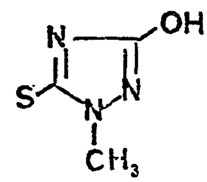 |

| n | X | n | X |
|---|---|---|---|
| 1 | 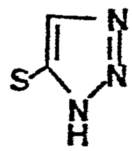 | 2 | 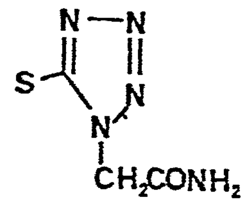 |
| 1 | 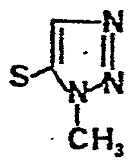 | 2 | 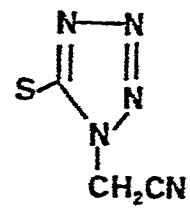 |
| 1 | 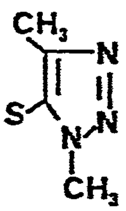 | 1 | 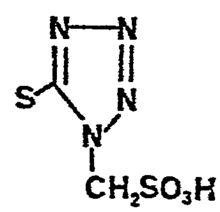 |
| 2 | 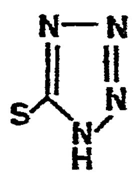 | 1 | 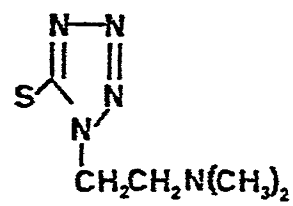 |
| 1 | 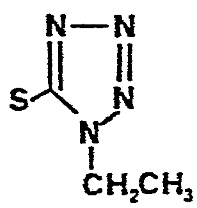 | 1 | 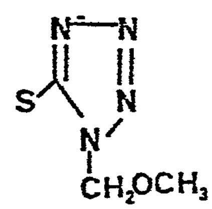 |
| 1 | 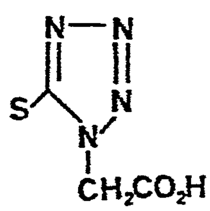 | 1 | 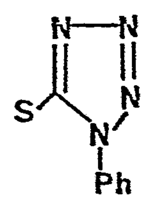 |

| n | X | n | X |
|---|---|---|---|
| 2 | 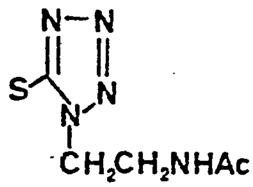 | 1 | 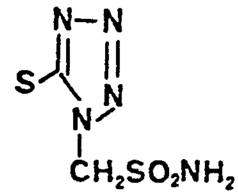 |
| 1 | 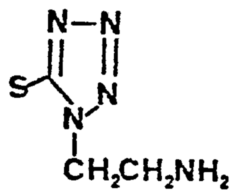 | 2 | 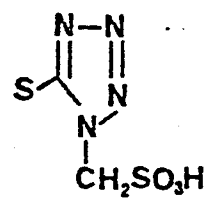 |
| 1 | 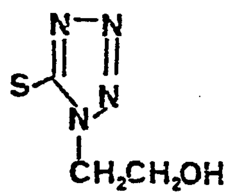 | | |
| 1 | 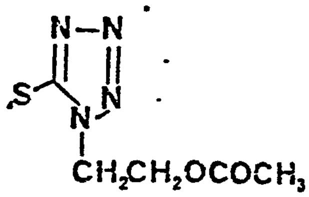 | | |
| 1 | 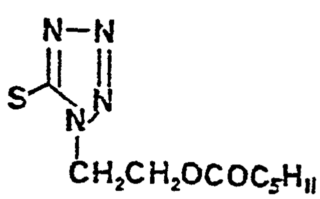 | | |
| 1 | 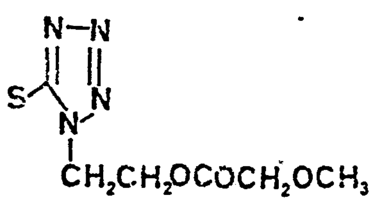 | 1 | 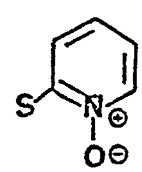 |

| n | X | n | X |
|---|---|---|---|
| 1 | 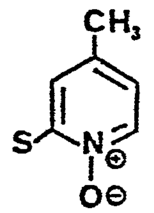 | 1 | 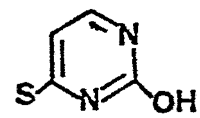 |
| 1 | 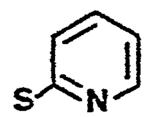 | 1 | 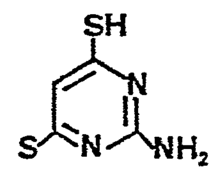 |
| 1 | 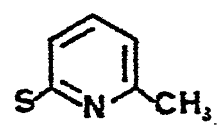 | 1 | 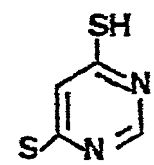 |
| 1 | 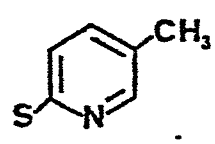 | 1 | 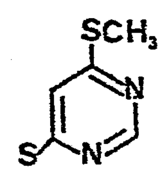 |
| 1 | 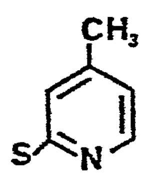 | 1 | 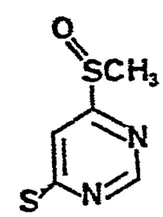 |
| 1 | 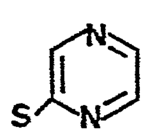 | 1 | 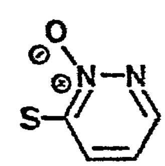 |
| 1 | 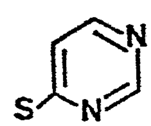 | 1 | 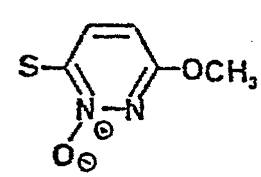 |

| n | X | n | X |
|---|---|---|---|
| 2 | 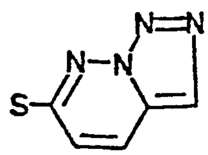 | 1 | 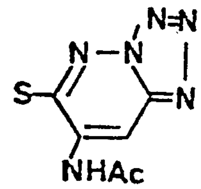 |
| 2 | 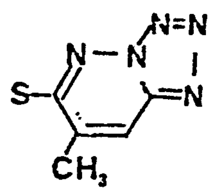 | 1 | 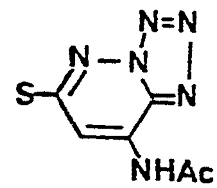 |
| 1 | 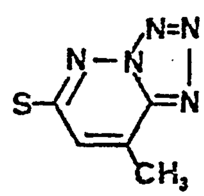 | 1 | 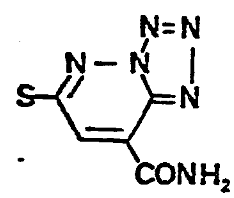 |
| 1 | 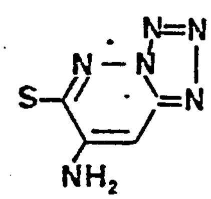 | 1 | 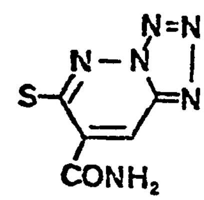 |
| 1 | 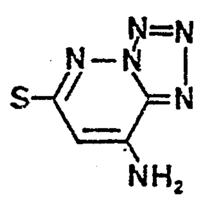 | 1 | 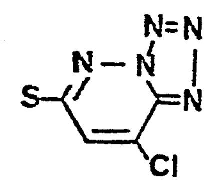 |
| | 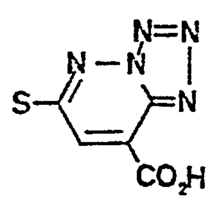 | 1 | 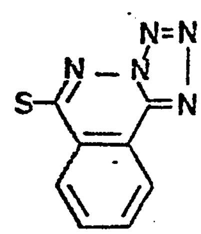 |
| | 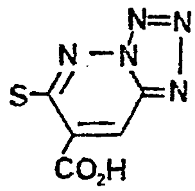 | | |

| n | X | n | X |
|---|---|---|---|
| 1 | 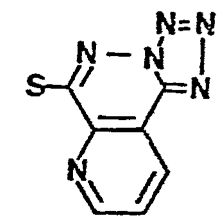 | 1 | 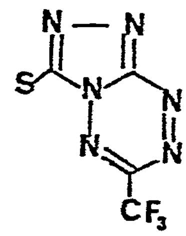 |
| 1 | 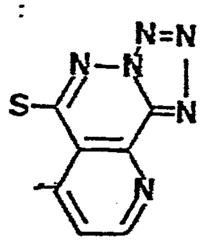 | 1 | 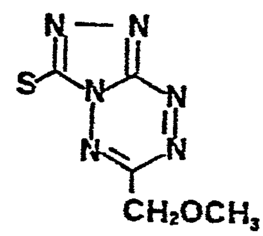 |
| 1 | 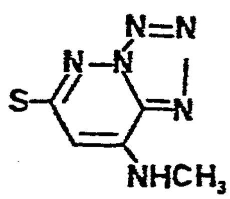 | 1 | 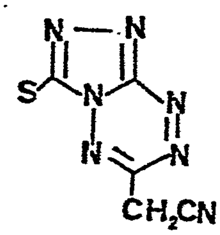 |
| 1 | 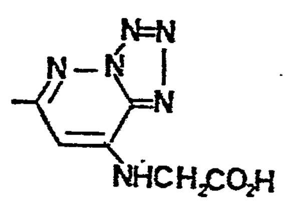 | 1 | 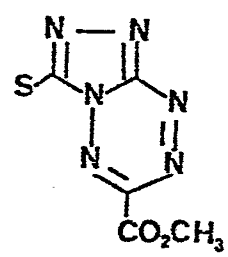 |
| 1 | 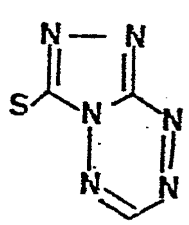 | | |

| n | X | n | X |
|---|---|---|---|
| 1 | 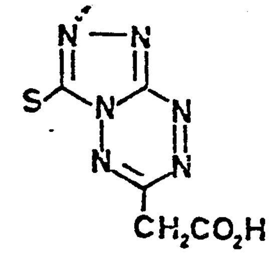 | 1 | 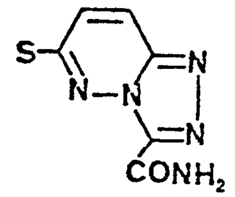 |
| 1 | 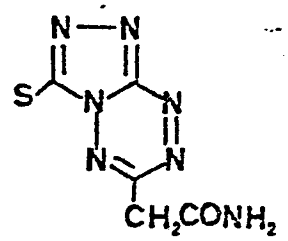 | 2 | 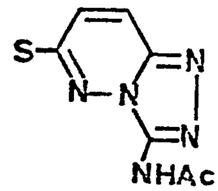 |
| 1 | 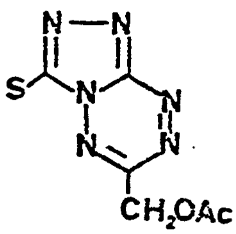 | 1 | 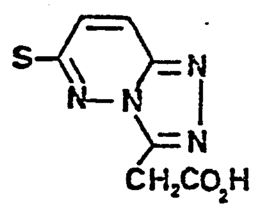 |
| 1 | 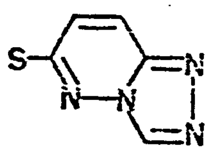 | 1 | 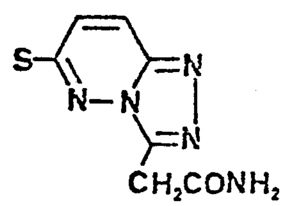 |
| | 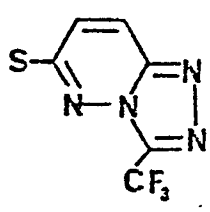 | 1 | 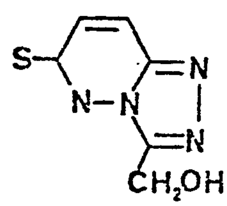 |
| 1 | 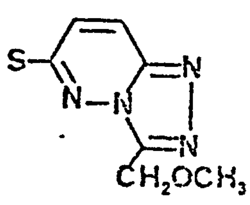 | 1 | 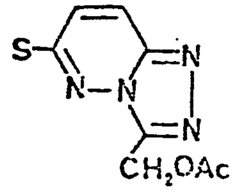 |

| n | X | n | X |
|---|---|---|---|
| 1 | 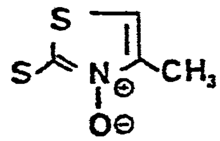 | 1 | 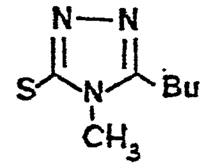 |
| 2 | 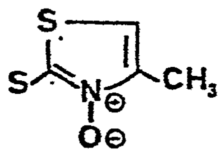 | 2 | 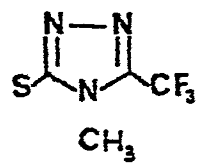 |
| 1 | 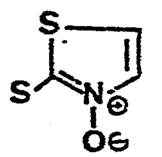 | 1 | 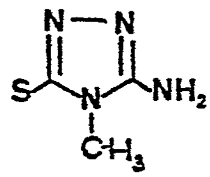 |
| 1 | 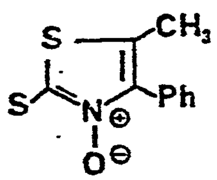 | 1 | 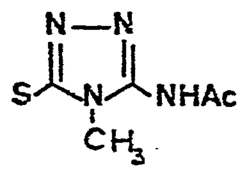 |
| 1 | 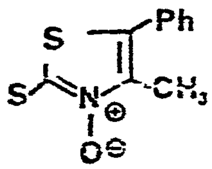 | 1 | 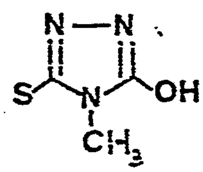 |
| 1 | 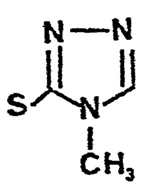 | 1 | 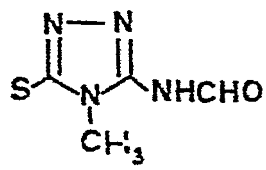 |
| 1 | 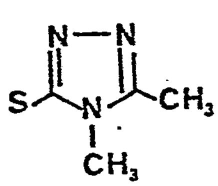 | 1 | 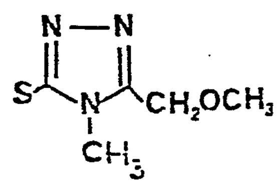 |

EXAMPLE 97

4-Nitrobenzyl (5R,6S)-6-(1(R)-hydroxyethyl)-3-(1,3,4-oxadiazol-2-yl-thio-ethyl-2-thio)-7-oxo-4-thia-1-azabicyclo[3.2.0]hept-2-ene-2-carboxylate.

---

To a stirred solution of 85 mg of 4-nitrobenzyl (5R,6S)-6-(1(R)-hydroxyethyl)-7-oxo-4-thia-3-thioxo-1-azabicyclo[3.2.0]hept-2-ene-2-carboxylate in 2 ml of dry THF was added 60 mg of 2-(2-iodoethylthio)-1,3,4-oxadiazole and 35 mg of diisopropylethylamine. The solution was stirred at room temperature for 16 hours and solvents evaporated in vacuo. The residue was chromatographed over silica gel eluting with hexane-ethyl acetate mixtures to give the title compound as a pale yellow gum (32 mg).

δ ($d^6$-DMSO)
1.17 (3H, d, J=6.2Hz)
3.22-3.65 (4H,m)
3.89 (1H, m)
3.94-4.06 (1H, m)
5.29, 5.44 (2H, ABq, J=14.1Hz)
5.77, (1H, d, J=1.5Hz)
7.67-8.26 (4H, m)
9.31 (1H, s)

0127847

EXAMPLE 98

Potassium (5R,6S)-6-(I(R)-hydroxyethyl)-3-(1,3,4-oxadiazol-2-yl-thio-ethyl-2-thio)-7-oxo-4-thia-1-azabicyclo[3.2.0]hept-2-ene-2-carboxylate

A mixture of 32 mg of 4-nitrobenzyl(5R,6S)-6-(I(R)-hydroxyethyl)-3-(1,3,4-oxadiazol-2-yl-thio-ethyl-2-thio)-7-oxo-4-thia-1-azabicyclo[3.2.0]hept-2-ene-2-carboxylate in 10 ml of ethyl acetate, 6.3 mg of potassium hydrogen carbonate in water (10 ml), and 64 mg of 10% palladium/charcoal catalyst was hydrogenated at 50 p.s.i. until TLC analysis indicated complete reaction. The mixture was filtered through a "Hyflo" (Trade Mark) pad and the aqueous layer was separated and lyophilised to give the title compound as an off-white solid (15 mg).

ν max (KBr) 1772 $cm^{-1}$

δ ($d^6$-DMSO) 1.14 (3H, d, J=6.3Hz)
2.92-3.60 (4H, m)
3.83-4.02 (2H, m)
5.46 (1H, d, J= 1.5Hz)
9.26 (1H, s)

EXAMPLE 99

4-Nitrobenzyl (5R,6S)-6-(1(R)-hydroxyethyl)-3-(5-methoxymethyl-1,3,4-oxadiazol-2-yl-thioethyl-2-thio)-7-oxo-4-thia-1-azabicyclo-[3.2.0]hept-2-ene-2-carboxylate.

---

To a stirred solution of 158.5 mg of 4-nitrobenzyl (5R,6S)-6-(1-(R)-hydroxyethyl-7-oxo-4-thia-3-thioxo-1-azabicyclo[3.2.0]hept-2-ene-2-carboxylate in 10 ml dry THF was added 131 mg of 2-(2-iodoethylthio)-5-methoxymethyl-1,3,4-oxadiazole and 59 mg of diisopropylethylamine. The solution was stirred at room temperature for 16 hours and solvents evaporated _in vacuo_. The residue was chromatographed over silica gel, eluting with hexane-ethyl acetate mixtures, to give the title compound as a pale yellow gum (132.5 mg).

δ ($d^6$-DMSO) 1.17 (3H, d, J=6.3Hz)
3.35 (3H, s)
3.31-3.64 (4H, m)
3.89 (1H, dd, J=5.8, 1.4Hz)
3.96-4.08 (1H, m)
4.65-(2H, s)
5.45, 5.29 (2H, ABq, J=14.1Hz)
5.77 (1H, d, J=1.5Hz)
7.67-8.27 (4H, m)

0127847

EXAMPLE 100

Potassium (5R, 6S)-6-(1(R)-hydroxyethyl)-3-(5-methoxymethyl-1,3,4-oxadiazol-2-yl-thio-ethyl-2-thio)-7-oxo-4-thia-1-azabicyclo-[3.2.0]hept-2-ene-2-carboxylate

---

A mixture of 158.5 mg of 4-nitrobenzyl (5R, 6S)-6-(1(R)-hydroxyethyl)-3-(5-methoxymethyl-1,3,4-oxadiazol-2-yl-thioethyl-2-thio)-7-oxo-4-thia-1-azabicyclo[3.2.0]hept-2-ene-2-carboxylate in 10 ml ethyl acetate, 24 mg of potassium hydrogen carbonate in water (10 ml), and 260 mg of 10% palladium/charcoal catalyst was hydrogenated at 50 p.s.i. until TLC analysis indicated complete reaction. The mixture was filtered through a "Hyflo" (Trade Mark) pad and the aqueous layer was separated and lyophilised to give the title compound as an off-white solid (46 mg).

| | |
|---|---|
| δ ($D_2O$) | 1.31 (3H, d, J=6.4Hz) |
| | 3.33 (3H, s) |
| | 3.19-3.66 (4H, m) |
| | 3.91 (1H, dd, J=6.0, 1.3Hz) |
| | 4.20-4.27 (1H, m) |
| | 4.65 (2H, s) |
| | 5.61 (1H, d, J=1.2Hz) |

EXAMPLE 101

4-Nitrobenzyl (5R,6S)-6-(1(R)-hydroxyethyl)-3-(5-phenyl-1,3,4-oxadiazol-2-yl-thio-ethyl-1-thio)-7-oxo-4-thia-1-azabicyclo[3.2.0]hept-2-ene-2-carboxylate

---

To a stirred solution of 116 mg of 4-nitrobenzyl (5R, 6S)-6-(1(R)-hydroxyethyl)-7-oxo-4-thia-3-thioxo-1-azabicyclo[3.2.0]hept-2-ene-2-carboxylate in 10 ml dry THF was added 106 mg of 5-(1-iodoethylthio)-2-phenyl-1,3,4-oxadiazole and 41 mg of diisopropylethylamine. The solution was stirred at room temperature for 2.5 hours and the solvents evaporated in vacuo. The residue was chromatographed over silica gel, eluting with hexane-ethyl acetate mixtures to give the title compound as a pale yellow gum (66 mg).

δ ($CDCl_3$) 1.36 (3H, d, J=6.3Hz)
1.96-2.05 (3H, m)
3.81 (1H, m)
4.26-4.38 (1H, m)
5.19-5.50 (3H, m)
5.73 (1H, d, J=1.6Hz)
7.57-8.31 (4H, m)
7.65-8.11 (5H, m)

0127847

EXAMPLE 102

Potassium (5R, 6S)-6-(1(R)-hydroxyethyl)-3-(5-phenyl-1,3,4-oxadiazol-2-yl-thioethyl-1-thio)-7-oxo-4-thia-1-azabicyclo[3.2.0]hept-2-ene-2-carboxylate

A mixture of 66 mg of 4-nitrobenzyl (5R,6S)-6-(1(R)-hydroxyethyl)-3-(5-phenyl-1,3,4-oxadiazol-2-yl-thio-ethyl-1-thio)-7-oxo-4-thia-1-azabicyclo[3.2.0]hept-2-ene-2-carboxylate in 10 ml of ethyl acetate, 11.3 mg of potassium hydrogen carbonate in water (10 ml), and 130 mg of 10% palladium/charcoal catalyst was hydrogenated at 50 p.s.i. until T.L.C. analysis indicated complete reaction. The mixture was filtered through a "Hyflo" (Trade Mark) pad and the aqueous layer was separated and lyophilised to give the title compound as an off-white solid (32 mg).

| | |
|---|---|
| νmax | (KBr) 1765 $cm^{-1}$ |
| δ($D_2O$) | 1.20 (3H, d, J=6.3Hz) |
| | 1.85-1.95 (3H, m) |
| | 3.75-3.66 (1H, m) |
| | 4.13-4.26 (1H, m) |
| | 5.19-5.42 (1H, m) |
| | 5.62-(1H, d, J=1Hz) |
| | 7.60-8.12 (5H, m) |

0127847

EXAMPLE 103

4-Nitrobenzyl (5R,6S)-6-(1(R)-hydroxyethyl)-3-(5-phenyl-1,3,4-oxadiazol-2-yl-thio-ethyl-2-thio)-7-oxo-4-thia-1-azabicyclo-[3.2.0]hept-2-ene-2-carboxylate

---

To a stirred solution of 218 mg of 4-nitrobenzyl (5R, 6S)-6-(1(R)-hydroxyethyl)-7-oxo-4-thia-3-thioxo-1-azabicyclo[3.2.0]-hept-2-ene-2-carboxylate in 5 ml dry THF was added 189 mg of 2-(2-iodoethylthio)-5-phenyl-1,3,4-oxadiazole and 81 mg of diisopropylethylamine. The solution was stirred at room temperature for 2 hours and solvents evaporated _in vacuo_. The residue was chromatographed over silica gel, eluting with hexane-ethyl acetate mixtures, to give the title compound as a pale brown gum (176 mg).

νmax ($CHCl_3$) 1791 $cm^{-1}$

δ($CDCl_3$) 1.36 (3H, d, J=6.3Hz)
3.41-3.63 (4H, m)
3.77 (1H, dd, J=6.6, 1.4 Hz)
4.19-4.35 (1H, m)
5.21, 5.47 (2H, ABq, J=13.7Hz)
5.70 (1H, d, J=1.4Hz)
7.46-8.28 (9H, m)

0127847

EXAMPLE 104

Potassium (5R, 6S)-6-(1(R)-hydroxyethyl)-3-(5-phenyl-1,3,4-oxadiazol-2-yl-thio-ethyl-2-thio)-7-oxo-4-thia-1-azabicyclo-[3.2.0]hept-2-ene-2-carboxylate

A mixture of 166 mg of 4-nitrobenzyl (5R,6S)-6-(1(R)-hydroxyethyl)-3-(5-phenyl-1,3,4-oxadiazol-2-yl-thio-ethyl-2-thio)-7-oxo-4-thia-1-azabicyclo[3.2.0]hept-2-ene-2-carboxylate in 10 ml of ethyl acetate, 28 mg of potassium hydrogen carbonate in water (10 ml), and 332 mg of 10% palladium/charcoal catalyst was hydrogenated at 50 p.s.i. until T.L.C. analysis indicated complete reaction. The mixture was filtered through a "Hyflo" (Trade Mark) pad and the aqueous layer was separated and lyophilised to give the title compound as a dark solid (73 mg).

| | |
|---|---|
| ν max (KBr) | 1786 $cm^{-1}$ |
| δ ($D_2O$) | 1.19 (3H, d, J = 6.4Hz) |
| | 3.16-3.68 (4H, m) |
| | 3.71 (1H, dd, J=5.7, 1.3Hz) |
| | 4.11-4.22 (1H, m) |
| | 5.51 (1H, d, J=1.3Hz) |
| | 7.53-8.06 (5H, m) |

Claims:

1. A compound of the general formula I

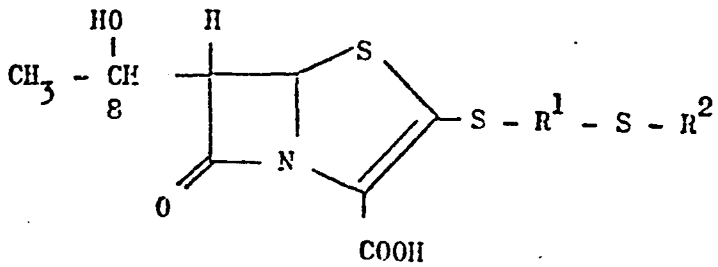

(I)

in which $R^1$ represents an alkylene, alkenylene or alkynylene group, having from 1 to 4 carbon atoms, and

$R^2$ represents a heterocyclic group having a five-membered or 6-membered ring with 1 to 4 heteroatoms, selected from oxygen sulfur and nitrogen, and being linked to the adjacent sulphur atom via a ring carbon atom,

or a pharmaceutically acceptable salt thereof.

2. A compound as claimed in claim 1 in which $R^1$ means the methylene group.

3. A compound according to claim 2 characterized in that $R^2$ is a monocyclic group.

4. A compound according to claim 3 characterized in that $R^2$ is an oxadiazolyl group.

5. A compound according to claim 3 characterized in that $R^2$ is a thiazolyl group.

6. A compound according to claim 3 characterized in that $R^2$ is a thiadiazolyl group.

7. A compound according to claim 3 characterized in that $R^2$ is a tetrazolyl group.

8. A process for the production of a compound of the general formula I as claimed in claim 1 characterized in that a compound of the general formula IIa or a compound of the general formula IIb or a mixture thereof

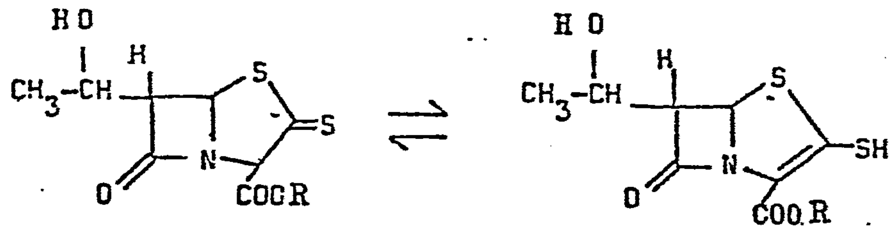

(IIa) (IIb)

in which R is a group removable by hydrolysis, by photolysis, by reduction or by enzyme action, is reacted with a compound of formula III

$$L - R^1 - S - R^2 \qquad (III)$$

in which $R^1$, $R^2$ are as defined above, and L represents a leaving group that is capable of being replaced by a nucleophilic group, generally carrying out the reaction in the presence of a base; and, if desired, carrying out any one or more of the following steps in any desired order on a resulting compound of formula I:

(i) converting an ester of formula I into the corresponding free acid,

(ii) converting a free acid of formula I into an ester thereof,

(iii) transesterifying an ester of a compound of formula I,

(iv) converting a free acid or an ester of formula I into a salt, or a salt into a free acid, an ester or another salt,

(v) removing any protective groups present other than an esterifying group,

(vi) converting a substituent of a group $R^2$ into another substituent thereof.

9. A pharmaceutical preparation characterized in that it consists of an active ingredient of the general formula I as claimed in claim 1 in admixture with a pharmaceutically suitable carrier.

HOE 83/S 002 0127847

Claims for Austria:

1. Process for the preparation of a compound of the general formula I

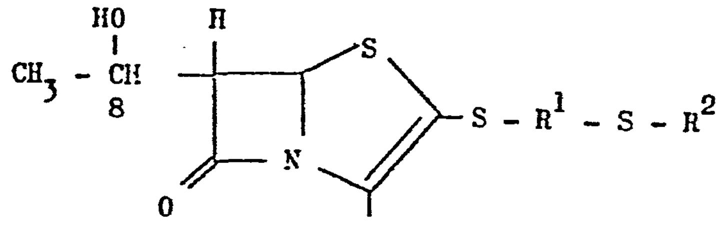

(I)

in which $R^1$ represents an alkylene, alkenylene or alkynylene group, having from 1 to 4 carbon atoms, and

$R^2$ represents a heterocyclic group, having a five-membered or 6-membered ring with up to 4 heteroatoms, selected from oxygen, sulphur and nitrogen, and being linked to the adjacent sulphur atom via a ring carbon atom

and of physiologically tolerable esters and salts thereof, which comprises

reacting a compound of the general formula IIa or a compound of the general formula IIb or a mixture thereof

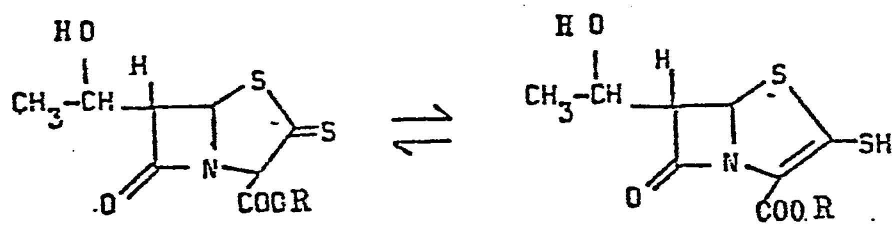

(IIa) (IIb)

in which R is a group removable by hydrolysis, by photolysis, by reduction or by enzyme action,

with a compound of formula III

$$L - R^1 - S - R^2 \quad \text{(III)}$$

in which $R^1$, $R^2$ are as defined above, and L represents a leaving group that is capable of being replaced by a nucleophilic group, generally carrying out the reaction in the presence of a base;
and, if desired, carrying out any one or more of the following steps in any desird order on a resulting compound of formula I:

(i) converting an ester of formula I into the corresponding free acid,
(ii) converting a free acid of formula I into an ester thereof,
(iii) transesterifying an ester of a compound of formula I,
(iv) converting a free acid or an ester of formula I into a salt, or a salt into a free acid, an ester or another salt,
(v) removing any protective groups present other than an esterifying group,
(vi) converting a substituent of a group $R^2$ into another substituent thereof.

2. Process according to claim 1 which comprises carrying out the reaction in a solvent that is inert under the reaction conditions.

3. Process as claimed in claims 1 and/or 2 which comprises using a non-nucleophilic base.

4. Process as claimed in any one of claims 1 - 3 which comprises a reaction temperature of from -80 to +30°C.

5. Process as claimed in any one of claims 1 - 4 in which $R^1$ represents a methylene group.

6. Process as claimed in any one of claims 1 - 5 in which $R^2$ is a five-membered or 6-membered ring with 1 - 4 heteroatoms.

7. Process according to claims 1 - 6 in which $R^2$ represents a thiazolyl radical.

8. Process according to claims 1 - 6 in which $R^2$ represents an oxadiazolyl radical.

9. Process according to claims 1 - 6 in which $R^2$ represents a thiadiazolyl radical.

10. Process for the production of a pharmaceutical preparation characterized in that an active ingredient of the general formula I as claimed in claim 1 is mixed with a pharmaceutically suitable carrier.

0127847

European Patent Office

# EUROPEAN SEARCH REPORT

Application number

EP 84 10 5904

## DOCUMENTS CONSIDERED TO BE RELEVANT

| Category | Citation of document with indication, where appropriate, of relevant passages | Relevant to claim | CLASSIFICATION OF THE APPLICATION (Int Cl 3) |
|---|---|---|---|
| A | EP-A-0 002 210 (MERCK)<br>* Claims 1-6 *<br>--- | 1,9 | C 07 D 499/00<br>C 07 D 519/00<br>A 61 K 31/43 //<br>C 07 D 257/04<br>(C 07 D 519/00<br>C 07 D 499/00<br>C 07 D 471/00 )<br>(C 07 D 519/00<br>C 07 D 499/00<br>C 07 D 487/00 ) |
| A | CHEMICAL ABSTRACTS, vol. 99, no. 23, December 5, 1983, page 730, no. 194712w, Columbus, Ohio, US; & JP - A - 58 43 978 (SANKYO CO., LTD.) 14-03-1983<br>* Abstract *<br>--- | 1,8,9 | |
| P,X | US-A-4 431 654 (V.M. GIRIJAVALLABHAN)<br>* Column 4; claims *<br>----- | 1-9 | |
| | | | TECHNICAL FIELDS SEARCHED (Int. Cl. 3) |
| | | | C 07 D 499/00<br>C 07 D 519/00<br>A 61 K 31/00 |

The present search report has been drawn up for all claims

| Place of search | Date of completion of the search | Examiner |
|---|---|---|
| THE HAGUE | 23-08-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document